# EUROPEAN PATENT SPECIFICATION

(11) **EP 2 411 410 B1**
(45) Date of publication and mention of the grant of the patent: **08.07.2015**
(21) Application number: 10745031.4
(22) Date of filing: 26.03.2010
(51) Int. Cl.: C07K 16/12

(54) **COMPOSITIONS AND METHODS FOR SCREENING AND USING COMPOUNDS ANTAGONIZING SPORE-SURFACE INTERACTIONS**
ZUSAMMENSETZUNGEN UND VERFAHREN FÜR SCREENING UND VERWENDUNG VON VERBINDUNGEN ALS ANTAGONISTEN FÜR SPOREN-OBERFLÄCHEN-INTERAKTIONEN
COMPOSITIONS ET PROCÉDÉS POUR CRIBLER ET UTILISER DES COMPOSÉS ANTAGONISTES DES INTERACTIONS SPORE-SURFACE

(30) Priority: 27.03.2009 US 164154 P
(43) Date of publication of application: 01.02.2012
(73) Proprietor: Gojo Industries, Inc., Akron, Ohio 44309 (US)
(72) Inventor: MACINGA, David, R., Stow OH 44224 (US); BINGHAM, James, Edmund, Akron OH 44313 (US); EDMONDS, Sarah, L., Canal Fulton OH 44614 (US)
(74) Representative: Makovski, Priscilla Mary
(86) International application number: PCT/US2010/028902
(87) International publication number: WO 2010/126670

(56) References cited:
- WO-A2-2007/148065
- WELKOS SUSAN ET AL: "The role of antibodies to Bacillus anthracis and anthrax toxin components in inhibiting the early stages of infection by anthrax spores", MICROBIOLOGY (READING), vol. 147, no. 6, June 2001 (2001-06), pages 1677-1685, XP002609760, ISSN: 1350-0872
- SEBAIHIA MOHAMMED ET AL: "The multidrug-resistant human pathogen Clostridium difficile has a highly mobile, mosaic genome", NATURE GENETICS, vol. 38, no. 7, July 2006 (2006-07), pages 779-786, XP002609761, ISSN: 1061-4036
- WILLIAMSON P ET AL: "A new method for the quantitative investigation of cutaneous bacteria.", THE JOURNAL OF INVESTIGATIVE DERMATOLOGY DEC 1965 LNKD- PUBMED:5321315, vol. 45, no. 6, December 1965 (1965-12), pages 498-503, XP002609762, ISSN: 0022-202X
- KASPER D J ET AL: "Comparison of Plate Stamp and Glove Juice Methods for Assessing Removal of Clostridium difficile Spores from Hands", ABSTRACTS OF THE INTERSCIENCE CONFERENCE ON ANTIMICROBIAL AGENTS AND CHEMOTHERAPY, vol. 48, 2008, page 566, XP001525627, & 48TH ANNUAL INTERSCIENCE CONFERENCE ON ANTIMICROBIAL AGENTS AND CHEMOTHERAPY/46TH ANNUAL MEETING OF; WASHINGTON, DC, USA; 20081025, ISSN: 0733-6373
- JEDRZEJAS M J: "THE STRUCTURE AND FUNCTION OF NOVEL PROTEINS OF BACILLUS ANTHRACIS AND OTHER SPORE-FORMING BACTERIA: DEVELOPMENT OF NOVEL PROPHYLACTIC AND THERAPEUTIC AGENTS", CRITICAL REVIEWS IN BIOCHEMISTRY AND MOLECULAR BIOLOGY, CRC PRESS, BOCA RATON, FL, US, vol. 37, no. 5, 1 January 2002 (2002-01-01), pages 339-373, XP009041491, ISSN: 1040-9238, DOI: DOI:10.1080/10409230290771537

## Description

### Field Of Invention

The present invention is related to the field of infection control. Particularly, the invention is related to compositions and methods for maintenance of antiseptic conditions, bacterial recontamination, and/or bacterial exposure prophylaxis. In one embodiment, the invention contemplates infection control compositions that inhibit microbial spore binding to a substrate surface. For example, the inhibition disrupts and/or displaces an interaction, attachment, and/or stabilization of a microbial spore to a substrate surface.

### Background

Spore-forming bacteria may be responsible for contaminations including, but not limited to, hospital infections, food spoilage and/or food-borne illness problems. As the production of minimally processed refrigerated products becomes more efficient and aseptic, background microflora are eliminated, and it is the spore-forming organism which eventually may limit the shelf-lives of these products. Spore-forming bacteria are suspected of being responsible for the spoilage of canned foods, bread, vacuum packed meats, pasteurized dairy products, and fruit juices. The presence of bacterial spores at high levels in ingredients going into any of these types of products may increase the potential for spoilage of the finished product.

In the healthcare environment, spore-forming bacteria frequently cause infections including nosocomial diarrhea, tetanus, and gangrene. *Clostridium difficile* is a spore forming anaerobic bacterium which causes antibiotic associated diarrhea and pseudomembraneous colitis. The recent emergence of a highly pathogenic epidemic strain with increased attributable morbidity and mortality has further increased the importance of this nosocomial pathogen. Reductions in hospital staff-to-patient infectious transmission would be expected to drastically improve recovery times thereby resulting in accelerated patient release. This is beneficial from both patient health and societal cost viewpoints. Despite efforts by the medical community to increase the use of antiseptics to prevent microbial transmission, the occurrence of patient infection and reinfection leads to unnecessary morbidity and mortality.

Bacteria that form spores that can reside in a donnant form for decades before germinating in a host cell and may be responsible for unexpected contamination, whether related to the food industry or in the practice of medicine. Furthermore, bacterial endospores are resistant to skin and surface disinfectants and thus current antimicrobial measures are largely ineffective. What is needed is a method to screen for infection control compositions that remove and/or prevent the attachment to, and subsequent infection of, a host by a microbial spore.

### Summary

According to a first aspect of the invention there is provided a screening method for screening for infrection control compositions in accordance with claim 1 of the appended claims.

The present invention is related to the field of infection control. Particularly, the invention is related to compositions and methods for maintenance of antiseptic conditions, bacterial decontamination, and/or bacterial exposure prophylaxis. In one embodiment, the invention contemplates infection control compositions that inhibit microbial spore binding to a substrate surface. For example, the inhibition antagonizes, disrupts, and/or displaces an interaction, attachment, and/or stabilization of a microbial spore to a substrate surface.

In one embodiment, the present invention contemplates an infection control composition capable of blocking and/or inhibiting the binding of a microbial spore to a substrate surface. In one embodiment, the composition comprises a small organic molecule. In one embodiment, the composition comprises a protein. In one embodiment, the peptide comprises a spore surface peptide. In one embodiment, the peptide is derived from a *Clostridia* spore surface protein. In one embodiment, the *Clostridia* spore surface protein comprises a *Clostridia difficile* spore surface protein. In other unclaimed embodiments, the peptide is derived from a *Bacillus* spore surface protein. The *Bacillus* spore surface protein comprises a *Bacillus anthracis* spore surface protein. In one embodiment, the *Clostridium difficile* spore surface peptide comprises SEQ ID NO: 1 or a homologue of SEQ ID NO: 1. In one embodiment, the spore surface peptide is selected from the group comprising SEQ ID NO: 3, SEQ ID NO: 5, SEQ ID NO: 7, and SEQ ID NO: 15. In one embodiment, the composition comprises an antibody. In one embodiment, the antibody is a polyclonal antibody. In one embodiment, the antibody is a monoclonal antibody. In one embodiment, the composition comprises a synthetic peptide. In one embodiment, the composition comprises an inorganic ion complex. In one embodiment, the composition comprises a protein mimetic. In one embodiment, the substrate surface comprises an inanimate surface. In one embodiment, comprises an animate surface. In one embodiment, the animate surface comprises a body tissue surface. In one embodiment, the body tissue surface comprises an epithelial surface. In one embodiment, the epithelial surface comprises a mammalian skin surface. In one embodiment, the inanimate surface comprises stainless steel. In one embodiment, the inanimate surface comprises ceramic. In one embodiment, the inanimate surface comprises vinyl. In one embodiment, the inanimate surface comprises tile. In one embodiment, the composition further comprises at least one antimicrobial drug. In one embodiment, the composition further comprises a carrier. In one embodiment, the carrier further comprises a detergent. In one embodiment, the carrier is sterile and suitable for human administration. In one embodiment, the carrier comprises an antimicrobial skin cleaner. In one embodiment, the skin cleaner comprises a handwash cleaner. In one embodiment, the carrier comprises an antimicrobial surface cleaner.

Also described but not claimed is an infection control composition comprising an amino acid sequence capable of blocking and/or inhibiting the binding of a microbial spore to a substrate surface, wherein the amino acid sequence has substantial homology to a microbial spore surface protein. In one embodiment, the substantial homology comprises approximately 75% homology, preferably 85% homology, but most preferably 90% or 95% homology. In one embodiment, the surface protein comprises a spore surface protein. In one embodiment, the spore surface protein is a *Clostridium* spore surface protein. In one embodiment, the *Clostridium* spore surface protein is a C. *difficile* spore surface protein. In one embodiment, the spore surface protein is a *Bacillus* spore surface protein. In one embodiment, the *Bacillus* spore surface protein is a *B. anthracis* spore surface protein. In one embodiment, the spore surface protein comprises a spore surface peptide.. In one embodiment, the peptide is derived from a *Clostridia* surface protein. In one embodiment, the *Clostridia* surface protein comprises a *Clostridia difficile* surface protein. In one embodiment, the peptide is derived from a *Bacillus* surface protein. In one embodiment, the *Bacillus* surface protein comprises a *Bacillus anthracis* surface protein. In one embodiment, the *Clostridium difficile* spore surface peptide comprises SEQ ID NO: 1 or a homologue of SEQ ID NO: 1. In one embodiment, the spore surface peptide is selected from the group comprising SEQ ID NO: 3, SEQ ID NO: 5, SEQ ID NO: 7, and SEQ ID NO: 15. In one embodiment, the amino acid sequence is derived from a C-terminal end of the spore surface protein. In one embodiment, the amino acid sequence is derived from an N-terminal end of a spore surface protein. In one embodiment, the composition further comprises a carrier. In one embodiment, the carrier comprises a detergent. In one embodiment, the composition further comprises at least one antimicrobial drug. In one embodiment, the carrier is suitable for human administration. In one embodiment, the composition further comprises an antimicrobial skin cleaner. In one embodiment, the skin cleaner is a handwash cleaner. In one embodiment, the composition further comprises an antimicrobial surface cleaner. In one embodiment, the composition further comprises a skin barrier. In one embodiment, the composition further comprises a surface barrier.

Also described is an infection control composition comprising an antagonistic compound, wherein the compound has affinity for a microbial spore surface protein amino acid sequence. In one embodiment, the affinity is directed at a substrate surface specific binding site region. In one embodiment, the compound comprises an antibody. In one embodiment, the compound comprises a small organic molecule. In one embodiment, the spore surface protein amino acid sequence comprises a peptide fragment. In one embodiment, the peptide fragment is derived from a C-terminal end of the spore surface protein. In one embodiment, the peptide fragment is derived from an N-terminal end of the spore surface protein. In one embodiment, the spore surface protein is a *Clostridium* spore surface protein. In one embodiment, the *Clostridium* spore surface protein is a C. *difficile* spore surface protein. In one embodiment, the spore surface protein is a *Bacillus* spore surface protein. In one embodiment, the *Bacillus* spore surface protein is a *B. anthracis* spore surface protein. In one embodiment, the composition further comprises at least one antimicrobial drug. In one embodiment, the composition further comprises a carrier. In one embodiment, the carrier is suitable for human administration. In one embodiment, the composition further comprises an antimicrobial hand cleaner. In one embodiment, the composition further comprises an antimicrobial surface cleaner.

Also described but not claimed is a method, comprising: a) providing: i) a subject at risk for exposure to a microbe, wherein the exposure is likely to result in a microbial infection; and ii) an infection control composition; b) administering the composition to the subject before the microbial exposure, under conditions such that a microbial infection barrier is formed. In one embodiment, the microbial infection barrier reduces the microbial infection. In one embodiment, the microbial infection barrier prevents the microbial infection. In one embodiment, the microbe comprises a bacteria. In one embodiment, the bacteria is selected from the group comprising *Clostridia,Bacillus, Desulfotomqculum. Sporolactobacillus, Sporosarcina*, *or Thermoactinomyces* In one embodiment, the bacteria comprises a bacterial spore. In one embodiment, the bacterial spore comprises at least one spore surface protein. In one embodiment, the *Clostridium* spore surface protein is a C. *difficile* spore surface protein. In one embodiment, the *Bacillus* spore surface protein is a *B. anthracis* spore surface protein. In one embodiment, the infection control composition comprises an amino acid sequence. In one embodiment, the amino acid sequence is derived from a microbial spore surface protein. In one embodiment, the amino acid sequence has substantial homology to a microbial spore surface protein. In one embodiment, the infection control composition comprises is capable of inhibiting, antagonizing, disrupting, displacing, and/or blocking the binding of a microbial spore to a substrate surface. In one embodiment, the infection control composition further comprises an antagonistic compound, wherein the compound has affinity for a microbial spore surface protein amino acid sequence. In one embodiment, the composition further comprises a carrier. In one embodiment, the infection control composition further comprises at least one antimicrobial drug. In one embodiment, the subject at risk is a healthcare worker. In one embodiment, the subject at risk is a medical first responder. In one embodiment, the subject at risk is a medical patient. In one embodiment, the patient is in a healthcare setting. In one embodiment, the medical patient has been administered at least one antibiotic. In one embodiment, the subject at risk is a fireman. In one embodiment, the subject at risk is a policeman. In one embodiment, the subject at risk is a military personnel. In one embodiment, the subject at risk is a food handler. In one embodiment, the subject at risk is an inanimate surface and/or object. In one embodiment, the administering is selected from the group comprising topical, oral, parenteral, pulmonary, anal, vaginal, ocular, or intranasal.

Also described is a method, comprising: a) providing: i) a subject having been exposed to a microbe, wherein the exposure resulted in a microbial infection; ii) an infection control composition; b) administering the composition to the subject after the microbial exposure, under conditions such that the microbial infection is reduced. In one embodiment, the microbe comprises a bacteria. In one embodiment, the bacteria is selected from the group comprising *Clostridia, Bacillus, Desulfotomaculum, Sporolactobacillus*, *Sporosarcina, or Thermoactinomyces.* In one embodiment, the bacteria comprises a bacterial spore. In one embodiment, the bacterial spore comprises at least one spore surface protein. In one embodiment, the *Clostridium* spore surface protein is a C. *difficile* spore surface protein. In one embodiment, the *Bacillus* spore surface protein is a *B. anthracis* spore surface protein. In one embodiment, the infection control composition comprises an amino acid sequence. In one embodiment, the amino acid sequence is derived from a microbial spore surface protein. In one embodiment, the amino acid sequence has substantial homology to a microbial spore surface protein. In one embodiment, the infection control composition is capable of inhibiting, antagonizing, disrupting, displacing and/or blocking the binding of a microbial spore to a substrate surface. In one embodiment, the infection control composition further comprises an antagonistic compound, wherein the compound has affinity for a microbial spore surface protein amino acid sequence. In one embodiment, the composition further comprises a carrier. In one embodiment, the infection control composition further comprises at least one antimicrobial drug. In one embodiment, the exposed subject is a healthcare worker. In one embodiment, the exposed subject is a medical first responder. In one embodiment, the exposed subject is a fireman. In one embodiment, the exposed subject is a policeman. In one embodiment, the exposed subject is a military personnel. In one embodiment, the administering is selected from the group comprising topical, oral, parenteral, pulmonary, anal, vaginal, ocular, or intranasal. In one embodiment, the exposed subject is an inanimate surface and/or object.

Also described is a screening method comprising: a) providing; i) an isolated peptide, wherein the peptide is derived from a microbial spore surface protein; and ii) an infection control test compound suspected of having an interaction with the peptide; b) contacting the peptide with the infection control test compound; and c) detecting the interaction of the peptide with the infection control test compound. In one embodiment, the method further comprises a substrate capable of attaching the isolated peptide. In one embodiment, the substrate surface comprises pigskin. In one embodiment, the peptide is derived from a *Clostridia* spore surface protein. In one embodiment, the *Clostridia* spore surface protein comprises a *Clostridia difficile* exosporium. The peptide may be derived from a *Bacillus* spore surface protein. Alternatively, the *Bacillus* exosporium comprises a *Bacillus anthracis* spore surface protein. In one embodiment, the peptide comprises a spore surface peptide. In one embodiment, the *Clostridium difficile* spore surface peptide comprises SEQ ID NO: I or a homologue of SEQ ID NO: 1. In one embodiment, the spore surface peptide is selected from the group comprising SEQ ID NO: 3, SEQ ID NO: 5; SEQ ID NO: 7, and SEQ ID NO: 15. In one embodiment, the infection control test compound comprises a small organic molecule. In one embodiment, the infection control test compound comprises a synthetic peptide. In one embodiment, the infection control test compound comprises a protein mimetic. In one embodiment, the infection control test compound comprises an antibody.

Also described is a method, comprising: a) providing; i) an isolated labeled microbial spore surface peptide, wherein said peptide creates a first fluorescent intensity pattern; and ii) an infection control test compound capable of interacting with said peptide; b) contacting said peptide with said compound to produce a contacted peptide having a second fluorescent intensity pattern; c) comparing said first intensity pattern with the second fluorescence intensity pattern. In one embodiment, the method further comprises step d) detecting a difference between the first intensity pattern and the second intensity pattern. In one embodiment, the difference indicates that said compound binds to said peptide. In one embodiment, the peptide is derived from a bacterial spore surface protein. In one embodiment, the bacterial spore surface protein is derived from a *Clostridia* spore surface protein. In one embodiment, the *Clostridia* spore surface protein comprises *Clostridia difficile* spore surface protein. In one embodiment, the bacterial spore surface protein is derived from a *Bacillus* spore surface protein. In one embodiment, the *Bacillus* spore surface protein comprises *Bacillus anthracis* spore surface protein. In one embodiment, the spore surface protein comprises a peptide fragment. In one embodiment, the peptide is selected from the group comprising SEQ ID NO:1, SEQ ID NO: 3, SEQ ID NO: 5, SEQ ID NO: 7, or SEQ ID NO: 9. In one embodiment, the label is selected from the group consisting of 4'-6diamidino- 2-phenylindole (DAPI), anilinonapthalene sulfonate (ANS), bis-ANS (Bisanilinonapthalene sulfonate), N-phenyl-1-naphthalene (NPN), ruthenium red, cresol violet, and 4-(dicyanovinyl)julolidine (DCVJ).

Also described is a method of screening for a compound, comprising: a) providing; i) a bacteria comprising a recombinant expression vector, wherein said vector comprises at least a portion of a spore surface oligonucleotide sequence; and ii) a compound suspected of having infection control activity; b) expressing said vector such that a spore surface protein is displayed on the bacterial membrane; c) contacting said bacteria with said compound; and d) detecting infection control activity of said compound. In one embodiment, the infection control activity comprises forming a binding pair of said compound with said displayed protein. In one embodiment, the oligonucleotide sequence comprises SEQ ID NO: 2. In one embodiment, the oligonucleotide sequence selected from the group comprising SEQ ID NO: 4, SEQ ID NO: 6, SEQ ID NO: 8, or SEQ ID NO: 16. In one embodiment, the recombinant expression vector further comprises a fusion sequence, wherein the fusion sequence comprises reporter sequence. In one embodiment, the reporter sequence is a β-galactosidase sequence.

Also described is a method for detecting the presence of polynucleotide sequences encoding at least a portion of a microbial spore surface protein gene in a sample, the method comprising: a) providing; i) at least a portion of SEQ ID NO: 2; and ii) a sample suspected of containing a spore surface oligonucleotide sequence; b) combining the SEQ ID NO: 2 portion and the sample under conditions such that a hybridization complex is formed between the SEQ ID NO: 2 portion and the spore surface oligonucleotide sequence; and c) detecting the hybridization complex. In one embodiment, the spore surface oligonucleotide is derived from a microbial spore. In one embodiment, the spore is derived from a *Clostridia* species. In one embodiment, the spore is derived from a *Bacillus* species. In one embodiment, the oligonucleotide sequence is RNA. In one embodiment, the oligonucleotide sequence is DNA.

Also described is a method comprising; providing an infection control composition and a substrate surface comprising bacterial spores; applying the composition to the surface, wherein at least 50%, preferably 60%, more preferably 70%, even more preferably 80%, most preferably 90%, or 100% of the spores are removed from the surface. In one embodiment, the surface may be selected from the group consisting of skin, body tissues, inorganic surfaces (i.e., for example, stainless steel, granite, plastic, tile, ceramic etc), gloves, laboratory coats, or medical instruments.

Also described is a method comprising; providing an infection control composition and a substrate surface at high risk of bacterial spore binding; applying the composition to the surface, wherein at least 50%, preferably 60%, more preferably 70%, even more preferably 80%, most preferably 90%, or 100% of bacterial spore binding is prevented. In one embodiment, the surface may be selected from the group consisting of skin, body tissues, inorganic surfaces (i.e., for example, stainless steel, granite, plastic, tile, ceramic etc.) or medical instruments.

Also described is a method of screening a plurality of infection control compositions for binding to a microbial spore protein, comprising: a) providing a chromatography column comprising beads carrying said microbial spore proteins; b) contacting said plurality of infection control compositions with said beads under conditions such that at least one of said infection control compositions form a binding pair with said beads; c) controllably releasing said at least one bound infection control composition from said bead; d) detecting said at least one bound infection control composition. In one embodiment, said binding pair comprises a microbial spore surface protein. In one embodiment, the detecting of the at least one bound infection control composition comprises the production of a fluorescent signal. In one embodiment, the detecting of the at least one bound infection control composition comprises the formation of an enzyme-substrate product. In one embodiment, the step of controllably releasing comprises exposing the beads to a releasing agent. In one embodiment, the releasing agent is selected from a group consisting of light, acid and base.

Also described is an infection control composition comprising a protein mimetic compound and a carrier, wherein the protein mimetic compound has a first specific binding site pattern on a substrate surface wherein the first binding pattern has at least 50% identity, preferably 60% identity, more preferably 70% identity, even more preferably 80% identity, most preferably 90% identity, or 100% identity to a second specific binding site pattern of an amino acid sequence, wherein the sequence has substantial homology to a microbial spore surface protein. In one embodiment, the protein mimetic compound comprises a small organic molecule. In one embodiment, the protein mimetic compound comprises a synthetic peptide. In one embodiment, the protein mimetic compound comprises an inorganic ion complex. In one embodiment, the surface protein comprises a spore surface protein. In one embodiment, the microbial spore surface protein is derived from a *Clostridium* spore surface protein. In one embodiment, the *Clostridium* spore surface protein is a C. *difjicile* spore surface protein. In one embodiment, the spore surface protein is derived from a *Bacillus* spore surface protein. In one embodiment, the *Bacillus* spore surface protein is a *B. anthracis* spore surface protein. In one embodiment, the amino acid sequence is derived from a C-terminal end of the microbial spore surface protein. In one embodiment, the amino acid sequence is derived from a N-terminal end of the microbial spore surface protein. In one embodiment, the substrate surface comprises an animate surface. In one embodiment, the substrate surface comprises an inanimate surface. In one embodiment, the animate surface comprises a body tissue surface. In one embodiment, the body tissue surface comprises a mammalian skin surface. In one embodiment, the inanimate surface comprises stainless steel. In one embodiment, the inanimate surface comprises ceramic. In one embodiment, the inanimate surface comprises vinyl. In one embodiment, the inanimate surface comprises tile. In one embodiment, the carrier comprises a detergent. In one embodiment, the composition further comprises at least one antimicrobial drug. In one embodiment, the carrier is sterile and suitable for human administration. In one embodiment, the composition further comprises an antimicrobial skin cleaner. In one embodiment, the composition further comprises an antimicrobial surface cleaner.

### Definitions

The term "pharmaceutically" or "pharmacologically acceptable", as used herein, refer to molecular entities and compositions that do not produce adverse, allergic, or other untoward reactions when administered to an animal or a human.

The term, "pharmaceutically acceptable carrier", as used herein, includes any and all solvents, or a dispersion medium including, but not limited to, water, ethanol, polyol (for example, glycerol, propylene glycol, and liquid polyethylene glycol, and the like), suitable mixtures thereof, and vegetable oils, coatings, isotonic and absorption delaying agents, liposome, commercially available cleansers, and the like. Supplementary bioactive ingredients also can be incorporated into such carriers.

The term, "purified" or "isolated", as used herein, may refer to a peptide composition that has been subjected to treatment (i.e., for example, fractionation) to remove various other components, and which composition substantially retains its expressed biological activity. Where the term "substantially purified" is used, this designation will refer to a composition in which the protein or peptide forms the major component of the composition, such as constituting about 50%, about 60%, about 70%, about 80%, about 90%, about 95% or more of the composition (i.e., for example, weight/weight and/or weight/volume). The term "purified to homogeneity" is used to include compositions that have been purified to 'apparent homogeneity" such that there is single protein species (i.e., for example, based upon SDS-PAGE or HPLC analysis). A purified composition is not intended to mean that some trace impurities may remain.

As used herein, the term "substantially purified" refers to molecules, either nucleic or amino acid sequences, that are removed from their natural environment, isolated or separated, and are at least 60% free, preferably 75% free, and more preferably 90% free from other components with which they are naturally associated. An "isolated polynucleotide" is therefore a substantially purified polynucleotide.

"Nucleic acid sequence" and "nucleotide sequence" as used herein refer to an oligonucleotide or polynucleotide, and fragments or portions thereof, and to DNA or RNA of genomic or synthetic origin which may be single- or double-stranded, and represent the sense or antisense strand.

The term "an isolated nucleic acid", as used herein, refers to any nucleic acid molecule that has been removed from its natural state (e.g., removed from a cell and is, in a preferred embodiment, free of other genomic nucleic acid).

The term "functionally equivalent codon", as used herein, refers to different codons that encode the same amino acid. This phenomenon is often referred to as "degeneracy" of the genetic code. For example, six different codons encode the amino acid arginine.

The terms "amino acid sequence" and "polypeptide sequence" as used herein, are interchangeable and to refer to a sequence of amino acids.

The term "spore surface protein" as used herein, refers to any protein or peptide fragment that is derived from, or has substantial similarity (i.e., is homologous) to, any bacterial spore exoporium protein. Microbial spore surface proteins are believed responsible for attaching, and immobilizing, a microbial spore to an inanimate and/or animate surface until conditions are sufficient to induce germination.

The term "derived from" as used herein, refers to the source of a compound or sequence. In one respect, a compound or sequence may be derived from an organism or particular species. In another respect, a compound or sequence may be derived from a larger complex or sequence.

As used herein, "BclA" or "BclA polypeptide" or "BclA protein" or "BclA homologue" are used interchangeably to refer to the amino acid sequence of substantially purified *Bacillus-*like protein, obtained from any species, particularly bacterial species which include gram negative, gram positive, aerobic, and anaerobic bacteria, and obtained from any source whether natural, synthetic, semi-synthetic or recombinant. For example, a *Clostridium difficile* BclA homologue would have the same function and activity as a *Bacillus anthracis* BclA peptide.

A "variant" of a protein is defined as an amino acid sequence which differs by one or more amino acids from a polypeptide sequence (i.e., for example, SEQ **ID** NO: 1) or any homolog of the polypeptide sequence. The variant may have "conservative" changes, wherein a substituted amino acid has similar structural or chemical properties, e.g., replacement ofleucine with isoleucine. More rarely, a variant may have "nonconservative" changes, e.g., replacement of a glycine with a tryptophan. Similar minor variations may also include amino acid deletions or insertions (i.e., additions), or both. Guidance in determining which and how many amino acid residues may be substituted, inserted or deleted without abolishing biological or immunological activity may be found using computer programs including, but not limited to, DNAStar® software.

A "variant" of a nucleotide is defined as a novel nucleotide sequence which differs from a reference oligonucleotide by having deletions, insertions and substitutions. These may be detected using a variety of methods (e.g., sequencing, hybridization assays etc.). Included within this definition are alterations to the genomic DNA sequence which encodes a *Clostridium* spore surface protein (i.e., for example, by alterations in the pattern of restriction enzyme fragments capable of hybridizing to SEQ ID NO: 2 (RFLP analysis), the inability of a selected fragment of SEQ ID NO: 2 to hybridize under high stringency conditions to a sample of genomic DNA (e.g., using allele-specific oligonucleotide probes), and improper or unexpected hybridization, such as hybridization to a locus other than the normal chromosomal locus for a *Clostridium* spore surface gene (e.g., using fluorescent in situ hybridization (FISH)).

A "deletion" is defined as a change in either nucleotide or amino acid sequence in which one or more nucleotides or amino acid residues, respectively, are absent.

An "insertion" or "addition" is that change in a nucleotide or amino acid sequence which has resulted in the addition of one or more nucleotides or amino acid residues, respectively, as compared to, for example, the naturally occurring *Clostridium* spore surface protein.

A "substitution" results from the replacement of one or more nucleotides or amino acids by different nucleotides or amino acids, respectively.

The term "derivative" as used herein, refers to any chemical modification of a nucleic acid or an amino acid. Illustrative of such modifications would be replacement of hydrogen by an alkyl, acyl, or amino group. For example, a nucleic acid derivative would encode a polypeptide which retains essential biological characteristics.

As used herein the term "portion" when in reference to a protein (as in "a portion of a given protein") refers to fragments of that protein. The fragments may range in size from four amino acid residues to the entire amino acid sequence minus one amino acid. For example, a protein "comprising at least a portion of the amino acid sequence of SEQ ID NO: 1" encompasses a full-length *Clostridium* spore surface protein and fragments thereof of four (4) amino acids or more.

The term "portion" when used in reference to a nucleotide sequence refers to fragments of that nucleotide sequence. The fragments may range in size from 5 nucleotide residues to the entire nucleotide sequence minus one nucleic acid residue.

The term "biologically active" refers to any molecule having structural, regulatory or biochemical functions. For example, spore surface protein biological activity may be determined, for example, by restoration of wild-type growth in cells lacking spore surface protein activity (i.e., for example, spore surface protein null cells and/or "knock out" cells). Cells lacking spore surface protein activity may be produced by many methods (i.e., for example, point mutation and frame-shift mutation). Complementation is achieved by transfecting cells which lack spore surface protein activity with an expression vector which expresses spore surface protein, a derivative thereof, or a portion thereof.

The term "immunologically active" defines the capability of a natural, recombinant or synthetic peptide (i.e., for example, a spore surface protein), or any oligopeptide thereof, to induce a specific immune response in appropriate animals or cells and/or to bind with specific antibodies.

The term "antigenic determinant" as used herein refers to that portion of a molecule that is recognized by a particular antibody (i.e., an epitope). When a protein or fragment of a protein is used to immunize a host animal, numerous regions of the protein may induce the production of antibodies which bind specifically to a given region or three-dimensional structure on the protein; these regions or structures are referred to as antigenic determinants. An antigenic determinant may compete with the intact antigen (i.e., the immunogen used to elicit the immune response) for binding to an antibody.

The terms "immunogen," "antigen," "immunogenic" and "antigenic" refer to any substance capable of generating antibodies when introduced into an animal. By definition, an immunogen must contain at least one epitope (the specific biochemical unit capable of causing an immune response), and generally contains many more. Proteins are most frequently used as immunogens, but lipid and nucleic acid moieties complexed with proteins may also act as immunogens. The latter complexes are often useful when smaller molecules with few epitopes do not stimulate a satisfactory immune response by themselves.

The term "antibody" refers to immunoglobulin evoked in animals by an immunogen (antigen). It is desired that the antibody demonstrates specificity to epitopes contained in the immunogen. The term "polyclonal antibody" refers to immunoglobulin produced from more than a single clone of plasma cells; in contrast "monoclonal antibody" refers to immunoglobulin produced from a single clone of plasma cells.

The terms "specific binding" or "specifically binding" when used in reference to the interaction of an antibody and a protein or peptide means that the interaction is dependent upon the presence of a particular structure (i.e., for example, an antigenic determinant or epitope) on a protein; in other words an antibody is recognizing and binding to a specific protein structure rather than to proteins in general. For example, if an antibody is specific for epitope "A", the presence of a protein containing epitope A (or free, unlabelled A) in a reaction containing labeled "A" and the antibody will reduce the amount of labeled A bound to the antibody.

The term "recombinant DNA molecule" as used herein refers to a DNA molecule which is comprised of segments of DNA joined together by means of molecular biological techniques.

The term "recombinant protein" or "recombinant polypeptide" as used herein refers to a protein molecule which is expressed using a recombinant DNA molecule.

As used herein, the terms "vector" and "vehicle" are used interchangeably in reference to nucleic acid molecules that transfer DNA segment(s) from one cell to another.

The term "expression vector" or "expression cassette" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals.

The terms "in operable combination", "in operable order" and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

The term "transfection" as used herein refers to the introduction of foreign DNA into cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, biolistics (i.e., particle bombardment) and the like.

As used herein, the terms "complementary" or "complementarity" are used in reference to "polynucleotides" and "oligonucleotides" (which are interchangeable terms that refer to a sequence of nucleotides) related by the base-pairing rules. For example, the sequence "C-A-G-T," is complementary to the sequence "G-T-C-A." Complementarity can be "partial" or "total." "Partial" complementarity is where one or more nucleic acid bases is not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. This is of particular importance in amplification reactions, as well as detection methods which depend upon binding between nucleic acids.

The terms "homology" and "homologous" as used herein in reference to nucleotide sequences refer to a degree of complementarity with other nucleotide sequences. There may be partial homology or complete homology (i.e., identity). A nucleotide sequence which is partially complementary, i.e., "substantially homologous," to a nucleic acid sequence is one that at least partially inhibits a completely complementary sequence from hybridizing to a target nucleic acid sequence. The inhibition of hybridization of the completely complementary sequence to the target sequence may be examined using a hybridization assay (Southern or Northern blot, solution hybridization and the like) under conditions o flow stringency. A substantially homologous sequence or probe will compete for and inhibit the binding (i.e., the hybridization) of a completely homologous sequence to a target sequence under conditions of low stringency. This is not to say that conditions of low stringency are such that non-specific binding is permitted; low stringency conditions require that the binding of two sequences to one another be a specific (i.e., selective) interaction. The absence of non-specific binding may be tested by the use of a second target sequence which lacks even a partial degree of complementarity (e.g., less than about 30% identity); in the absence of non-specific binding the probe will not hybridize to the second non-complementary target.

The terms "homology" and "homologous" as used herein in reference to amino acid sequences refer to the degree of identity of the primary structure between two amino acid sequences. Such a degree of identity may be directed a portion of each amino acid sequence, or to the entire length of the amino acid sequence. Two or more amino acid sequences that are "substantially homologous" may have at least 50% identity, preferably at least 75% identity, more preferably at least 85% identity, most preferably at least 95%, or 100% identity.

An oligonucleotide sequence which is a "homolog" of a *Clostridium* spore surface protein nucleic acid sequence (i.e., for example, SEQ ID NO: 2) is defined herein as an oligonucleotide sequence which exhibits greater than or equal to 50% identity to the sequence of SEQ ID NO: 2 when sequences having a length of 100 bp or larger are compared. Alternatively, a homolog of SEQ ID NO: 2 is defined as an oligonucleotide sequence which encodes a biologically active *Clostridium* spore surface protein amino acid sequence. For example, a *Clostridium* spore surface protein homolog may comprise a portion of an oligonucleotide sequence encoding a spore surface protein.

Low stringency conditions comprise conditions equivalent to binding or hybridization at 42°C in a solution consisting of 5 x SSPE (43.8 g/l NaCl, 6.9 g/l NaH₂PO₄-H₂O and 1.85 g/l EDTA, pH adjusted to 7.4 with NaOH), 0.1 % SDS, 5x Denhardt's reagent {50x Denhardt's contains per 500 ml: 5 g Ficoll (Type 400, Pharmacia), 5 g BSA (Fraction V; Sigma)} and 100 µg/ml denatured salmon sperm DNA followed by washing in a solution comprising 5x SSPE, 0.1% SDS at 42°C when a probe of about 500 nucleotides in length is employed. Numerous equivalent conditions may also be employed to comprise low stringency conditions; factors such as the length and nature (DNA, RNA, base composition) of the probe and nature ofthe target (DNA, RNA, base composition, present in solution or immobilized, etc.) and the concentration of the salts and other components (e.g., the presence or absence of formamide, dextran sulfate, polyethylene glycol), as well as components of the hybridization solution may be varied to generate conditions of low stringency hybridization different from, but equivalent to, the above listed conditions. In addition, conditions which promote hybridization under conditions of high stringency (e.g., increasing the temperature of the hybridization and/or wash steps, the use of formamide in the hybridization solution, etc.) may also be used.

As used herein, the term "hybridization" is used in reference to the pairing of complementary nucleic acids using any process by which a strand of nucleic acid joins with a complementary strand through base pairing to form a hybridization complex. Hybridization and the strength of hybridization (i.e., the strength ofthe association between the nucleic acids) is impacted by such factors as the degree of complementarity between the nucleic acids, stringency of the conditions involved, the Tₘ of the formed hybrid, and the G:C ratio within the nucleic acids.

As used herein the term "hybridization complex" refers to a complex formed between two nucleic acid sequences by virtue of the formation of hydrogen bounds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cₒ t or Rₒ t analysis) or between one nucleic acid sequence present in solution and another nucleic acid sequence immobilized to a solid support (e.g., a nylon membrane or a nitrocellulose filter as employed in Southern and Northern blotting, dot blotting or a glass slide as employed in in situ hybridization, including FISH (fluorescent in situ hybridization)).

As used herein, the term "Tₘ" is used in reference to the "melting temperature." The melting temperature is the temperature at which a population of double-stranded nucleic acid molecules becomes half dissociated into single strands. As indicated by standard references, a simple estimate of the Tₘ value may be calculated by the equation: Tₘ = 81.5 + 0.41 (% G+C), when a nucleic acid is in aqueous solution at 1M NaCl. Anderson et al., "Quantitative Filter Hybridization" In: Nucleic Acid Hybridization (1985). More sophisticated computations take structural, as well as sequence characteristics, into account for the calculation of Tₘ.

As used herein the term "stringency" is used in reference to the conditions of temperature, ionic strength, and the presence of other compounds such as organic solvents, under which nucleic acid hybridizations are conducted. "Stringency" typically occurs in a range from about Tm to about 20°C to 25°C below Tₘ. A "stringent hybridization" can be used to identity or detect identical polynucleotide sequences or to identity or detect similar or related polynucleotide sequences. For example, when fragments of SEQ ID NO: 2 are employed in hybridization reactions under stringent conditions the hybridization of fragments of SEQ ID NO: 2 which contain unique sequences (i.e., regions which are either non-homologous to or which contain less than about 50% homology or complementarity with SEQ ID NOs: 2) are favored. Alternatively, when conditions of "weak" or "low" stringency are used hybridization may occur with nucleic acids that are derived from organisms that are genetically diverse (i.e., for example, the frequency of complementary sequences is usually low between such organisms).

As used herein, the term "amplifiable nucleic acid" is used in reference to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of a target sequence of interest. In contrast, "background template" is used in reference to nucleic acid other than sample template which mayor may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

"Amplification" is defined as the production of additional copies of a nucleic acid sequence and is generally carried out using polymerase chain reaction. Dieffenbach C. W. and G. S. Dveksler (1995) In: PCR Primer, a Laboratory Manual, Cold Spring Harbor Press, Plainview, N.Y.

As used herein, the term "polymerase chain reaction" ("PCR") refers to the method of K.B. Mullis U.S. Pat. Nos. 4,683,195 and 4,683,202, herein incorporated by reference, which describe a method for increasing the concentration of a segment of a target sequence in a mixture of genomic DNA without cloning or purification. The length of the amplified segment of the desired target sequence is determined by the relative positions of two oligonucleotide primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified". With PCR, it is possible to amplify a single copy of a specific target sequence in genomic DNA to a level detectable by several different methodologies (e.g., hybridization with a labeled probe; incorporation ofbiotinylated primers followed by avidin-enzyme conjugate detection; incorporation of ³²P-labeled deoxynucleotide triphosphates, such as dCTP or dATP, into the amplified segment). In addition to genomic DNA, any oligonucleotide sequence can be amplified with the appropriate set of primer molecules. In particular, the amplified segments created by the PCR process itself are, themselves, efficient templates for subsequent PCR amplifications.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers; to an oligonucleotide (i.e., a sequence ofnucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used will be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the terms "restriction endonucleases" and "restriction enzymes" refer to bacterial enzymes, each of which cut double-stranded DNA at or near a specific nucleotide sequence.

DNA molecules are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. Therefore, an end of an oligonucleotide is referred to as the "5' end" if its 5' phosphate is not linked to the 3' oxygen of a mononucleotide pentose ring. An end of an oligonucleotide is referred to as the "3' end" if its 3' oxygen is not linked to a 5' phosphate of another mononucleotide pentose ring. As used herein, a nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements. This terminology reflects the fact that transcription proceeds in a 5' to 3' fashion along the DNA strand. The promoter and enhancer elements which direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

As used herein, the term "an oligonucleotide having a nucleotide sequence encoding a gene" means a nucleic acid sequence comprising the coding region of a gene, i.e. the nucleic acid sequence which encodes a gene product. The coding region may be present in a cDNA, genomic DNA or RNA form. When present in a DNA form, the oligonucleotide may be single-stranded (i.e., the sense strand) or double-stranded. Suitable control elements such as enhancers/promoters, splice junctions, polyadenylation signals, etc. may be placed in close proximity to the coding region of the gene if needed to permit proper initiation of transcription and/or correct processing of the primary RNA transcript. Alternatively, the coding region utilized in the expression vectors may contain endogenous enhancers/promoters, splice junctions, intervening sequences, polyadenylation signals, etc. or a combination of both endogenous and exogenous control elements.

As used herein, the term "regulatory element" refers to a genetic element which controls some aspect of the expression of nucleic acid sequences. For example, a promoter is a regulatory element which facilitates the initiation of transcription of an operably linked coding region. Other regulatory elements are splicing signals, polyadenylation signals, termination signals, etc.

Transcriptional control signals in eukaryotes comprise "promoter" and "enhancer" elements. Promoters and enhancers consist of short arrays of DNA sequences that interact specifically with cellular proteins involved in transcription. Maniatis, T. et al., Science 236:1237 (1987). Promoter and enhancer elements have been isolated from a variety of eukaryotic sources including genes in plant, yeast, insect and mammalian cells and viruses (analogous control elements, i.e., promoters, are also found in prokaryotes). The selection of a particular promoter and enhancer depends on what cell type is to be used to express the protein of interest.

The presence of "splicing signals" on an expression vector often results in higher levels of expression of the recombinant transcript. Splicing signals mediate the removal of introns from the primary RNA transcript and consist of a splice donor and acceptor site. Sambrook, J. et al.., In: Molecular Cloning: A Laboratory Manual, 2nd ed., Cold Spring Harbor Laboratory Press, New York (1989) pp. 16.7-16.8. A commonly used splice donor and acceptor site is the splice junction from the 16S RNA of SV40.

The term "poly A site" or "poly A sequence" as used herein denotes a DNA sequence which directs both the termination and polyadenylation of the nascent RNA transcript. Efficient polyadenylation of the recombinant transcript is desirable as transcripts lacking a poly A tail are unstable and are rapidly degraded. The poly A signal utilized in an expression vector may be "heterologous" or "endogenous." An endogenous poly A signal is one that is found naturally at the 3' end of the coding region of a given gene in the genome. A heterologous poly A signal is one which is isolated from one gene and placed 3' of another gene. Efficient expression of recombinant DNA sequences in eukaryotic cells involves expression of signals directing the efficient termination and polyadenylation of the resulting transcript. Transcription termination signals are generally found downstream of the polyadenylation signal and are a few hundred nucleotides in length.

The term "transfection" or "transfected" refers to the introduction of foreign DNA into a cell.

As used herein, the terms "nucleic acid molecule encoding", "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The DNA sequence thus codes for the amino acid sequence.

As used herein, the term "antisense" is used in reference to RNA sequences which are complementary to a specific RNA sequence (e.g., mRNA). Antisense RNA may be produced by any method, including synthesis by splicing the gene(s) of interest in a reverse orientation to a viral promoter which permits the synthesis of a coding strand. Once introduced into a cell, this transcribed strand combines with natural mRNA produced by the cell to form duplexes. These duplexes then block either the further transcription of the mRNA or its translation. In this manner, mutant phenotypes may be generated. The term "antisense strand" is used in reference to a nucleic acid strand that is complementary to the "sense" strand. The designation (-) (i.e., "negative") is sometimes used in reference to the antisense strand, with the designation (+) sometimes used in reference to the sense (i.e., "positive") strand.

The term "Southern blot" refers to the analysis of DNA on agarose or acrylamide gels to fractionate the DNA according to size, followed by transfer and immobilization of the DNA from the gel to a solid support, such as nitrocellulose or a nylon membrane. The immobilized DNA is then probed with a labeled oligodeoxyribonucleotide probe or DNA probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists. J. Sambrook et al. (1989) In: Molecular Cloning: A Laboratory Manual, Cold Spring Harbor Press, NY, pp 9.31-9.58.

The term "Northern blot" as used herein refers to the analysis of RNA by electrophoresis of RNA on agarose gels to fractionate the RNA according to size followed by transfer of the RNA from the gel to a solid support, such as nitrocellulose or a nylon membrane. The immobilized RNA is then probed with a labeled oligodeoxyribonucleotide probe or DNA probe to detect RNA species complementary to the probe used. Northern blots are a standard tool of molecular biologists. J. Sambrook, J. 20 et al. (1989) *supra,* pp 7.39-7.52.

The term "reverse Northern blot" as used herein refers to the analysis of DNA by electrophoresis of DNA on agarose gels to fractionate the DNA on the basis of size followed by transfer of the fractionated DNA from the gel to a solid support, such as nitrocellulose or a nylon membrane. The immobilized DNA is then probed with a labeled oligoribonuclotide probe or RNA probe to detect DNA species complementary to the ribo probe used.

As used herein the term "coding region" when used in reference to a structural gene refers to the nucleotide sequences which encode the amino acids found in the nascent polypeptide as a result of translation of a mRNA molecule. The coding region is 30 bounded, in eukaryotes, on the 5' side by the nucleotide triplet "ATG" which encodes the initiator methionine and on the 3' side by one of the three triplets which specify stop codons (i.e., TAA, TAG, TGA).

As used herein, the term "structural gene" refers to a DNA sequence coding for RNA or a protein. In contrast, "regulatory genes" are structural genes which encode products which control the expression of other genes (e.g., transcription factors).

As used herein, the term "gene" means the deoxyribonucleotide sequences comprising the coding region of a structural gene and including sequences located adjacent to the coding region on both the 5' and 3' ends for a distance of about 1 kb on either end such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into heterogeneous nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In addition to containing introns, genomic forms of a gene may also include sequences located on both the 5' and 3' end of the sequences which are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers which control or influence the transcription of the gene. The 3' flanking region may contain sequences which direct the termination of transcription, posttranscriptional cleavage and polyadenylation.

The term "sample" as used herein is used in its broadest sense and includes environmental and biological samples. Environmental samples include material from the environment such as soil and water. Biological samples may be animal, including, human, fluid (e.g., blood, plasma and serum), solid (e.g., stool), tissue, liquid foods (e.g., milk), and solid foods (e.g., vegetables). A biological sample suspected of containing nucleic acid encoding a spore surface protein may comprise a cell, tissue extract, body fluid, chromosomes or extrachromosomal elements isolated from a cell, genomic DNA (in solution or bound to a solid support such as for Southern blot analysis), RNA (in solution or bound to a solid support such as for Northern blot analysis), cDNA (in solution or bound to a solid support) and the like.

The term "infection control composition" refers to any compound or compounds which prevent and/or reduce the rate of growth of an organism (i.e., for example, a microbial organism, including spores derived from a microbial organism) compared to the rate of growth of the organism in the absence of the composition. An infection control composition can be natural (e.g., derived from a microbe such as a bacteria, virus, fungi, algae, mold etc.), synthetic, or recombinant. An infection control composition may be a competitive inhibitor of a microbial spore protein and/or a microbial spore surface protein. Alternatively, an infection control composition may form stable and/or transient binding pairs with microbial spore proteins and/or microbial spore surface proteins. An 'infection control composition" may also include antibacterial and/or antimicrobial agents and/or drugs (i.e., for example, antibiotics). Consequently, "infection control activity" is interpreted to mean any reduction and/or prevention of microbial growth as a result of the application of an infection control composition to a surface.

The terms "antibacterial", and "antimicrobial" are used interchangeably to refer to a composition which prevents and/or reduces the rate of growth of an organism compared to the rate of growth of the organism in the absence of the composition. An antibacterial and/or antimicrobial composition can be bacteriostatic, bactericidal, both, or neither. An antibacterial and/or antimicrobial composition is bacteriostatic if it inhibits cell division without affecting the viability of the inhibited cell. An antibacterial and/or antimicrobial composition is bactericidal if it causes cell death. Cell death is commonly detected by the absence of cell growth in liquid growth medium (e.g., absence of turbidity) or on a solid surface (e.g., absence of colony formation on agar). An antibacterial and/or antimicrobial composition may be effective to reduce the growth rate and/or cause cell death for viruses, molds, and fungi. A composition which is bacteriostatic at a given concentration may be bactericidal at a higher concentration, while other certain bacteriostatic compositions are not bactericidal at any concentration.

The term "bacteria" and "bacterium" refer to all prokaryotic organisms, including those within all of the phyla in the Kingdom Procaryotae. It is intended that the term encompass all microorganisms considered to be bacteria including, but not limited to, *Clostridia, Bacillus, Mycoplasma, Chlamydia, Actinomyces, Streptomyces,* and *Rickettsia.* All forms of bacteria are included within this definition including cocci, bacilli, spirochetes, spheroplasts, protoplasts, spores etc. Also included within this term are prokaryotic organisms which are gram negative or gram positive. "Gram negative" and "gram positive" refer to staining patterns with the Gram-staining process. Finegold and Martin, In: Diagnostic Microbiology, 6th Ed. (1982), C. V. Mosby St. Louis, pp 13-15. "Gram positive bacteria" are bacteria which retain the primary dye used in the Gram stain, causing the stained cells to appear dark blue to purple under the microscope. "Gram negative bacteria" do not retain the primary dye used in the Gram stain, but are stained by a counter stain. Thus, gram negative bacteria appear red.

The term "test agent" refers to an agent that is to be screened in one or more of the assays described herein. The agent can be virtually any chemical compound. It can exist as a single isolated compound or can be a member of a chemical (e.g., combinatorial) library. In a particularly preferred embodiment, the test agent will be a small organic molecule.

The term "small organic molecule" as used herein, refers to any molecule of a size 15 comparable to those organic molecules generally used in pharmaceuticals. The term excludes biological macromolecules (e.g., proteins, nucleic acids, etc.). Preferred small organic molecules range in size from approximately 10 Da up to about 5000 Da, more preferably up to 2000 Da, and most preferably up to about 1000 Da.

The term "label" or "detectable label" are used herein, to refer to any composition detectable by spectroscopic, photochemical, biochemical, immunochemical, electrical, optical or chemical means. Such labels include biotin for staining with labeled streptavidin conjugate, magnetic beads (e.g., Dynabeads®), fluorescent dyes (e.g., fluorescein, texas red, rhodamine, green fluorescent protein, and the like), radiolabels (e.g., ³H, ¹²⁵I, ³⁵S, ¹⁴C, or ³²P), enzymes (e.g., horse radish peroxidase, alkaline phosphatase and others commonly used in an ELISA), and calorimetric labels such as colloidal gold or colored glass or plastic (e.g., polystyrene, polypropylene, latex, etc.) beads. Patents teaching the use ofsuch labels include, but are not limited to, U.S. Pat. Nos. 3,817,837; 3,850,752; 3,939,350; 3,996,345; 4,277,437; 4,275,149; and 4,366,241 (all herein incorporated by reference). The labels contemplated in the present invention may be detected by many methods. For example, radiolabels may be detected using photographic film or scintillation counters, fluorescent markers may be detected using a photodetector to detect emitted light. Enzymatic labels are typically detected by providing the enzyme with a substrate and detecting, the reaction product produced by the action of the enzyme on the substrate, and calorimetric labels are detected by simply visualizing the colored label.

The term "primary surfactant" as used herein, means any surfactant which acts upon or in conjunction with a substituted phenol to enhance further, or at the least, to not significantly reduce infection control activity of a substituted phenol.

The term "inhibit", "disrupt", "displace", "antagonize", or "block" as used herein refer to any means wherein a microbial spore and/or microbial spore surface protein is removed from, and/or prevented from attaching to, a substrate surface.

The term "antagonist" as used herein, refers to any compound that is capable of preventing the attachment of a microbe (i.e., for example, bacteria, virus, mold, fungi etc) and/or a reproductive component of the microbe (i.e., for example, a spore) to a surface. The 'antagonist' may prevent attachment by either competitive displacement or by forming a binding pair (i.e., for example, stable or transient) with the microbe and/or reproductive component of the microbe. For example, an antagonist may prevent the attachment of a bacterial spore to a surface by binding to a spore surface protein.

The term "binding antagonist" as used herein, refers to any compound that is capable of forming a binding pair with either a microbial spore nap layer protein (i.e., for example, a *Clostridium* spore surface protein) or a substrate surface. A formation of either binding pair prevents and/or displaces the attachment of a microbial spore to a substrate surface.

The term "substrate surface" as used herein, refers to any material comprising a binding site for a microbial spore surface protein attachment. A substrate surface may including but not limited to, an animate surface and/or an inanimate surface.

The term "inanimate surface" as used herein, refers to any non-living material. For example, an inanimate surface may include but is not limited to, vinyl, stainless steel, plastic, wood, ceramic, glass, chrome, tile etc. Such surfaces commonly encountered including but not limited to countertops, chairs, tables, surgical equipment, medical devices, floors, walls, windows, sinks, cabinets etc.

The term "animate surface" as used herein, refers to any living material. For example, an animate surface may include but is not limited to, epidermal skin surfaces, mucosal epithelial tissue surfaces, and/or internal organ surfaces. As used herein, the term "animate surface" also comprises vegetative surfaces including but not limited to, fruit surfaces (i.e., for example, strawberries, apples, oranges, tangerines etc.), vegetable surfaces (eggplant, squash, tomatoes, potato etc), legume surfaces (i.e., for example, peanuts, cashews, beans etc), grain surfaces (i.e., for example, wheat, soy, rice, rye etc), flower surfaces, stem surfaces, stalk surfaces, leaf surfaces, bark surfaces, limb surfaces, branch surfaces, etc.

The term "interaction antagonist" as used herein, refers to any compound that is capable of interfering with the formation of a microbial spore nap layer protein/substrate surface binding pair.

The term "binding" as used herein, refers to any interaction between an infection control composition and a surface. Such as surface is defined as a "binding surface". Binding may be reversible or irreversible. Such binding may be, but is not limited to, non-covalent binding, covalent bonding, ionic bonding, Vande Waal forces or friction, and the like. An infection control composition is bound to a surface if it is impregnated, incorporated, coated, in suspension with, in solution with, mixed with, etc.

The term "infection control test composition", as used herein, refers to the collection of compounds that are to be screened for their ability to prevent microbial spore binding to a surface. Such test compositions may include, but are not limited to, a wide variety of different compounds, including chemical compounds, mixtures of chemical compounds, e.g., polysaccharides, small organic or inorganic molecules, biological macromolecules, e.g., peptides, proteins, nucleic acids, or an extract made from biological materials such as bacteria, plants, fungi, or animal cells or tissues, naturally occurring or synthetic compositions.

The term "at risk" as used herein, refers to any person or inanimate surface having a more than average probability (i.e., greater than 50%) of becoming contaminated with a microbe. 'At risk' probabilities of exposure may be found in vocations such as first responders including, but not limited to, fireman, policeman, emergency medical personnel. 'At risk' probabilities of exposure may also be found in vocations such as healthcare workers including, but not limited to, doctors, nurses, orderlies, hospital janitorial staff and the like. 'At risk' probabilities of exposure may also be found in vocations such as military personnel including, but not limited to, Army personnel, Navy personnel, Air Force personnel, Marine personnel, National Guard personnel, Coast Guard personnel. 'At risk' probabilities of exposure may also be found in medical patients, including, but not limited to, immunocompromised patients, bum patients, gunshot patients etc.

The term "specific binding site pattern" as used herein refers to any reproducible set of binding sites on either a body surface or inanimate surface. Such binding sites have inherent affinity for compositions including, but not limited to, microbial spores, infection control compositions, or small organic molecules as discussed herein. Although it is not necessary to understand the mechanism of an invention, it is believed that such a binding pattern is a result of unique arrangements of surface chemical groups and/or topography that is three-dimensionally compatible (i.e., for example, via stereospecific chemical group interactions) with a composition. It is further believed that a specific binding site pattern predicts an arrangement of non-specific binding (i.e., that binding not mediated via a dedicated receptor) within a designated surface area.

### Brief Description Of The Figures

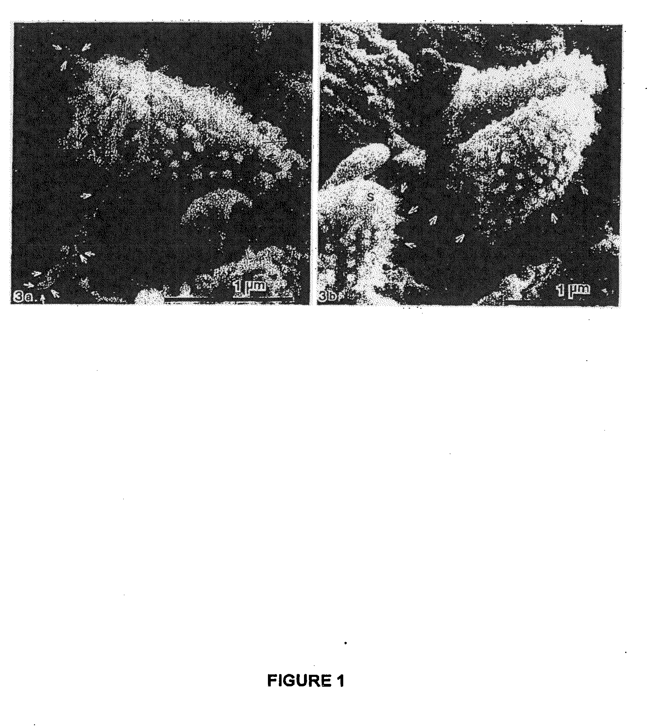

Figure 1 presents an exemplary scanning electron micrograph of a C*. sporogens* endospores (S) having long, sinuous, or filifonn exosporial membrane projections (arrows). Projections facing the surface form tenacious contacts.
Figure 2 presents an exemplary electron micrograph showing C*. difficile 43594* smooth-surfaced, elliptical endospore before activation (insert), and covered with exosporial projections (arrows) following activation.
Figure 3 presents an exemplary electron micrograph showing a C*. difficile 43594* spore as germination proceeds wherein an exosporial membrane is covered with low bumps and a thick anchoring structure at one pole, designated the tail (T) region.
Figure 4 presents exemplary data showing an SDS-PAGE gel identifying the total spore proteins present in the 18 kDa band (see arrow). This 18 kDa band corresponds with Western Blot 18kDa bands capable of hybridizing C. *difficile* antibodies.
Figure 5 presents exemplary date showing an SDS-PAGE gel identifying the total spore proteins present I he 140, 10, ad 10 kDa bands (see p1, p2, and p3, respectively). These 140, 150, and 10 kDa bands coresond with Western Blot 140, 150, and 160 kDa bands capable of hybridizing C. *difficile* antibodies.
Figure 6A presents exemplary data showing an SDS-PAGE gel used to identify C. *difficile* exosporium proteins at approximately 300 kDa (arrow 1); 275 kDa (arrow 2); 250 kDa (arrow 3); 160 kDa (arrow 4); 150 kDa (arrow 5); 140 kDa (arrow 6); 35 kDa 30 (arrow 7); 28 kDa (arrow 8); and 15 kDa (arrow 9). Six other non-hybridizing bands (arrows 10-15) were also identified to determine the non-immunoreactive exosporium proteins.
Figures 6B and 6C present exemplary data showing Western blots demonstrating C. *difficile* antibody hybridization to 300 kDa (arrow 1); 275 kDa (arrow 2); 250 kDa (arrow 3); 160 kDa (arrow 4); 150 kDa (arrow 5); 140 kDa (arrow 6); 35 kDa (arrow 7); 28 kDa (arrow 8); and 15 kDa (arrow 9) bands corresponding to those bands shown in Figure 6A.

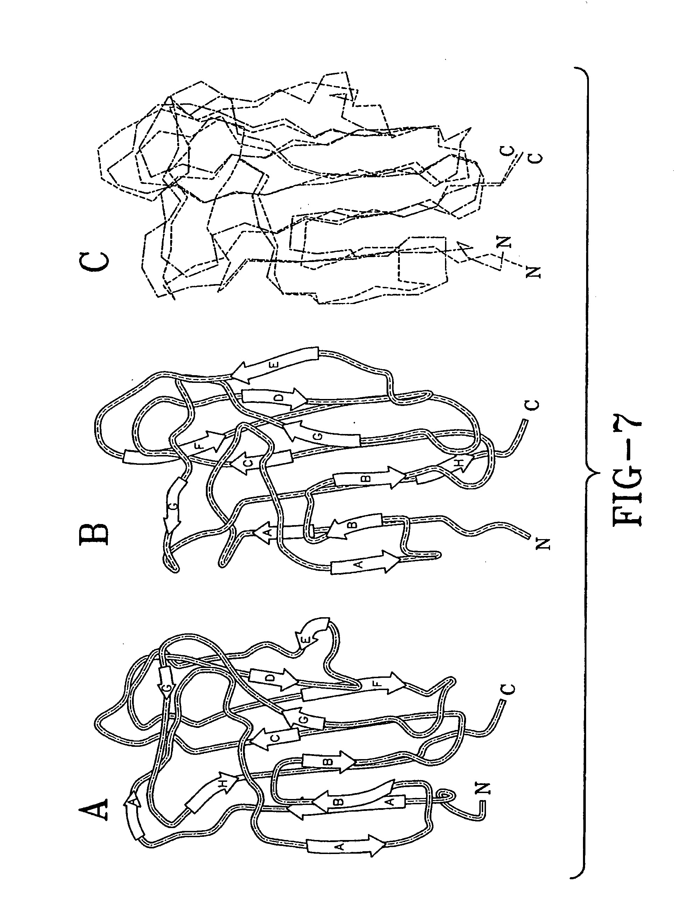

Figure 7 presents one embodiment of a structure for: BclA (green) and Clq (mauve) monomers (A and B), respectively and their superposition (C). The N and C termini and the β-strands are labeled.

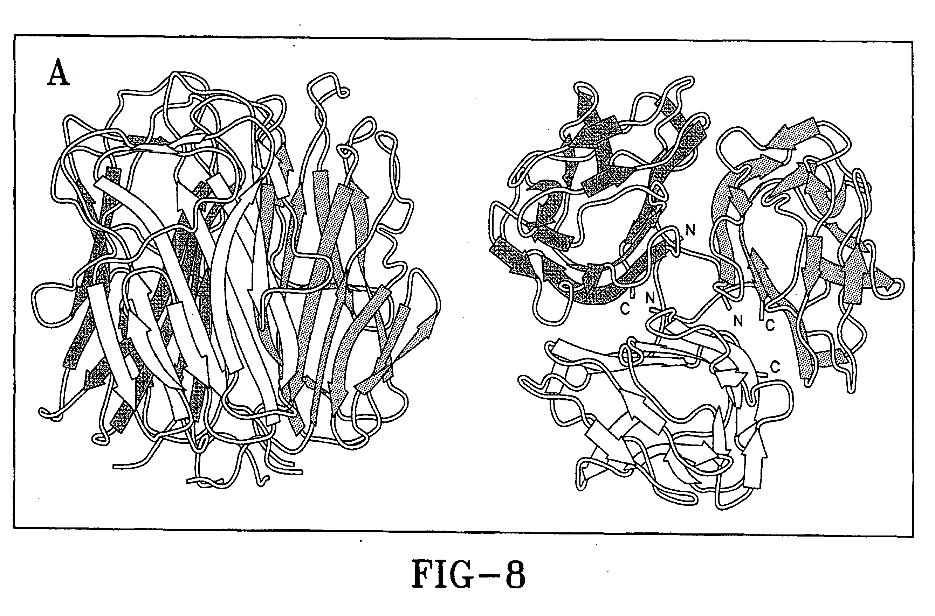

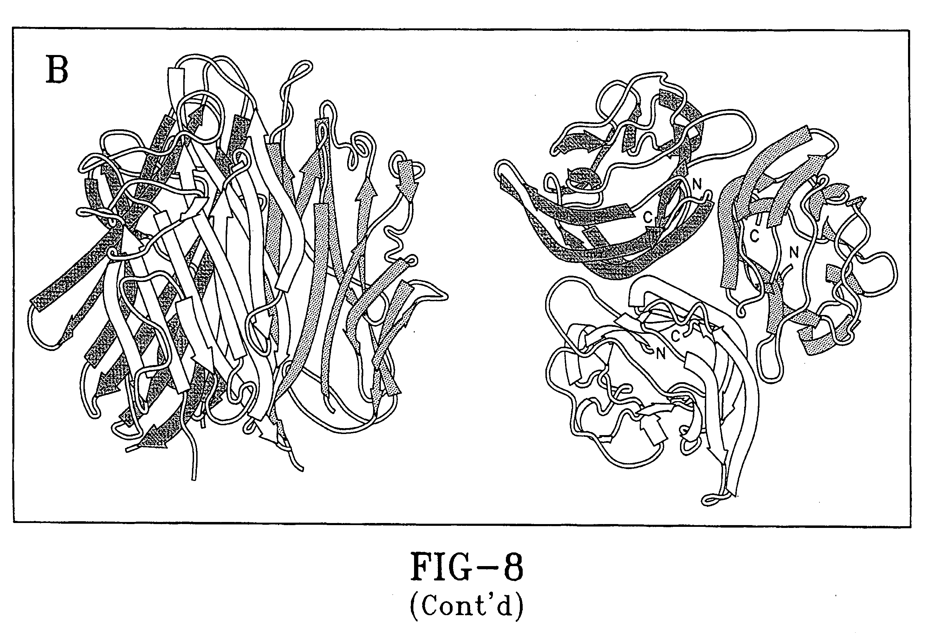

Figure 8 presents one embodiment of BelA (A) and Clq (B) trimers viewed from the side and from above. The three monomers comprising each trimer are colored blue, green, and red.

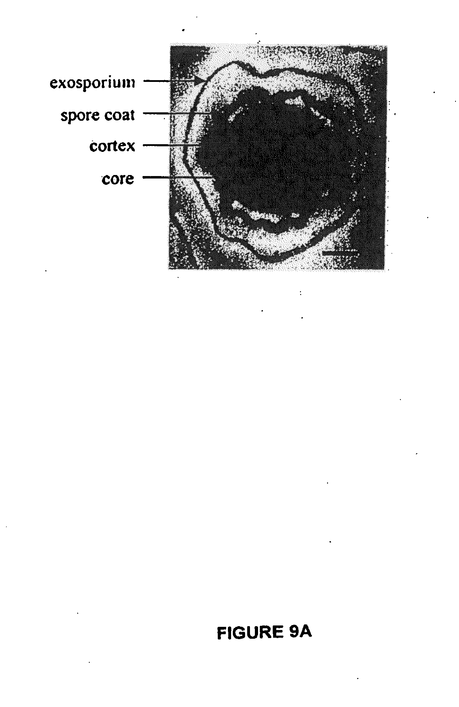

Figure 9A presents an exemplary transmission electron micrographs of a *B. cereus* ATCC 10876 spore showing the exosporium.

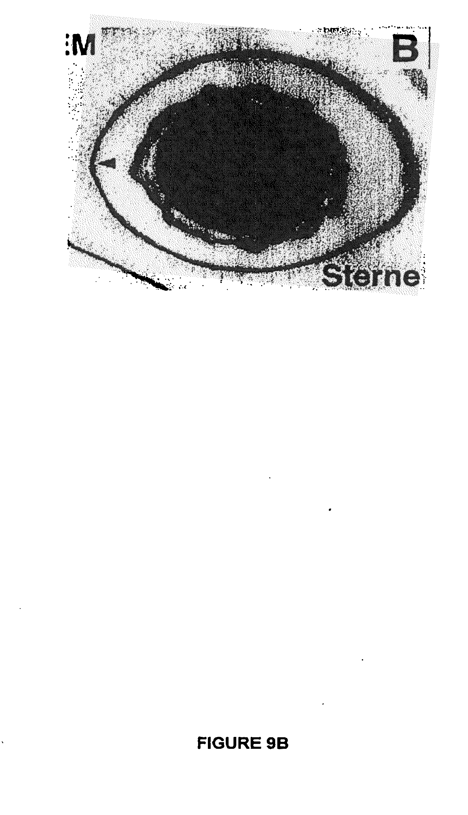

Figure 9B presents an exemplary transmission electron micrograph of a *B. anthracis* spore showing a nap layer projecting from the exosporium.

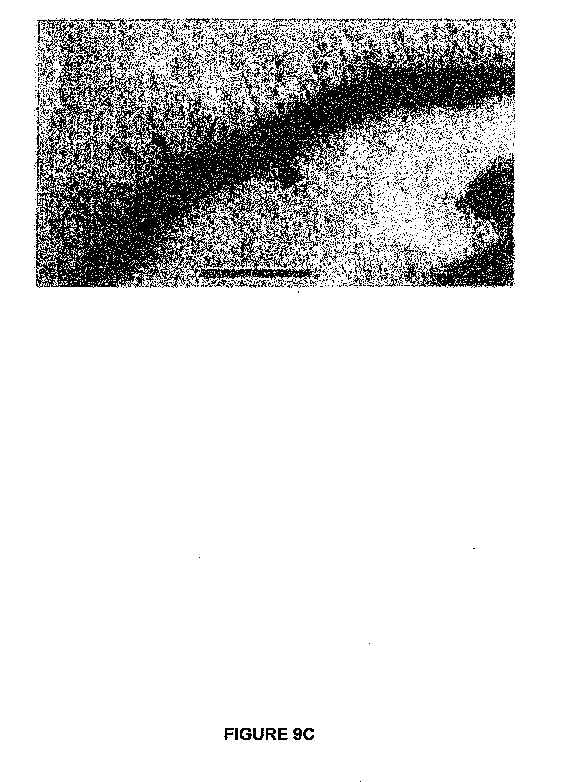

Figure 9C presents an exemplary close-up of a *B. anthracis* exosporium layer showing the BclA nap layer and the interior basal layer.
Figure 10 presents an illustration of embodiments showing microbial spore interactions with surface substrates (i.e., for example, skin surfaces).
Figure 10A illustrates the attachment of a microbial spore nap layer (i.e., for example, by spore surface protein projections) to the epithelial layer of mammalian skin.
Figure 10B illustrates the attachment of a microbial spore surface protein (i.e., for example a native to a surface substrate and various infection control compositions capable of disrupting the attachment of the microbial spore surface proteins to a surface substrate.
Figure 11 presents a schematic of one embodiment of a pET-19b plasmid comprising a spore surface protein gene.
Figure 12 presents exemplary data showing an SDS-PAGE electrophoretic separation of recombinant CD 1067 (red arrow) expression from a pET-19b vector. Lane 1: MW standards ladder. Lane 2: 1 hour sample. Lane 3: 2 hour sample. Lane 4: 3 hour sample: 30 Lane 5: 4 hour sample: Lane 6: 5 hour sample. Lane 7: 6 hour sample. Protein Stain: Coomassie Blue.
Figure 13 presents exemplary data showing Western Blot analysis of recombinant CD1067 (red arrows) expression from a pET-19b vector. Lane 1: MW standards ladder. Lane 2: 1 hour sample. Lane 3: 2 hour sample. Lane 4: 3 hour sample: Lane 5: 4 hour sample: Lane 6: 5 hour sample. Lane 7: 6 hour sample.
Figure 13A: Detection using F1373 anti-Co *difficile* antibody.
Figure 13B: Detection using anti-HIS antibody.
Figure 14 presents exemplary data showing an SDS-PAGE electrophoretic separation of recombinant CD3620 (red arrow) expression from a pET-19b vector. Lane 1: MW standards ladder. Lane 2: 0 hour sample. Lane 3: 1 hour sample. Lane 4: 2 hour sample: Lane 5: 3 hour sample: Lane 6: 4 hour sample. Lane 7: 4.5 hour sample. Protein Stain: Coomassie Blue.
Figure 15 presents exemplary data showing Western Blot analysis of recombinant CD3620 (red arrows) expression from a pET-19b vector. Lane 1: MW standards ladder. Lane 2: 0 hour sample. Lane 3: 1 hour sample. Lane 4: 2 hour sample: Lane 5: 3 hour sample: Lane 6: 4 hour sample. Lane 7: 4.5 hour sample.
Figure 15A: Detection using F1373 anti-Co *difficile* antibody.
Figure 15B: Detection using anti-HIS antibody.
Figure 16 presents exemplary data showing antibody detection control data for recombinant CD3620 (red arrows) expression from a pET-19b vector. Lane 1: MW standards ladder. Lane 2: 6 hour pJEB02 sample. Lane 3: 4.5 hour pJEB03 sample.
Figure 16A: SDS-PAGE Coomassie Blue reference electrophoresis separation identifying recombinant CD1067 (top red arrow) and recombinant CD3620 (bottom red arrow).
Figure 16B: Western Blot analysis using preimmune F1373 detection.
Figure 16C: Western Blot analysis using preimmune F1997 detection.
Figure 16D: Western Blot analysis using F1997 anti-Co *difficile* antibody detection.
Figure 17 illustrate several embodiments of a method to remove a C. *diffcile* exosporium coat (see, Example IV).
Figure 18A depicts a representative C. *difficile* 630 spore that may be used as starting material.
Figure 18B depicts a representative C. *difficile* 630 spore after exosporium stripping.
Figure 18C depicts a purified and concentrated representative C. difficile 630 spore after exoporium after stripping.

### Detailed Description

The present disclosure is related to the field of infection control. Particularly, there are described compositions and methods for maintenance of antiseptic conditions, bacterial decontamination, and/or bacterial exposure prophylaxis. In one embodiment, there are described infection control compositions that inhibit microbial spore binding to a substrate surface. For example, the inhibition disrupts and/or displaces an interaction, attachment, and/or stabilization of a microbial spore to a substrate surface.

For approximately 70 years, the first line of defense against bacterial infection has been antibiotics. The first antibiotics were used clinically in the 1940s and 1950s, and their use has been increasing significantly since this period. Although an invaluable advance, antibiotic and antimicrobial therapy suffers from several problems. First, antibiotics are ineffective against spores. Second, the development of antibiotic resistance is a serious and life-threatening event of worldwide importance. For example, some microbial strains are known to be immune to a majority of available antibiotics. (Travis, Science 264:360-362 (1994). Among other drug resistant organisms are: pneumococci that cause pneumonia and meningitis; *Cryptosporidium* and *E. coli* that cause diarrhea; and enterococci that cause blood-stream, surgical wound and urinary tract infections. (Berkelman et. al., J Infect Dis. 170:272-277 (1994). The present invention contemplates compositions and methods directed towards solving problems regarding contamination control of microbial spores and the clinical resistance of microbial infection to conventional antibiotic administration.

### I. Bacterial Spores

Some microbes shed spores that enter a dormant phase when exposed to environments that do not favor growth and proliferation. Spore survival is enhanced when attachment and/or adhesion to a solid surface is made. When the environmental conditions again change to favor growth and proliferation the microbial spore germinates and proceeds to contaminate the surrounding area. A person or animal coming into contact with the proliferating new microbial growth then becomes infected from an area previously unknown to be contaminated. In some cases, a person and/or animal may come into contact with the microbial spore wherein germination and infection proceeds after contact. Alternatively, such undetected spore germination can result in food spoilage thereby initiating food-borne illness outbreaks. Because of their relative resistance to chemical and physical sterilization agents, spores of these bacteria are used as indicators of sterilization efficiency for treatments involving moist and dry heat, UV irradiation, and hydrogen peroxide. Ito et al., "Sterilization of packaging materials using aseptic systems" Food Technol. 38:60-62 (1984).

An understanding the surface properties of bacterial spores and their interactions with inanimate and animate substrates is important for selection of packaging materials and for evaluation of the surface sterilization procedures used in the packaging of food, pharmaceutical agents, and medical supplies. For example, the role of hydrophobic interactions in the adhesion of bacteria (i.e., the vegetative cell) to the surfaces of inert materials is well accepted. Relatively few studies, however, have thoroughly examined the surface hydrophobicity of bacterial spores or the adhesion of spores to inanimate substrata. The hydrophobicity of bacterial spores can be determined by applying methodologies used for measuring vegetative cell hydrophobicity. Established techniques for measuring surface hydrophobicity include, but are not limited to: i) adherence to hydrocarbons (Beck et al., "Effect of growth on surface charge and hydrophobicity of Staphylococcus aureus" Ann. Inst. PasteurlMicrobiol. (Paris) 139:655-664 (1988)); ii) hydrophobic interaction chromatography (HIC) (Doyle et al., "Hydrophobic characteristics of Bacillus spores" Curro Microbiol. 10:329-332 (1984); iii) salt aggregation (Takubo et al., "Isolation and characterization of outermost layer deficient mutant spores of Bacillus megaterium" Microbiol.Immunol. 9:973-979 (1988); iv) contact angle measurements (Minagi et al., "Cell-surface hydrophobicity of Candida species as determined by the contact-angle and hydrocarbon-adherence methods" J Gen. Microbial. 132:1111-1115 (1986)); and v) bacterial adherence to hexadecane (BATH) (Wienek et al., Hydrophobicity of Bacillus and Clostridium spores" Appl Environ Microbial 56:2600-2605 (1990).

>

Studies demonstrate that spore hydrophobicity varies among species and strains. However, for each organism, the hydrophobicity of spores is believed to be greater than the vegetative cell hydrophobicity. Further, moderate heat treatment increases the hydrophobicity of most *Bacillus* spores. One hypothesis suggests that increases in spore hydrophobicity following heat treatment may result from the disruption of outer coat or exosporium proteins.

Although it is not necessary to understand the mechanism of an invention, it is believed that the increased hydrophobicity of bacterial spores is due to the relative abundance of protein in the outer coats and exosporium as compared with peptidoglycan on vegetative cells. Doyle et al., "Hydrophobic characteristics of Bacillus spores" Curro Microbiol. 10:329-332 (1984); Matz et al., "Chemical composition of exosporium from spores of Bacillus cereus" J. Bacteriol. 101 :196-201 (1970); and Takumi et al., "Isolation and partial characterization of exosporium from spores of a highly sporogenic mutant ofClostridium botulinum type A" Microbial. Immunol. 28:443-454 (1979). An association between spore hydrophobicity and the presence of an exosporium has been reported recently for several Bacillus species. Kjelleberg, S. "Adhesion to inanimate surfaces" In: Microbial adhesion and aggregation, pp. 51-70. K. C. Marshall (ed.), Springer-Verlag KG, Berlin (1984). Decreased spore hydrophobicity ofB. cereus T spores was observed of when spore surfaces were removed by chemical treatments. Kutima et al., "Involvement of the spore surface in germination of Bacillus cereus T spores" Appl. Environ. Microbiol. 53:47-52 (1987). Decreases in spore hydrophobicities have also been seen in "knock-out" outer-coat-negative mutants of *B. megaterium* (QMB1551: ATCC 12872). Takubo et al., "Isolation and characterization of outermost layer deficient mutant 15 spores of Bacillus megaterium" Microbiol. Immunol. 9:973-979 (1988); and Koshikawa et al., "Surface hydrophobicity of spores of Bacillus spp." J. Gen. Microbial. 135:27172722 (1989). These observations suggest that the outer coats of the exosporium playa role in spore hydrophobicity.

There are described compositions and methods to improve surface sanitation, sterilization of equipment and/or packaging materials used in the medical, pharmaceutical, and/or food industries. In one embodiment, there are described compositions and methods to improve skin sanitation.

### A. Clostridium difficile Spore Surface Proteins

Bacteria from the genus *Clostridium,* are thought to produce such diseases including, but not limited to, gas gangrene, wound infections, botulism, tetanus, pseudomembranous colitis, septicemia, or nosocomial diarrhea. This genus survives well under adverse conditions by forming dehydrated endospores that are highly resistant to heat, chemicals, and radiation. These spores can be found in soil, carried by the wind, recovered from mammalian digestive and urogenital tracts, and have the ability to remain dormant for very extended periods of time until conditions for growth support spore activation and germination.

C. *difficile* spores are prevalent within healthcare and hospital environments. C. *difficile* spores survive on animate and/or inanimate surfaces for a prolonged period, are resistant to many disinfectants and antibiotics, and are easily transmitted from patient to patient. Understanding C. *difficile* spore attachment to surfaces, as well as sporulation and germination, will help in the prevention, control, and treatment of C. *difficile* associated disease. For example, C. *difficile* lacks several genes that are involved in the phospho-relay system that triggers the sporulation process in *Bacillus* species; this, and other aspects of the sporulation system in *Clostridia spp* have recently been reviewed. Paredes et al., "A comparative genomic view of clostridial sporulation and physiology" Nat. Rev. Microbiol. 3:969-978 (2005). Further, C. *difficile* also lacks a tricistronic *ger* operon that are usually present in most *Bacillus* and *Clostridium* species. Moir et al., "Spore germination" Cell Mol Life Sci. 59:403-409 (2002); and Broussolle et al., "Molecular and physiological characterisation of spore germination in Clostridium botulinum and C. sporogenes" Anaerobe 8:89-100 (2002). These observations suggest that the C. *difficile* genninant receptors are substantially different from, and should not be identified on the basis of sequence similarity to, other *Clostridium spp.* and *Bacillus spp.*

Microbial spore resistance to heat, solvents, enzymes, detergents, ultraviolet and ionizing radiation, sodium hydroxide, ethanol, performic acid, phenol, cold, and most sporicides, as well as their ability to grow aggressively when conditions are optimum, make them a serious threat to animal and especially human health. Tipper et al., "Structure of the bacterial endospore" In: Spores V, Ed. H. Halvorson, R. Hanson and L. Campbell. Amer. Soc. for Microbiol., Bethesda, Maryland, 3-12 (1972); and Russell, A., "Bacterial spores and chemical sporicidal agents" Clin. Microbiol. Rev., 3, 99-119 (1990). Nosocomial acquisition of *Clostridium difficile* infection in hospitals and nursing homes presents a serious epidemiologic problem because spores are found on walls, floors, utensils, bed sheets, and on the hands of caregivers in health facilities. The spores easily seem to infect individuals including, but not limited to, those who have: i) an altered intestinal flora from antibiotic therapy; ii) a compromised immune system, or iii) require multiple enemas in relation to diagnostic testing or treatment. Zaleznik, D. "Clostridium difficile: an important nosocomial pathogen for the 1990s" Clin. Microbiol. Newsletter 13, 145-152 (1991).

The exosporia from two species of *Clostridium* spores (i.e., for example, *Clostridium sporogenes* ATCC 3584 and C. *difficile* ATCC 9689 and ATCC 43594) may playa role in attachment, germination, and/or colonization. Panessa-Warren et al.., "Exosporial membrane plasticity of Clostridium sporogens and Clostridium difficile" Issue & Cell 29:449-461 (1997). Ultrastructural change was associated with germination and outgrowth of C. *bifermentans* spore appendages but no specific function was determined. Samsonoff et al., "Ultrastructural changes associated with germination and outgrowth of an appendage-bearing clostridial spore" J Bacteriol., 101, 1038-1045 (1970). Examination ofC. *sporogenes* spores by thin-section transmission electron microscopy did reveal an exosporial outer layer, with hair-like projections, but no discussion regarding distribution, function, or appearance in relation to *Clostridium* colonization was offered. Hoeniger et al., "Ultrastructural aspects of spore germination and outgrowth in Clostridium sporogenes" Can. J Microbiol., 15, 1061-1065 (1969). Transmission electron microscopy thin-section reconstruction is known to have significant problems when observing and interpreting an exosporial morphology of numerous spores at one time, and the problems associated with obtaining thin-section slices of such small, delicate, and poorly staining, spore attachments to a nutritive substrate.

C. *sporogenes* spores may exhibit specific morphological stages once dormancy is broken, and that the exosporium seems to play an active role in successful germination, outgrowth, vegetative proliferation, and colonization of a substrate surface binding site. Panessa-Warren et aI., "Electron microscopy of C. sporogenes endospore attachment and germination" Scanning 16, 227-240 (1994). A *Clostridium* exosporium is believed to be an outer 'membranous' layer of a spore, has been described as a passive participant in germination. However, once dormancy is broken, C. *sporogenes* ATCC 3584 spores appear to produce very specific exosporial morphological structures visible by scanning electron microscopy (SEM), which seem to facilitate attachment of a spore to a substrate or to other spores (i.e., for example, spore co-aggregation). Exosporial projections are later replaced by a single stalk extending from the distal (i.e., for example, the tail region) of the spore, which appears to anchor the spore during the final stages of vegetative cell development and outgrowth.

During the initial stages of C. *sporogenes* attachment, delicate filiform projections of the exosporial membrane extend outward with some of the shorter projections attaching to a surface. See, Figure I, white arrows. Usually long filamentous projections from the exosporial membrane originate from the top surface or sides of the spore, and 30 extend to either the surface or to a nearest neighboring spores, binding the spores together (co-aggregation). Exosporial membrane projections appear all over the outer surface of a C. *sporogenes* activated spore eventually developing into a single, thick, attachment structure extending to a surface, appearing to anchor the spore.

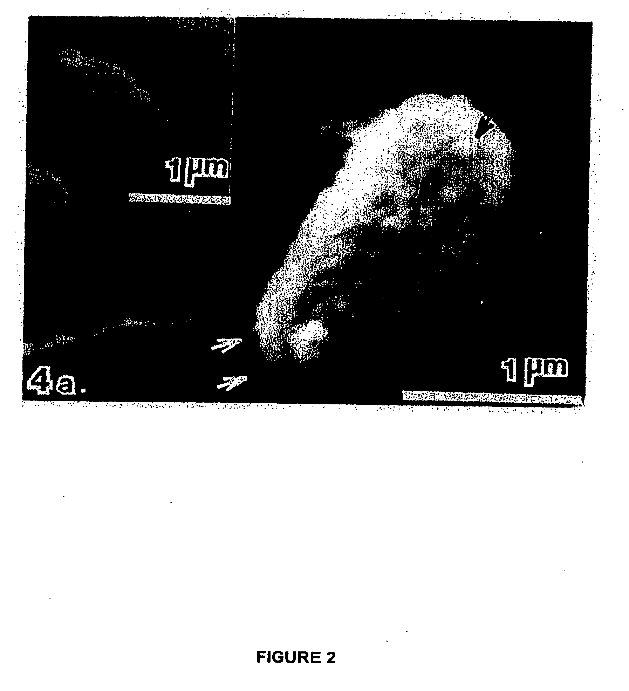

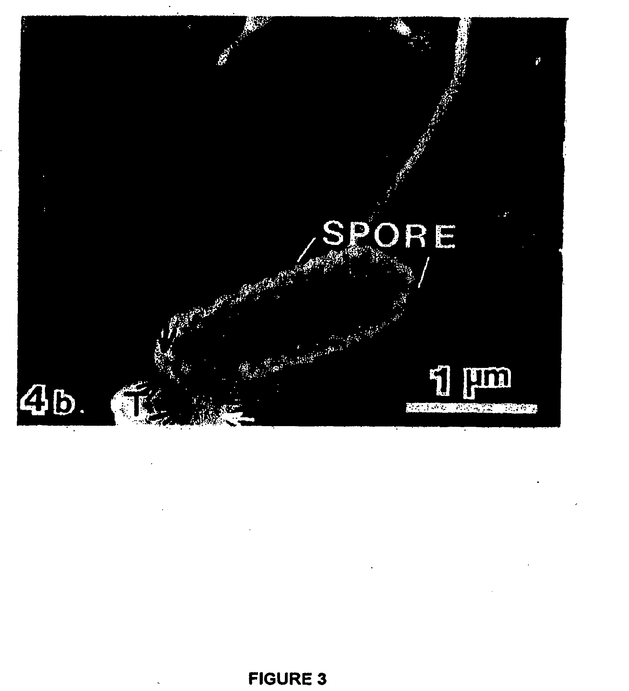

Unlike C. *sporogenes,* C *difficile* has a reduced number of attached spores and requires a longer incubation time to allow attachment to take place. Dormant C. *difficile* spores are small (approximately 2.0 grn long x 1.1 grn wide), elliptical, and predominantly smooth. See, Figure 2, insert. Once activation had begun, spores exhibited low prominences covering the exosporium. See, Figure 3. In the early stage of attachment, C. *difficile* 43594 developed exosporial bumps and knobs covering the entire surface of the elongate spore. These spores developed more elongate exosporial projections at the tail region, which were replaced later during the germination cycle by a thickened attachment structure. Figure 3, black arrows. Unlike C. *sporogenes,* C. *difficile* exhibits fewer exosporial membrane extensions but produces a thickened exosporial membrane extension that developed at the tail end of the spore.

The ability of C. *sporogenes* and C. *difficile* spores to remain attached to an agar surface during agitation (i.e., for example, a 300 rpm centrifugation) in water or buffer, strongly suggests that these spores have a formidable means ofadhesion and/or attachment to surfaces. Under ideal conditions, the attachment of C. *sporogenes* to an agar surface has been observed to take place within 27 min following inoculation. Under anaerobic conditions on pre-reduced agar, maximal attachment of C. *sporogenes* spores has been observed to take place within 88 to 105 min. C. *difficile* spore attachment took considerably longer, for example, 105 min for C. *difficile* 9689 and 180 min for C. *difficile* ATCC 43594.

Once attached, the spores can remain tenaciously anchored to a solid surface. C. *difficile* 43594 appears unique in that the spores attach very slowly. However, C. *difficile* may have an adaptive response that accelerates attachment following exposure to an animate surface. For example, an increased incidence of C. *difficile* nosocomial infection have been reported in patients having increased intestinal motility and those who have undergone multiple enemas. Bartlett, J. "Introduction" In: Clostridium Difficile: Its Role in Intestinal Disease, Ed. R. Rolfe and S. Finegold). Academic Press, New York, pp. 113 (1988); Silva, J. Jr., "Prevention of Clostridium difficile-associated intestinal disease" In: Clostridium difficile: Its Role in Intestinal Disease, Ed. R. Rolfe and S. Finegold. 30 Academic Press, New York, pp. 368-378 (1988).; and McFarland et al., "Nosocomial acquisition of Clostridium difficile infection. New Engl. J Med., 320:204-210 (1989). C. *difficile* spores may be routinely found in the digestive tract of healthy, non-symptomatic individuals. Consequently, a triggering mechanism that initiates attachment to the gut and begins the infective process may be a combination of the loss of normal flora (i.e., for example, caused by antibiotics, disease, or repeated enemas) and/or hypermobility of the colon.

*Clostridium* spore exosporium attachment outgrowths typically have a diameter of approximately 7 nm and may vary in length between 0.1 and 2.9 nm. These outgrowths comprise fibrils that are usually less than 200 nm in length and can be extremely delicate. Hancock, I, "Microbial cell surface architecture" In: Microbial Cell Surface Analysis, Ed. N. Mozes, P. Handley, H. Busscher and P. Rouxhet, VCH Publishers, New York, pp. 23-59 (1991). Usually exosporium outgrowths of *C*. *sporogenes* measure 85 to 1015 nm in width, however, some outgrowths, especially those also comprising co-aggregation attachments, can be as long as 2000 nm in length. Knob-like protrusions from *C. sporogenes* exosporium can range from 68 to 75 nm in diameter, and swollen projections seen in the late stages of germination can be as large as 180 to 240 nm in diameter.

Clearly, the spore exosporium, which has always been thought to be 'a passive participant in germination', appears to play an active role in spore attachment and 15 colonization of *C*. *sporogenes* and *C*. *difficile.* In one embodiment, a *C. difficile* protein is derived from an exosporium surface layer, wherein the exosporium layer comprises the outermost protein of a *C*. *diffcile* spore. In one embodiment, the present invention contemplates a composition comprising at least one outermost surface protein of the *Clostridium difficile* spore. In one embodiment, the surface protein comprises a spore surface protein. Although it is not necessary to understand the mechanism of an invention, it is believed that these spore surface proteins present an interface between the bacterial spore and a substrate surface binding site. It is also believed that these proteins can be used to define molecular attachment interactions including, but not limited to: i) between the spore and animate surfaces; and ii) between the spore and inanimate surfaces. Size characterizations of *Clostridium difficile* exosporium proteins are presented in Table I.

**Table I. Size Characterizations Of Representative C. difficile Exosporium Proteins.**

| Protein (alternative name) | Size(kDa) | BAS gene no | GI |
|---|---|---|---|
| CD 1581 | 20 | 1581 | 126699184 |
| CD 1067 | 47 | 1067 | 126698654 |
| CD 3620 | 14 | 3620 | 126701246 |
| CD 1613 | 35 | 1613 | 126699217 |
| CD 1711 | 12 | 1711 | 126699318 |
| Putative spore surface protein | 22 | 598 | 126698176 |
| Putative spore surface protein | 22 | 2401 | 126700016 |
| Superoxide dismutase | 27 | 1631 | 126699235 |
| Putative proline racemase | 24 | 3237 | 126700857 |
| 60 kDa chaperonin | 58 | 0194 | 126697767 |
| Chaperone protein | 67 | 2461 | 126700078 |
| 10 kDa chaperonin | 10 | 0193 | 126697766 |
| Pyruvate-flavodoxin oxidoreductase | 130 | 2682 | 126700296 |
| CD 0881 | 36 | 0881 | 126698458 |
| CD 1511 | 35 | 1511 | 126699115 |
| Cell surface protein | 76 | 2793 | 126700409 |
| 3-hydroxybutyryl-CoA dehydrogenase | 31 | 1058 | 126698642 |
| Putative spore cortex-lytic enzyme | 47 | 0551 | 126698130 |
| Putative aliphatic sulfonates ABC transporter, substrate binding lipoprotein | 36 | 1484 | 126699088 |
| Putative fructose-bisphosphate aldolase | 33 | 0403 | 126697972 |
| Dipicolinate synthase, subunit B | 21 | 2967 | 126700588 |
| Putative indolepyruvate oxidoreductase, subunit B | 21 | 2380 | 126699995 |
| Electron transfer flavoprotein alpha-subunit | 36 | 1056 | 126698640 |
| Putative phage-related cell wall hydrolase | 28 | 1898 | 126699508 |
| Cell surface protein | 66 | 2791 | 126700407 |
| Formnate-tetrahydrofolate ligase | 60 | 0718 | 126698298 |
| Oligopeptide ABC transporter, substrate-binding protein | 58 | 2672 | 126700286 |
| Gamma-aminobutyrate metabolism dehydratase/isomerase | 56 | 2341 | 126699959 |
| NAD-specific glutamate dehydrogenase | 46 | 0179 | 126697752 |
| Putative aminoacyl-histidine dipeptidase | 53 | 0708 | 126698287 |
| Proline reductase subunit proprotein | 68 | 3244 | 126700863 |
| Glycine/sarcosine/betaine reductase complex component C beta subunit | 55 | 2349 | 126699967 |
| Glycine dehydrogenase subunit | 2 | 54 | 1658 |
| Acetyl CoA acetyltransferase | 41 | 1059 | 126698643 |
| Aspartate aminotransferase | 45 | 1339 | 126698938 |
| Putative bifunctional protein: peroxidoredoxinlchitinase | 82 | 1433 | 126699037 |
| Glyceraldehydes-3 -phosphate dehydrogenase | 36 | 3174 | 126700794 |
| Butyryl-CoA dehydrogenase | 41 | 1054 | 126698638 |
| d-ribose ABC transporter, substrate-binding protein | 35 | 0300 | 126697872 |
| Putative ruberythrin | 20 | 1474 | 126699078 |
| Succinate-semialdehyde dehydrogenase | 51 | 2342 | 126699960 |
| Oxidoreductase, thiamine diP-binding subunit | 39 | 0116 | 126697688 |

In one embodiment, *Clostridium difficile* proteins may be identified by probing total spore protein preparations with antibodies directed against purified, intact, C. *difficile* spores, to created a hybridized protein-antibody complex. In one embodiment, a C. *difficile* spore surface protein includes, but is not limited to, CDI067, CD3620, CD 1581, CD 598, or CD 2401. In one embodiment, C. *difficile* enzymes are associated with C. *difficile* spore surface protein preparations.

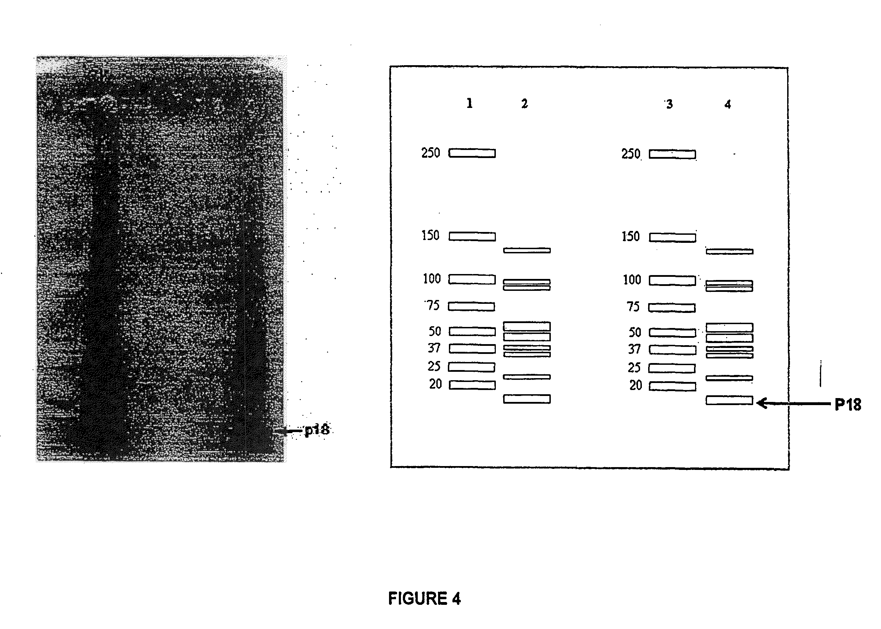

In one embodiment, a *Clostridium* hypothetical protein (CD 1581) may be isolated as a hybridized antibody-protein complex comprising a molecular weight including, but not limited to, 18, 140, 150, or 160 kDa. See, Figures 4-6. In one embodiment, the hypothetical protein (∼20 kDa) comprises an amino acid sequence of SEQ ID NO:1 (Accession Numbers YP_001088081, GI: 126699184): 
1 MENKKCYSED WYERGESTAK WFQNDREEYE REAYDEDRER RGSNCGCSDS
51 GENRPRNCER FRREAEIRER EAREAFCESS EKKKEALAYE CEARKLWEEA
101 EKYWDEYSKY NYKGIEYLAE AARLFDEGME CEARRNGNNG GNNNNCCHKC
151 HKCNCNCCRK
 encoded by a *Clostridium* hypothetical nucleic acid SEQ ID NO: 2:

1 atggaaaata aaaaatgtta ttcagaagat tggtatgaaa gaggagaatc tacagctaaa
61 tggttccaaa atgatagaga agaatatgaa agagaagcat atgatgaaga tagagaaaga
121 agaggttcaa actgtggatg ttcagattca ggagaaaata gacctagaaa ctgtgaaaga
181 tttagaagag aagctgagat aagagaaaga gaagcaagag aagcattctg tgaatcttca
241 gagaaaaaga aagaggcatt agcatatgaa tgtgaagcta gaaaattatg ggaagaagca
301 gaaaaatact gggatgaata ttcaaaatac aactataaag gaatcgaata tttagcagaa
361 gctgctagat tatttgatga aggtatggaa tgtgaagcta gaagaaatgg aaataatgga
421 ggaaacaata ataattgttg ccataaatgc cataaatgta attgtaactg ctgtagaaaa
481 taa

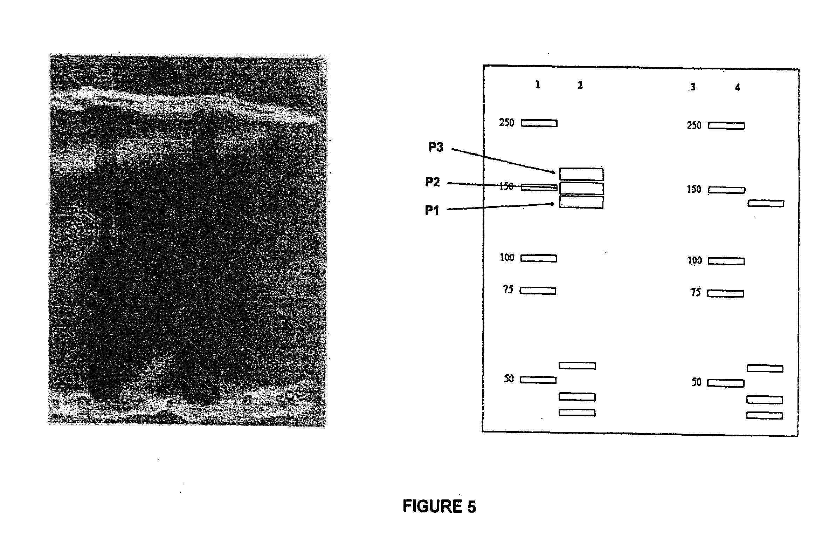

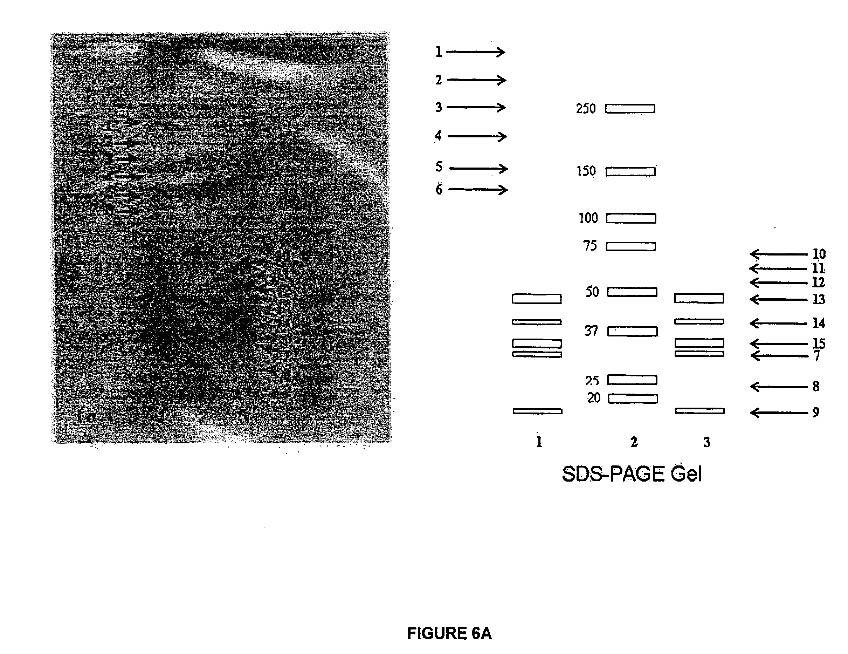

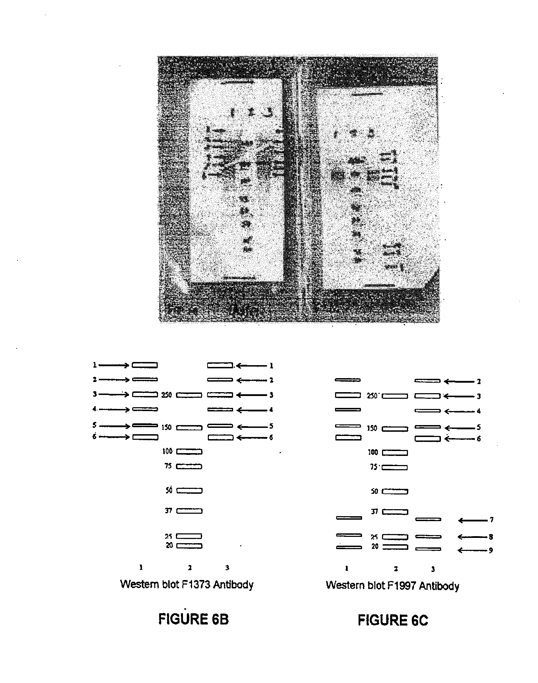

In one embodiment, a *Clostridium* hypothetical protein (CD 1067) may be isolated as a hybridized antibody-protein complex comprising a molecular weight including, but not limited to, 140, 150, and 160 kDa. See, Figures 5 ,6. In one embodiment, the hypothetical protein (- 47 kDa) comprises an amino acid sequence of SEQ ID NO: 3 (Accession Numbers YP_001087551; GI: 126698654): 
1 MQDYKKNKRR MMNQPMSTMN EEEVYTDEIN SEDMRGFKKS HHHNGCNTDN
51 KCECHDDCNP CNPCNPCKPN PCNPCKPNPC DDNCGCHDNC KCDCEPCEMD
101 SDECFENKCG PECCNPISPR NFSVSNAVPF AIEANRIFDT MQFQTFTDAT
151 GPNGEPLTFE TEVVEVFGSV PSAGQASVTI EKICLSNDGI VIDTGMTTLE
201 DFDLDPLGDIVGRNCETTFEFAVCGERNSECCRQGKGKSVAYKQRGLTVA
251 VRNLVLELRG RCGCTEFVAL APPAVRAGGG CKRRVDYVEF TFNTLSAPIC
301 LPADGRAVTL RQEYQTNLTV DCIGKSILKL ECNECCEPFY ELIIPNDIDL
351 VLCLQETVST LISEQIVVLA SPNPIQPRLV DTFSKVCDFS QCGPNHGSGK
401 PSCHR
 encoded by a *Clostridium* hypothetical nucleic acid SEQ ID NO: 4:

1 atgcaagatt ataaaaaaaa taaaagaaga atgatgaatc agccaatgtc tacaatgaat
61 gaagaagaag tgtatacaga tgaaataaat tcagaagaca tgagaggttt taaaaaatca
121 caccatcata atggatgtaa tactgataat aagtgtgagt gccatgatga ttgcaatcca
181 tgcaacccat gtaatccatg taaacctaac ccatgcaatc catgcaaacc taatccatgt
241 gatgacaatt gtggatgcca tgacaattgt aaatgtgatt gtgaaccatg tgaaatggat

301 tcagatgaat gttttgaaaa caaatgtgga ccagaatgct gtaatcctat atctccaaga
361 aacttctctg tatcaaatgc agtgccattt gcaatagagg ctaatagaat atttgatact
421 atgcaattcc aaacatttac agatgcaaca ggaccaaatg gagagccatt aacttttgaa
481 acagaagtag tagaagtatt tggttcagtt ccaagtgcag gtcaagcaag tgtaactata
541 gaaaaaatat gcttaagtaa tgatggaatc gttatagaca caggaatgac aactttagaa
601 gatttcgatt tagacccatt aggagatata gtaggaagaa actgtgaaac aacttttgaa
661 tttgcagttt gtggagaaag aaactctgag tgctgtagac aaggaaaagg caaatcagta
721 gcttataaac aaagaggatt aactgtagca gttcgtaatt tagtactaga gctaagaggt
781 agatgtggat gtacagagtt cgttgcatta gctttcccag cagttagagc aggaggtgga
841 tgtaagagaa gagttgatta tgtagaattt acttttaaca cactttcagc accaatatgc
901 ttgccagctg acggaagagc tgttacttta agacaagaat atcaaactaa cttaactgta
961 gattgtatag gaaaatctat attaaaatta gaatgcaacg aatgttgtga acctttctat
1021 gaattaatta taccaaatga tatagattta gtactttgct tacaagaaac agttagcaca
1081 ttaataagtg aacaaatagt agttttagca tcaccaaatc caatccaacc aagacttgtt
1141 gatactttct ctaaagtatg tgatttttcg caatgtggac ctaatcatgg aagtggaaag
1201 ccaagttgcc acagatag

In one embodiment, a *Clostridium* hypothetical protein (CD 3620) may be isolated as a hybridized antibody-protein complex comprising a molecular weight including, but not limited to, 140 kDa. See, Figures 5 & 6. In one embodiment, the hypothetical protein (∼ 14 kDa) comprises an amino acid sequence of SEQ ID NO: 5 (Accession Numbers YP 001090143; GI: 126701246): 
1 MYDDYKYNKC NKCYNDYEEM THVHEYSESV KLAEECEDRH NHRAAGVTGE
51 AIPINGGTNH VHKINDNVDF LDHFHKICVT TGPAIRIPGT DKHIHLICGE
101 TTVNDGHCHKFLFTTQIEAPLV
 encoded by a *Clostridium* hypothetical nucleic acid SEQ ID NO: 6:

1 ttatactaaa ggtgcttcta tttgagtagt aaaaaggaat ttatgacaat gaccatcatt
61 aacagtagtt tctccacata ttaagtgaat gtgtttatca gttccaggta ttctaattgc
121 tggccctgtt gtaacacata tcttatgaaa atggtcaagg aaatctacat tatcatttat
181 tttatgaaca tgatttgtac ctccatttat tggtatagct tcaccagtaa cccctgctgc
241 acggtggtta tgtctatcct cacattcttc tgctaactta acactttctg aatattcatg
301 aacatgtgtc atttcctcat aatcattata gcacttgttg cacttattgt atttataatc
361 atcatacac

In one embodiment, a Clostridium hypothetical protein (CD 1613) may be isolated as a hybridized antibody-protein complex. In one embodiment, the hypothetical protein comprises an amino acid sequence of SEQ ID NO: 7 (Accession Number YP 001088114): 
1 mennkcredf rftqeyeedy pntneryyen yqvadryyny pnkykepkik qccckksmre
61 alellrydal rpfvnfuqfa fisdffivga nlvgidlsap pkdnlsgldg tferfsacnc
121 dlidiagrvs ypipvpltle glintigtip gvaelialid avipptidlg aildailaai
181 idfilaastp lanvdlaslc nlkavafdit padyedfias lgyyldkkhy kecncncdcd
241 dcccnkgild nlymsninnq vtvvagslvl tgvevlgkkn dvivlgnsnd sriyfvcvds
301 idyia
 encoded by a Clostridium hypothetical nucleic acid SEQ ID NO: 8:

1 ttatgcaata taatctatag aatctacaca tacaaagtat attcttgaat cattagaatt
61 tccaagtact ataacatcat ttttcttacc tagaacttca acaccagtta gaaccaaact
121 accagctact acagtaactt gattatttat atttgacata taaagattat ctaggatacc
181 tttattacaa cagcaatcat cacaatcgca gttacaatta cattctttgt aatgtttttt
241 atcaagatag taacctaaag atgctatgaa atcttcataa tctgcaggtg taatatcaaa
301 tgcaacagct ttaagattac acaatgatgc taaatctacg tttgctaatg gagtagatgc
361 agcaagtata aaatcaatta tagcagcaag tattgcatct aatatagccc caaggtctat

421 cgtaggagga ataactgcat caataagtgc aattaattca gctactcctg gtatagttcc
481 tatagtatta attaatccct caagagttaa agggactgga ataggataag atactctacc
541 agctatatct attaaatcac agttacaagc agaaaatctt tcaaaagtac catcaagtcc
601 agataaatta tcttttggag gagctgaaag atctatacct accaaattag cacctactat
661 aaagaaatct gagataaaag caaattgatt aaagtttaca aaaggtctta gagcatcata
721 tcttagaagt tctaaggcct ctctcatact ttttttacaa caacattgtt ttattttagg
781 ttctttatat ttatttggat aattatagta tctatcagct acttgataat tttcatagta
841 tctttcattt gtatttggat aatcttcctc atattcttgt gtaaatctaa agtcctctct
901 acatttatta ttttccac

In one embodiment, a *Clostridium* hypothetical protein (CD 1711) maybe isolated as a hybridized antibody-protein complex. In one embodiment, the hypothetical protein comprises an amino acid sequence of SEQ ID NO: 9: 
1 mlivttekve gkkiskvlgl vrgstirakh vgkdigasfk nlvggeltgy
51 nemltearqi aigrmvedae akganaviaf rlssasvmqg aaemlaygta
101 vvleddnsil ek:
 encoded by a *Clostridium* hypothetical nucleic acid SEQ ID NO: 10:

1 atgttaatag taactacaga aaaagtagaa ggtaaaaaga tatcaaaggt tttaggatta gtgagaggaa
71 gtacaataag agcaaaacat gttggaaaag atataggagc aagttttaaa aatcttgttg gaggagaact
141 tactggatat aatgaaatgc tcactgaagc aagacaaatt gccataggta gaatggttga agatgcagaa
211 gctaaaggtg caaacgcagt aatagcattt agactgtctt cagcttcagt tatgcaaggg gcagcagaaa
281 tgcttgctta tggaacagca gttgttttag aagatgataa tagtattctt gaaaaataa

In one embodiment, a *Clostridium* hypothetical protein (CD 0881) may be isolated as a hybridized antibody-protein complex. In one embodiment, the hypothetical protein comprises an amino acid sequence of SEQ ID NO: 11: 
1 mgtkivlsiv livvvvaisl tcirvikqsk vgiimrlgkf qkvaetgvhf lipfldkmay
61 vidlreivid fppqpvitkd nvtmqidtvv yykvtdpvry vfeianpiaa ienltattlr
121 niigeldlde tltsrdiinv kmrtildeat dkwgikvnrv elknimppqd iqvamekqmr
181 aererreail qaegnksaai lqaegekqsa iltaeakkea mvrvaegeke sailvaegea
241 eairqtaiak aqgeaemikr tqmataeglk lvfsamkead idnnilalks mealekmaeg
301 kstklvlpse avnflgtfkg ikevmsddnk evldikevln dneslkk
 encoded by a Clostridium hypothetical nucleic acid SEQ ID NO: 12

1 atgggtacta aaattgtttt aagtattgta ttaatagtag tagtcgtagc aataagttta
61 acttgtataa gagttataaa acaatctaaa gtaggtataa taatgagact tggtaagttt
121 caaaaagtgg ctgaaacagg tgtacatttc ttaataccat ttttagataa aatggcatat
181 gtaattgacc ttagagaaat tgtaatagat tttccacctc aaccagttat aactaaagat
241 aatgtaacta tgcagattga tacagttgta tattataaag ttacagaccc agttagatat
301 gtgtttgaga tagctaatcc aatagctgct attgaaaatt taacagctac aacactaaga
361 aatataattg gtgaacttga tttagatgaa acattaacat caagagatat aataaatgta
421 aaaatgagaa ctattcttga tgaagcaaca gataaatggg gtataaaagt aaatagagta
481 gagttaaaaa atataatgcc tcctcaggat attcaagttg caatggaaaa acaaatgaga
541 gcagaaagag aaagaagaga ggcaatactt caagcagaag gtaataagtc agctgcaata
601 cttcaagctg aaggtgaaaa acaatctgct atattaacag cagaagccaa aaaagaagct
661 atggtacgtg tagcagaagg tgaaaaagaa tctgctatat tagtggcaga aggtgaagct
721 gaagctataa gacagacagc tattgctaag gcacaaggtg aagctgaaat gataaaaaga
781 actcaaatgg caacagcaga aggtttaaaa ttagtttttt cagcaatgaa agaggctgat
841 atagacaata atattctagc attaaaatct atggaagctc ttgaaaaaat ggctgaaggt
901 aagtcaacaa aactggtttt accttcagaa gcagttaatt tcctaggaac attcaaagga
961 ataaaggaag ttatgagtga tgataacaaa gaagtacttg atataaaaga agttttaaat
1001 gataatgaat cattaaaaaa ataa

In one embodiment, a *Clostridium* hypothetical protein (CD 1511) maybe isolated as a hybridized antibody-protein complex. In one embodiment, the hypothetical protein comprises an amino acid sequence of SEQ ID NO: 13: 
1 midnqkyvil slelhlffsr imkehalfle agftnknynl ameadhykkq fedllsytvs
61 asngiirpdi lyseelvttl tsvaeqktee ftgieinkni ttrelnlqsg vnpqvgqdlv
121 nyvaqlnsda irlldglinf kerv1dgvls ctiftsnyp1 1 1ehiihean 1yrsyvvdle
181 nkidieskna keielfWdhi mmehalfinrg lldpsegeli ntsndfaikf neliektnem
241 tdsniknite et1netvefk dfkeagasgi eqckiksiil plladhvlre anhyiri1es
301 yknm
 encoded by a *Clostridium* hypothetical nucleic acid SEQ ID NO: 14:

1 ttacatgttt ttataactct ccaatattct aatataatga tttgcctctc ttaaaacatg
61 gtctgctaaa agtggtaata ttatagattt tatcttacac tgttctattc ctgatgctcc
121 tgcttcttta aaatctttaa actcaacagt ttcatttaga gtttcttctg taatgttctt
181 gatattagaa tcagtcattt cgtttgtttt ttcaattaat tcattaaatt ttatagcaaa
241 atcatttgaa gtatttatta gttcaccttc tgaggggtct agtaatcctc tcataaacag
301 agcatgttcc atcataatat ggtcccagaa taattctatt tctttagcgt tttttgactc
361 aatatctatt ttattttcaa ggtcaactac ataagaacga tataaatttg cttcatgtat
421 tatatgttca agaagtagag ggtagtttga tgtaaatata gtacatgata gtacaccatc
481 taagactctt tctttaaaat taataagccc atcaagtaat cttattgcat cagagttaag
541 ttgagctaca tagttcacta aatcttgacc aacttgtggg tttacaccac tttgtagatt
601 taattctctt gtagtgatgt ttttgtttat ttctatccct gtaaactctt ctgttttttg
661 ttctgcaact gatgtgagag tagttacaag ttcttctgaa tataatatat caggtctaat
721 tataccatta ctagcactaa cagtgtatga taataaatct tcaaattgct tttatagtg
781 gtcagcttcc atagcaagat tataattttt atttgtgaat cctgcttcta aaaaaagagc
841 atgctctttc ataattcttg aaaaaaataa atgtaattct agtgataaaa taacatattt
901 ttgattatct atcat

### B. Clostridium difficile Collagen-Like Proteins

The *B. anthracis* exosporium protein BclA was compared to the C. *difficile* genome, wherein three C. *difficile* genomic areas were identified as homologous to *B. anthracis* BclA (i.e., for example, spore surface genomic areas).

In one embodiment, a *Clostridium* spore surface homolog protein comprises an amino acid sequence of SEQ ID NO:15 (Accession Number CAJ67154): 
mmiilylnd dtfiskkypd knfsnldycl igskcsnsfv keklitffkv ripdilkdks
ilkaelfihi dsnknhifke kvdieikris eyynlrtitw ndrvsmenir gylpigisdt
snyiclnitg tikawamnky pnyglalsln ypyqilefts srgcnkpyil vtfedriidn
10 cypkcecppi ritgpmgprg atgstgpmgv tgptgstgat gsigptgptg ntgatgsigp
tgvtgptgst gatgsigptg vtgptgntgv tgsigptgat gptgntgvtg sigptgvtgp
tgntgeigpt gatgptgvtg sigptgatgp tgeigptgat gatgsigptg atgptgatgv
tgeigptgei gptgatgptg vtgsigptga tgptgatgei gptgatgptg vtgsigptga
tgptgatgei gptgatgptg vtgeigptga tgptgntgvt geigptgatg ptgntgvtge
15 igptgatgpt gvtgeigptg ntgatgsigp tgvtgptgat gsigptgatg atgvtgptgp
tgatgnssqp vanflvnaps pqtlnngdai tgwqtiigns ssitvdtngt ftvqengvyy
isvsvalqpg sssinqysfa ilfpilggkd laglttepgg ggvlsgyfag flfggttfti
nnfssttvgi rngqsagtaa tltifriadt vmt
 encoded by a *Clostridium* spore surface homolog nucleic acid SEQ ID NO: 16:

atgagaaata ttatacttta tttaaatgat gatactttta tatctaaaaa atatccagat
aaaaacttta gtaatttaga ttattgctta ataggaagta aatgttcaaa tagttttgta
aaagaaaagt tgattacttt ttttaaagtg agaataccag atatattaaa agacaaaagt
atattaaaag cagagttatt tattcatatt gattcaaata agaatcatat ttttaaagaa
aaagtagata ttgaaattaa aagaataagt gaatattata atttacgaac tataacatgg
aatgatagag tgtctatgga aaatatcagg ggatatttac caattgggat aagtgataca
tccaactata tttgtttaaa tattacggga actataaaag catgggcaat gaataaatat
cctaattatg ggttagcttt atctttaaat tacccttatc agattcttga atttacatct
agtagaggtt gtaacaaacc gtatatactt gtaacatttg aagatagaat tatagataat
tgttatccta aatgtgagtg tcctccaatt agaattacag gtccaatggg accaagagga
gcgacaggaa gtacaggacc aatgggagta acaggcccaa ccggaagtac aggagcgaca
ggaagcatag gaccaacagg cccaaccgga aatacaggag caacaggaag tatagggcca
acgggagtaa caggcccaac cggaagtaca ggagcgacag gaagtatagg accaacagga
gtaacaggtc cgacaggaaa tacgggagtg acaggaagta taggaccaac gggagcaaca
ggcccgacag gaaatacggg agtgacagga agtataggac caacaggagt aacaggccca
acaggaaata caggagaaat aggaccaacg ggagcaacag gtccaacagg agtgacagga
agtataggac caacaggagc aacaggacca acaggagaaa taggaccaac gggagcaaca
ggagcgacag gaagtatagg accaacagga gcaacaggtc caacaggagc gacaggagtg
acaggagaaa tagggccaac aggagaaata ggaccaacgg gagcaacagg cccaacagga
gtgacaggaa gtataggacc aacgggagca acaggcccaa caggagcgac aggagaaata
ggaccaacag gagcaacagg cccaacagga gtgacaggaa gtataggacc aacgggagca
acaggcccaa caggagcgac aggagaaata ggaccaacgg gagcaacagg cccaacagga
gtaacaggag aaataggacc aacgggagca acaggcccaa caggaaatac aggagtaaca
ggagaaatag gaccaacggg agcaacgggt ccgacaggaa atacaggagt gacaggagaa
ataggaccaa cgggagcaac aggaccaaca ggagtgacag gagaaatagg gccaacagga
aatacaggag cgacaggaag tatagggcca acgggagtaa caggtccaac aggagcgaca
ggaagtatag gaccaacggg agcaacagga gcgacaggag taacaggacc aacaggtcca
acaggagcaa caggcaattc ctctcagcca gttgctaact tcctcgtaaa tgcaccatct
ccacaaacac taaataatgg agatgctata acaggttggc aaacaataat aggaaatagt
tcaagtataa cagtagatac aaatggtacg tttacagtac aagaaaatgg tgtgtattat
atatcagttt cagtagcatt acaaccaggt tcatcaagta taaatcaata ttctttcgct
atcctattcc caattttagg aggaaaagat ttggcagggc ttactactga gccaggaggc
ggaggagtac tttctggata ttttgctggt tttttatttg gtgggactac ttttacaata
aataattttt catctacaac agtagggata cgaaatgggc aatcagcagg aactgcggct
actttgacga tatttagaat agctgatact gttatgactt aa

In one embodiment, a *Clostridium* spore surface homolog protein comprises an amino acid sequence of SEQ ID NO: 17 (Accession Number CAJ70248): 
mlllimsmky fgpfddndyn ngydkyddcn ngrddynscd chhccppscv gptgpmgprg
rtgptgptgp tgpgvgatgp tgptgptgpt gntgntgatg 1rgptgatga tgptgatgai
gfgvtgptgp tgatgatgad gvtgptgptg atgadgitgp tgatgatgfg vtgptgptga
tgvgvtgatg ligptgatgt pgatgptgai gatgigitgp tgatgatgad gatgvtgptg
ptgatgadgv tgptgatgat gigitgptgp tgatgigitg atgligptga tgtpgatgpt
gatgptgvgv tgatgatgat gadgatgvtg ptgatgatga ng1vgptgat gaagtpgatg
ptgatgptgv gitgatgatg atgptgadga tgptgatgnt gadgvagptg atgntgadga
tgptgatgat gadgatgptg atgatgvaga tgatgptgat gadgatgptg atgatgadga
tgptgatgat gvtgatgptg ptgatgatga tgasaiipfa sgip1sltti agg1vgtpgf
vgfgssapg1 sivggvidlt naagtltnfa fsmprdgtit sisayfstta als1vgstit
itadyqsta pnnsftavpg atvt1applt gilsvgsiss givtg1niaa taqtpdrqyai
 encoded by a *Clostridium* spore surface homolog nucleic acid SEQ ID NO: 18:

ttatatggca tactgtctgt ctggagtttg tgctgttgct gctatattta atcctgttac
aattccacta gaaattgaac caactgataa tatacctgta agtggtggag ctagtgtaac
tgtcgctcct ggtacagctg taaatgagtt atttggtgca gtagattggt aaagtgttgc
tgtaattgta attgttgaac caacaagtga aagtgctgct gttgtactga agtatgctga
aatagatgtt attgttccat ctcttggcat tgaaaatgca aagttagtca atgttcctgc
tgcgtttgta aggtctatta ctccaccaac tatacttaat cctggagccg aactaccaaa
tccaacaaat ccaggtgtac ctactaatcc tccagctata gttgtaagtg atagtggtat
acctgatgca aaaggtatta ttgcactagc acctgttgct cctgttgctc ctgttgctcc
tgttgggcct gttgggcctg ttgctcctgt aactcctgtt gcccctgttg ctcctgttgg
acctgttgct ccatctgccc ctgttgctcc tgttgctcct gttggacctg ttgctccatc
tgctcctgtt gctcctgttg gacctgttgc tcctgttgct cctgccactc ctgttgctcc
tgttgctcct gttggacctg ttgctccatc tgctcctgtt gcccctgttg ctcctgttgg
acctgttgct ccatctgctc ctgtatttcc tgttgctcct gttggacctg ctactccatc
tgctcctgta tttcctgttg ctcctgttgg acctgttgct ccatctgctc ctgttggacc
tgtcgctcct gttgcccctg ttgctcctgt tattcctact cctgttgggc ctgttgctcc
tgttggacct gttgctccag gtgttcctgc tgctcctgtg gctcctgttg ggcctactaa
tccatttgct cctgttgccc ctgttgctcc tgttggacct gttactcctg ttgctccgtc
tgctcctgtt gctcctgttg ctcctgttgc tcctgttact cctactcctg ttgggcctgt
tgctcctgtt ggacctgttg ctccaggtgt tcctgttgcc cctgttggac ctattaatcc
tgttgcccct gttatcccta ttcctgttgc tcctgttgga cctgttgggc ctgttattcc
tattcctgtt gctcctgttg ctcctgttgg gcctgttact ccatctgctc ctgttgcccc
tgttgggcct gttgggcctg ttactcctgt tgctccatct gcccctgttg ctcctgttgc
tcctgttgga cctgttattc ctattcctgt tgctcctatt gcccctgttg gacctgttgc
tccaggtgtt cctgtcgctc ctgttggacc tattaatcct gttgctcctg ttactcctac
tcctgttgct cctgttgggc ctgttggacc tgttactcca aatcctgttg cccctgttgc
tcctgttgga cctgttattc catctgctcc tgttgctccc gttggacctg ttggacctgt
tactccatct gctcctgttg ctcctgtcgc tcctgttggg cctgttggac ctgttactcc
aaaccctata gctcctgtcg ctcctgttgg gcctgttgcc cctgttgctc ctgttggacc
tcttaatcct gttgctcctg tattccctgt atttcctgtt ggaccagtcg gaccggttgg
tcctgttggg cctgttgccc ctactcctgg acctgttgga cccgttggac ctgttgggcc
ggttctacct cttggaccca ttgggcctgt tggacctaca catgatggtg gacagcaatg
atggcaatca cagctattat aatcatcacg accattgtta caatcatcat atttatcata
gccattgttg taatcattat catcaaatgg tccaaaatat ttatttctac tcattattaa
aagcac

In one embodiment, a *Clostridium* spore surface homolog protein comprises an amino acid sequence of SEQ ID NO: 19 (Accession Number CAJ70128): 
msdisgpsly qdvgptgptg atgptgptgp rgatgatgan gitgptgntg atgangitgp
tgnmgatgpn gttgstgptg ntgatgangi tgptgntgat gangitgptg nkgatgangi
tgstgptgnt gatgangitg ptgntgatga tgptgltgat gatgangitg ptgntgatga
ngvtgatgpt gntgatgptg sigatgatgt tgatgpigat gatgadgevg ptgavgatgp
dglvgptgpt gptgatgang lvgptgptga tganglvgpt gatgatgvag aigptgavga
tgptgadgav gptgatgatg angatgptga vgatgangva gpigptgptg engvagatga
tgatgangat gptgavgatg angvagaigp tgptgangat gatgatgatg angatgptga
tgatgvlaan naqftvssss lvnntlvtfn ssfingtnit fptsstinla vggiynvsfg
iratlslagf msittnfngv tqnnfiakav ntltssdvsv slsflvdara aavtlsftfg
sgttgtsaag yvsvyriq
 encoded by a Clostridium spore surface homolog nucleic acid SEQ ID NO: 20:

ctattgtatt ctataaactg atacatatcc agctgcagaa gtacctgtcg tgcctgaacc
aaatgtaaaa cttaaagtaa cagctgctgc tctagcatca actaaaaagc ttaaacttac
acttacatct gatgaagtaa gtgtatttac tgcttttgca ataaagttat tttgagttac
tccattaaag ttagtagtaa ttgacataaa tcctgcaagt gaaagtgtgg cacgtatacc
gaaagataca ttgtatatcc ctccaactgc aagatttata gtactacttg ttggaaaagt
tatattagtt ccatttataa atgatgaatt aaatgtcact aatgtattat tcactaaact
tgaagaggac actgtaaatt gtgcattgtt tgctgctaac actcctgttg ctcctgtcgc
tccggttgga cctgttgctc catttgctcc tgttgctcct gtcgcccctg ttgctcctgt
cgctccattt gctccggttg ggcctgttgg tcctatcgct cctgctactc catttgctcc
cgttgctcct actgctcctg ttgggcctgt tgctccattt gcccctgttg ctcctgtcgc
tcctgttgct cctgctactc cattttctcc cgttggacct gttggaccta ttggacctgc
tactccattc gctcccgttg ctcctactgc tcccgttggg cctgttgctc catttgcccc
tgttgctcct gtcgctcctg ttggacctac tgctccatct gctcccgttg ggcctgttgc
tcctactgct cccgttggac ctattgcccc agctactcct gttgctcctg tcgctcctgt
tggacctacc aaaccatttg ctccggttgc tccggttggg cctgttgggc ctaccaaacc
atttgctccg gttgctccgg ttgggcctgt tgggcctgtt ggacctacca aaccatctgg
acctgttgct cctactgctc ctgttggacc tacctctcca tctgctcctg ttgctcctgt
tgctcctatt gggcctgttg ccccagttgt tcctgttgct ccagtcgctc ctatacttcc
tgttggacct gttgctcctg tatttcctgt tgggcctgta gcacctgtta ctccatttgc
tccggttgct cctgtatttc ctgttggtcc tgttattcca tttgctccgg ttgctcctgt
tgctcctgtt agtccggttg gacctgttgc tcctgttgct cctgtattcc ctgttggacc
tgttattcca tttgctccag tcgctcctgt atttcctgtt ggtcctgtag aacctgttat
tccatttgct ccggttgctc ctttgtttcc tgttggtcct gttattccat ttgctccggt
tgctcctgta ttccctgtcg gacctgttat tccattcgct ccagtcgctc ctgtatttcc
tgttggtcct gtagaacctg ttgttccatt tggtccagtc gctcccatat ttcctgttgg
acccgttatt ccattcgctc cagttgctcc tgtatttcct gttggtcctg ttattccatt
tgctccggtc gctccagttg cacctctagg ccccgtcggt cctgttggac cagtagcacc
tgttggccct gttggaccta catcttgata taaacttgga cctgaaatat cactcat

### C. Clostridium difficle Spore Proteins

### 1. Putative Clostridum Spore Proteins

In one embodiment, a *Clostridium* putative spore protein (CD 2401) may be isolated as a hybridized antibody-protein complex comprising a molecular weight including, but not limited to, 140, 150, or 160 kDa. See, Figures 5 & 6. In one embodiment, the hypothetical protein (∼22 kDa) comprises an amino acid sequence of SEQ ID NO: 21 (Accession Numbers YP_001088913; GI: 126700016): 
1 MWIYQKTIQH PVNIKTCDPR MAKFLITQFG GPNGELAASL RYLSQRYTMP
51 TGNMRALLTD IGTEELAHVE LICTMVYQLT SDASPEELKA AGLGSNYAQN
101 GYGIYPTDSN GVPFDVRPIA VMSNPVTDLH EDMAAEQKAL ATYYQLINLT
151 DDVDVIDVLK FLGQREIIHY QRFGEALMDA YELEESQKMF
 encoded by a *Clostridium* putative spore protein nucleic acid SEQ ID NO: 22:

1 atgtggatat atcagaaaac tatacaacac ccagttaata taaaaacttg tgaccctaga

61 atggctaaat ttcttataac tcaatttggt gggccaaatg gggaacttgc tgcatcttta
121 agatatttaa gccaaagata tacaatgcct actggtaata tgcgtgcact tttaacagat
181 attggtacag aagaactagc tcacgttgag cttatatgta ctatggttta tcagttaact
241 tctgatgcaa gcccagaaga gttaaaagct gcaggtcttg gttcaaacta tgctcaaaat
301 ggatatggaa tttatccaac agattcaaat ggtgttccat ttgatgtaag acctatagca
361 gttatgtcaa atcccgtaac cgatttacat gaggatatgg cagctgaaca aaaagcactt
421 gcaacttatt atcaacttat aaacctaaca gatgacgttg atgttataga tgtattaaaa
481 ttcttgggtc aaagagaaat aattcactat caaagatttg gtgaagcttt aatggatgct
541 tacgagttag aagaatctca aaaaatgttc taa

In one embodiment, a *Clostriclium* putative spore surface protein (CD 589) may be isolated as a hybridized antibody-protein complex comprising a molecular weight including, but not limited to, 140, 150, or 160 kDa. See, Figures 5 & 6. In one embodiment, a hypothetical protein (∼ 22 kDa) comprises an amino acid sequence of SEQ ID NO: 23 (Accession Numbers YP 001087073; GI: 126698176): 
1 MWIYQKTLEH PVNIRQADPR MAKYIMTQLG GPNGELAAAT RYLQQRYTMP
51 TGKSRALLTD IGTEEMAHVE IISSVLYQLI GNCTPEELKA AGLGSNYANF
101 GHGLQPVDSN GVNFTTSYIN VFGDSVTDLH EDMAAEQKAL ATYYQLINLT
151 DDPDLKDILR FLGEREVVHY QRFGEALMDV YEFTECKHQF
 encoded by a *Clostridium* putative spore protein nucleic acid SEQ ID NO: 24:

1 atgtggattt atcaaaaaac actggaacat ccagttaaca taagacaagc agaccctaga
61 atggcaaaat atatcatgac tcagttggga ggacctaatg gtgagttggc agctgcaact
121 agatatcttc aacaaagata tactatgcca actggaaaat ctcgtgcact tttaactgat
181 ataggtacag aggaaatggc tcatgttgag ataatttctt cagtgttata tcaattaata
241 ggcaattgta ctccagaaga gcttaaggct gctggacttg gtagtaatta tgctaatttt
301 ggacatggtc ttcagccagt agattctaat ggagtaaact ttactacaag ttatattaat
361 gtctttggcg attcggtaac tgatttacat gaggatatgg ctgctgaaca aaaagcattg
421 gctacgtact atcaattaat aaatttaact gatgaccctg atttgaaaga tatattgaga
481 tttttgggtg agagggaagt agttcactat caaagatttg gtgaagcatt aatggatgtt
541 tatgagttta cagagtgcaa gcatcagttt taa

### 2. Pyruvate-Flavodoxin Oxidoreductases

In one embodiment, a *Clostridium* spore protein comprises a pyruvate-flavodoxin oxidoreductase protein (CD 2682) described by the amino acid sequence of SEQ ID NO: 25 (Accession Numbers YP_001089193; GI: 126700296): 
1 MAKFMKTLDG NTAAAHVAYA FTDVAAlYPI TPSSTMAEVV DEWASQGRKN
51 IFGQTVNVVE MQSEAGASGT FHGSLQGGAL TSTFTASQGL LLMIPNMYKV
101 AGELLPGVFH VSARALASQA LSIFGDHQDV MAARQTGCVL LASGSVQEVA
151 DIAPVAHLAA IEGRLPFIHF FDcJFRTSHEI QKVELLENED YASLLNFEAV
201 QAFRDNALSP NHPVARGTAQ NPDIYFQTRE ASNKYYQNIV GIVEKYMEKM
251 SNLTGRKHSL FDYYGAEDAK YVLIAMGSVT ETIEETIDYL NAKGEKYGLV
301 KVHLYRPFSM KHFLDAMPST VERICVLDRT KEPGSTGEPL YLDVRDVFYG
351 KENAPMIIGG RYGLGSKDTT PSDIKTVFDN LVSEQPKNGF TVGIVDDVTN
401 TSLTPSEPIK IASKGTIRCK FWGLGSDGTV GANKQAIKII GDHTEKYAQA
451 YFDYDSKKSG GITMSHLRFG DTPIRATYLI DEADYIACHK QSYVYQYDLL
501 KGLKKGGTFV LNTIWDQAGL EEHLPAHMKQ YIAKNDIKFY TVNAVKLGQE
551 IGLGNRINMI MQSAFFKLAE IIPEEDAVKY LKDSIVKAYG KKGEKIVNMN
601 YAAVDAGINA LVKVEVPASW ENAVDADKGE VKEPEFIKNl LRPMTAQEGN
651 ZNLPVSTFNGI EDGTFPCGTA AYEKRGIAVD VPEWIIDNCI QCNQCSFICP
701 HACIRPVLVT EEELANAPEG FEAKKALGKG LEGLKYRIQV SPLDCTGCGN
751 CADICPAKEK ALVMKPIDTQ LDTELDNWAF AVNPDEVAPK VDVMPANTVK
801 GSQFRQPLME FSGACAGCGE TAYIKVVTQL YGDRMMIANA TGCSSIWGAS
 
851 APSIPYTCNH EGKGPSWANS LFEDNAEYGF GMYTAVKQIR NKIVDAMTEL
901 VSMDICEDAK AVFTEWLDSR NDGEASKVAS AKVVELLEKP ACDCTDEKAK
951 ELVKAIKDRK DYLVKRSQWI LGGDGWAYDI GYGGLDHVLA SGEDVNVLVF
1001 DTEVYSNTGG QASKATPAAAMAKFAASGKK SRKKDLGMMAMTYGNVYVAQ
1051 VAMGADKNQF lKAVLEAEKH DGPSLIIAYAPCINHGLKEG MGRTVANEAQ
1101 AVACGYWHLY RFNPEVKAEG KNPFTLDSKE PTASFKDFIL AQVRYSAIAK
1151 QFPEEADGLF AKAEADAKER YEGYKKLAE
 encoded by a *Clostridium* pyruvate-flavodoxin oxidoreductase nucleic acid SEQ ID NO: 26:

1 ttattcagct aattttttgt atccttcata tctttcttta gcatcagctt cagctttagc
61 aaataatcca tcagcttctt ctgggaattg tttagctata gcagaatatc ttacttgtgc
121 aagtatgaaa tctttgaagc ttgcagttgg ttcttttgaa tctaaagtga atgggttttt
181 accttcagct tttacttctg gattgaatct gtataaatgc cagtatccgc aagcaacagc
241 ttgtgcttcg ttagctacag ttcttcccat accttctttt aatccatggt ttatacatgg
301 agcataagct attattaaag atggcccatc atgtttttca gcttctaaaa cggctttgat
361 aaattggttt ttatcagcac ccatagcaac ttgagctaca tatacgtttc catatgtcat
421 agccatcatt cctaaatctt tctttctaga ttttttacct gaagcagcaa acttagccat
481 agcagcagct ggagtagctt tagaagcttg acctcctgta tttgaatata cttctgtatc
541 aaatacaagt acgtttacat cttcaccaga tgcaagaaca tggtctaatc caccgtaacc
601 gatatcatat gcccaaccgt ctccacctag tatccattga gatctcttaa ctagataatc
661 ttttctatct tttatagctt ttactaattc tttagctttt tcatctgtac aatcacaagc
721 tggtttttct aataattcaa caaccttagc acttgctact ttagaagctt ctccatcatt
781 tctagaatct aaccactctg taaatactgc tttagcatct tcacagatat ccatagaaac
841 taactctgtc atagcatcta ctattttatt tcttatttgc ttaacagctg tgtacatacc
901 aaatccatat tcagcattat cctcgaataa agagtttgcc caagatggac ctttaccttc
961 atggttacaa gtataaggaa ttgatggtgc agaagctccc catatagaag aacatccagt
1021 agcattagct atcatcattc tatctccata taattgtgta actactttaa tgtacgcagt
1081 ttctccacat ccagcacatg ctcctgaaaa ctccattaat ggttgtctaa attgacttcc
1141 tttaacagta tttgcaggca taacatcaac ttttggagct acttcatcag gatttactgc
1201 aaatgcccaa ttatctaact ctgtatcaag ttgagtatct attggcttca taactaaagc
1261 tttttcttta gctggacata tgtcagcaca gtttccacat cctgtacagt caagtggact
1321 aacttgaatt ctatatttta atccttctaa tcctttacct aatgcttttt tagcttcaaa
1381 tccttctgga gcattagcta attcttcttc agtaactaat actggtctta tacatgcatg
1441 aggacatata aaagaacatt ggttacattg aatacagtta tctattatcc attcaggaac
1501 atcaacagct ataccacgtt tttcataagc tgcagtacca caagggaatg ttccatcttc
1561 tataccattg aatgtactta ctggtaagtt gtttccttct tgtgcagtca ttggtcttag
1621 tatatttttt ataaattctg gttctttaac ttctccttta tctgcatcaa cagcattttc
1681 ccaagaagct ggaacttcaa ctttaactaa agcatttatt cctgcatcaa cagcagcata
1741 gttcatatta actatttttt cacctttttt accataagct ttaactatag aatcttttaa
1801 gtatttaaca gcatcttctt ctggtattat ttcagctaat ttaaagaatg cagattgcat
1861 ttatcatgtt attctgtttc caagacctat ttcttgtcct agtttaacag cgttaactgt
1921 atagaatttt atatcatttt tagctatata ttgtttcata tgagctggta aatgttcttc
1981 aagtcctgct tggtcccata tagtgttaag tacaaatgta ccaccttttt taagaccttt
2041 taataaatca tattgataaa catatgattg tttatgacag gcaatataat cagcttcatc
2101 tattaaataa gtagctctta ttggagtatc accaaatctt aagtgagaca tagttatacc
2161 accagatttt ttagagtcat aatcaaagta agcttgagca tatttttcag tatggtcacc
2221 gattattttg atagcttgtt tattagctcc aacagtaccg tcagaaccta atccccagaa
2281 cttacatctt atagttcctt tagaagctat ttttattggc tcagatggag ttaaagaagt
2341 attagttaca tcatcaacta tacctactgt aaatccattt ttaggttgtt ctgaaactaa
2401 gttatcaaaa acagttttta tatctgaagg agtagtatct tttgaaccta atccgtatct
2461 tccacctatt atcataggag cattttcttt tccatagaat acatcacgta catctaagta
2521 taatggttca ccagttgaac ctggctcttt agttctatca agtacacata ttctttcaac
2581 agtagatggc atagcatcta agaaatgttt cattgagaat ggtctatata aatgaacttt
2641 aactaaacca tatttttctc ccttagcatt taagtaatct atagtttctt ctattgtttc
2701 tgttactgaa cccatagcta ttaatacata tttagcatct tctgctccat agtaatcaaa
2761 taaactatgt tttctacctg ttaaattact cattttttcc atgtattttt caactatacc
2821 tactatattt tgatagtatt tattagaagc ttctctagtt tggaaatata tatctgggtt

2881 ttgagcagta ccacgagcaa ctggatgatt tggagataaa gcattatctc taaatgcttg
2941 aactgcttca aagtttaata aacttgcata atcttcattt tctagtaatt caactttttg
3001 gatttcatgt gaagttctga atccatcaaa gaaatgtata aatggtaatc taccttctat
3061 tgcagctaaa tgtgcaacag gtgctatatc agctacttct tgaactgaac cagaagctaa
3121 taatacacat ccagtttgtc tagcagccat aacgtcttgg tgatccccaa atattgataa
3181 cgcttgagat gctaaagcac gagcacttac atggaatact cctggtaata actctcctgc
3241 aactttatac atgttaggta tcattaataa taaaccttga gatgctgtaa aagtagaagt
3301 taaagctcct ccttgtaaag aaccgtggaa tgttcctgat gcacctgctt ctgactgcat
3361 ttcaacaaca ttaactgttt gtccaaatat attttttctt ccttgtgatg cccactcatc
3421 aactacttca gccatagttg aagatggtgt gattggatat atagctgcta catctgtaaa
3481 cgcataggca acatgagctg cagctgtatt tccatcaagt gttttcataa acttagccat

### 3. γ-Aminobutryrate Metabolism Dehydratases/Isomerases

In one embodiment, a *Clostridium* spore protein comprises a γ-aminobutyrate metabolism dehydratase/isomerase protein (CD 2341) described by the amino acid sequence of SEQ ID NO: 27 (Accession Numbers YP_001088856; GI: 126699959): 
1 MALMTGAQYI ESLRKLNTKV YMFGEEVKNW VDHPMIRPSI NCVAATYDLA
51 HDPEYADLMT VTSNITGEKI NRFGHLHQSV DDLIKKVKMQ RLCGQKTASC
101 FQRCVGMDAF NAVYSTTFEC DKAHGTNYHD NFVKYLTYIQ ENDLVVDGAM
151 TDPKGDRSLS PSAQPDPDMF LHIVERREDG IIVRGAKAHQ TGSINSHEHL
201 IMPTISMTEA DKDYAVSFAV PSDAEGVFMI YGRQSCDTRK LEEGADVDLG
251 NKEFGGQEAL VVFDNVFVPN DRIFLCGEWD FSGMLVERFA GYHRQSYGGC
301 KVGVGDVIIG AAALAADYNG ANKASHIKDK LIEMTHLNES LYCCGIACSS
351 EGHKTEAGNY QIDLLLANVC KQNVTRFPYE IVRLAEDIAG GLMVTMPSEK
401 DFKSDLKVGT SGMTIGEVCN KYFKASSVAS TEERMRILRF LENICLGSSA
451 VGYRTESMHG AGSPQAQRIM ISRQGNINQK KELAKKIAGI KKEEALNL
 encoded by a *Clostridium* gamma aminobutyrate metabolism dehydratase/isomerase nucleic acid SEQ ID NO: 28:

1 ttataaattt aaagcttcct ctttttttat tcccgcaatt ttcttagcta attctttctt
61 ttgatttatg tttccttgtc ttgaaatcat tattctttga gcttgtggtg aacctgcccc
121 atgcattgac tcagttctat aaccaacagc agatgaacca aggcatatat tttctaagaa
181 tcttaatatt ctcattcttt cttctgttga agccactgaa gaagctttaa aatacttgtt
241 acatacttcg ccaatagtca ttccacttgt tccaacttta aggtctgatt taaagtcttt
301 ttctgaaggc atagtaacca taagccctcc agctatatct tctgcaagtc ttactatttc
361 ataaggaaat ctagttacat tttgtttaca cacatttgca agaagtaaat ctatttgata
421 gtttccagct tctgttttgt gaccttctga tgaacatgct ataccacagc aatataagct
481 ttcattaagg tgagtcattt ctattagttt atcttttata tgtgaagctt tgtttgctcc
541 attataatca gctgctaaag ctgctgctcc tattattaca tcacctactc ctactttaca
601 tccaccataa ctttgtctat ggtatcctgc aaatctctct actaacatac cagagaaatc
661 ccattctcca cataagaaaa ttctatcatt tggaacaaat acattatcaa atactactaa
721 agcttcttga cctccaaact ccttatttcc taaatcaaca tctgcacctt cttctaattt
781 tctagtgtca caagattgtc ttccataaat cataaacact ccttcagcat ctgaaggaac
841 tgcaaatgat acagcgtagt ctttatctgc ttcagtcata cttatagtag gcattataag
901 gtgctcatgt gaatttattg aaccagtttg atgtgccttt gctcctctta caattatccc
961 atcttctctt ctttctacta tatgaaggaa catatctggg tcaggttgag cactaggtga
1021 tagacttcta tctcccttag ggtcagtcat agctccatca actactaaat cgttttcttg
1081 tatataagtt aagtatttta caaaattatc atgataatta gttccatggg ctttatcaca
1141 ctcaaatgtt gttgaataca ctgcattaaa cgcatccata ccaacacatc tttggaaaca
1201 agaagctgtt ttttgaccac atagtctttg catttttact ttttttatca aatcatcaac
1261 actttgatga agatgaccaa atctatttat tttttcacct gtgatatttg aagtaactgt
1321 cattaaatct gcatattctg ggtcatgagc taagtcataa gttgctgcta cacaatttat
1381 agatggtcta atcataggat ggtcaaccca attcttaacc tcttcaccaa acatataaac
1441 cttagtattt aattttctca aactttcaat atattgagct cctgtcatta atgccat

### 4. Butyryl-CoA Dehydrogenases

In one embodiment, a *Clostridium* spore protein comprises a butyryl -CoA dehydrogenase protein (CD 1054) described by the amino acid sequence of SEQ ID NO: 29 (Accession Numbers YP_0I087535; GI: 126698638): 
1 MDLNSKKYQM LKELYVSFAE NEVKPLATEL DEEERFPYET VEKMAKAGMM
51 GIPYPKEYGG EGGDTVGYIM AVEELSRVCG TTGVILSAHT SLGSWPIYQY
101 GNEEQKQKFL RPLASGEKLG AFGLTEPNAG TDASGQQTTA VLDGDEYILN
151 GSKIFITNAI AGDIYVVMAM TDKSKGNKGI SAFIVEKGTP GFSFGVKEKK
201 MGIRGSATSE LIFEDCRIPK ENLLGKEGQG FKIAMSTLDG GRIGIAAQAL
251 GLAQGALDET VKYVKERVQF GRPLSKFQNT QFQLADMEVK VQAARHLVYQ
301 AAINKDLGKP YGVEAAMAKL FAAETAMEVT TKAVQLHGGY GYTRDYPVER
351 MMRDAKITEI YEGTSEVQRM VISGKLLK
 encoded by a *Clostridium* butyryl-CoA dehydrogenase nucleic acid SEQ ID NO: 30:

1 atggatttaa attctaaaaa atatcagatg cttaaagagc tatatgtaag cttcgctgaa
61 aatgaagtta aacctttagc aacagaactt gatgaagaag aaagatttcc ttatgaaaca
121 gtggaaaaaa tggcaaaagc aggaatgatg ggtataccat atccaaaaga atatggtgga
181 gaaggtggag acactgtagg atatataatg gcagttgaag aattgtctag agtttgtggt
241 actacaggag ttatattatc agctcataca tctcttggct catggcctat atatcaatat
301 ggtaatgaag aacaaaaaca aaaattctta agaccactag caagtggaga aaaattagga
361 gcatttggtc ttactgagcc taatgctggt acagatgcgt ctggccaaca aacaactgct
421 gttttagacg gggatgaata catacttaat ggctcaaaaa tatttataac aaacgcaata
481 gctggtgaca tatatgtagt aatggcaatg actgataaat ctaaggggaa caaaggaata
541 tcagcattta tagttgaaaa aggaactcct gggtttagct ttggagttaa agaaaagaaa
601 atgggtataa gaggttcagc tacgagtgaa ttaatatttg aggattgcag aatacctaaa
661 gaaaatttac ttggaaaaga aggtcaagga tttaagatag caatgtctac tcttgatggt
721 ggtagaattg gtatagctgc acaagcttta ggtttagcac aaggtgctct tgatgaaact
781 gttaaatatg taaaagaaag agtacaattt ggtagaccat tatcaaaatt ccaaaataca
841 caattccaat tagctgatat ggaagttaag gtacaagcgg ctagacacct tgtatatcaa
901 gcagctataa ataaagactt aggaaaacct tatggagtag aagcagcaat ggcaaaatta
961 tttgcagctg aaacagctat ggaagttact acaaaagctg tacaacttca tggaggatat
1021 ggatacactc gtgactatcc agtagaaaga atgatgagag atgctaagat aactgaaata
1081 tatgaaggaa ctagtgaagt tcaaagaatg gttatttcag gaaaactatt aaaatag

### 5. Amino Acid Aminotransferases

In one embodiment, a *Clostridium* spore protein comprises an amino acid aminotransferase protein (CD 3664) described by the amino acid sequence of SEQ ID NO: 31 (Accession Numbers YP 001090189; GI: 126701292): 
1 MAVKYAKRMQ GLQGSEIREL LKLTQQPQII SFAGGMPAPE LFPVEEMKKV
51 SVAVLEENGR SAMQYTTTEG YEPLREKIAA RMNDKNKTNV NKDDILVTSG
101 SQQGLDFAGK VFIDEGDVIL CESPSYIGAI NAFKSYQPKF IDVPTDSDGM
151 IMEELEKILE TTDRIKMIYV IPDFQNPTGR TWPLERRKKF MEIVNKFEIP
201 VIEDNPYGDL RFEGETLPSL KSMDTKGLVI FLGTFSKIFC PGYRLGWTCA
251 SPEILSKFNF AKQGADLQAS TISQMEVSKF MDMYDLDAHV DKIKAVYVKR
301 RDVMLKTMEE EFPEGLVFTH PEGGLFTWVE LPSNLNAKEL MPKCLDKNVA
351 YVAGGGFFPN GGRENTFRLN YSNMPEEKII EGIKNIAAVL KEAMGVEA
 encoded by a *Clostridium* amino acid aminotransferase nucleic acid SEQ ID NO: 32:

1 ttaagcttct acacccatag cttcttttaa tacagctgct atatttttta taccttctat

61 tattttttct tctggcatat ttgaatagtt aagtctgaaa gtattttctc taccaccatt
121 agggaagaat cctcctccag caacataagc aacattctta tctaaacatt ttggcattaa
181 ttctttagca tttaaattac ttggaagctc tacccatgta aataatccac cttctggatg
241 agtaaatact aatccttctg ggaactcttc ttccatagtc ttaagcatta catctctacg
301 tttaacataa actgctttta ttttgtcaac atgtgcatct aggtcataca tatccataaa
361 tttgcttact tccatttgag atatagttga tgcttgtaag tctgcacctt gttttgcaaa
421 gttaaactta gataaaattt ctggagaagc acaagtccat cctaatctgt atcctgggca
481 gaatatttta gagaaagttc ctaagaatat tactaatcct tttgtatcca ttgattttaa
541 agatggtaaa gtctctcctt caaatcttaa atctccatat ggattatctt ctattactgg
601 tatttcaaat ttatttacta tttccatgaa ttttttacgt ctttcaagtg gccaagttct
661 tcctgttgga ttttggaagt caggtattac gtaaatcatt tttattctat cagttgtttc
721 tagtatcttt tctaattctt ccattatcat tccatcagaa tctgttggaa catctatgaa
781 tttaggttga taagatttaa atgcgtttat agctcctatg taagatggac tttcacataa
841 aattacatca ccctcatcaa tgaatacttt tcctgcaaaa tctagacctt gttgagaacc
901 acttgtaact aatatatcat ctttgtttac attagttttg tttttatcat tcattctagc
961 tgctattttt tctcttaatg gttcatatcc ttctgttgta gtatattgca tagctgatct
1021 tccattttct tcaagcactg caactgatac ttttttcatt tcctcaactg gaaataactc
1081 tggagccggc attccaccag caaaagatat tatttgtggt tgttgagtaa gttttaaaag
1141 ctcacgtatt tctgatcctt gtaatccttg cattcttttt gcatatttaa ctgccat

6. Succinic-Semialdehyde Dehydrogenases In one embodiment, a *Clostridium* spore protein comprises a succinic-semialdehyde dehydrogenase protein (CD 2342) described by the amino acid sequence of SEQ ID NO: 33 (Accession Numbers YP_001088857; GI: 126699960): 
1 MEKAVENFED LSKEYINGYI ERARKAQREF ECYTQEQVDK IVKIVGKVVY
51 YNAEYLAKLA VEETGMGVYE DKVAKNKSKA KVIYNNLKDK KSVGIIDIDR
101 ETGITKVAKP VGVVAAITPC TNPIVTPMSN AMFALKGRNA IIITPHHKAI
151 GCSTKTVEMI NEELEKIGAP ENLIQILDQQ SRENTRNLIS SADVVIATGG
201 MGMVKAAYSS GKPALGVGAG NVQCIIDRDV DIKEAVPKII AGRIFDNGII
251 CSGEQSVIVA EEMFDKIMDE FKNNKGFIVR DKVQKEAFRN AMFVNKSMNK
301 DAVGQSVHTI AKIAGVEIPE DTKIIVIEAD GPGEEDIIAK EKMCPVISAY
351 KYKSFEEGVA IAKANLNVEG KGHSVSIHSN TVKNIEYAGE NIEVSRFVIN
401 QCCATSAGGS FFNGLAPTNT LGCGSWGNNS ISENLDYKHL INISRIAYYM
451 PENEVPTDEE LWG
 encoded by a *Clostridium* succinic-semialdehyde dehydrogenase nucleic acid SEQ ID NO: 34:

1 ttatccccaa agttcttcat ctgtagggac ttcattttct ggcatataat aagcaattct
61 agaaatatta attagatgct tataatcaag attttcagag atgctgttat ttccccaaga
121 accacaacct agagtattag taggtgcaag accattaaag aaacttcctc cagcactagt
181 agcacaacat tgattaataa caaatcttga tacttctata ttttctcctg catattctat
241 atttttaact gtatttgaat gtatagatac actatgtcct tttccttcta catttaaatt
301 tgcttttgca atagctacac cttcttcaaa acttttatac ttgtatgcag atataactgg
31 acacattttt tcttttgcaa taatatcctc ttctcctggt ccatcagctt ctattactat
421 tatttttgta tcttctggta tctcaacacc tgcaattttt gcaattgtat gaactgattg
481 acctactgca tctttattca tagatttatt tacaaacata gcatttctga aagcttcttt
541 ttgtacttta tctctaacta taaaaccttt gttattttta aattcatcca ttattttatc
601 aaacatttct tctgctacta ttactgactg ttcacctgaa caaatgatac cattatcaaa
661 aattcttcct gctatgattt taggtactgc ttctttaatg tctacatctc tatctatgat
721 acattgaaca tttcctgccc ctacaccaag tgctggtttt ccacttgagt acgcagcttt
781 taccattccc attcctccag tggcaattac tacatctgct gatgaaatta aatttcttgt
841 attttctctt gattgttggt caagtatttg tattaaattt tctggagctc ctattttttc
901 taattcttca ttaatcatct ctacagtttt tgtactacat ccaattgctt tatgatgagg
961 tgtgataata attgcatttc ttcccttaag tgcaaacatt gcattgctca taggtgtaac
1021 gattggattt gtacaaggag ttattgccgc tactactcca actggtttag ctacctttgt
1081 tattccagtt tctctatcta tatcaattat accaactgat tttttatctt ttaaattgtt
1141 atatataact tttgctttgc ttttattttt agctacctta tcttcatata ctcccattcc

1201 agtctcttct acagcaagtt tagctaaata ttcagcatta taatacacta cttttccaac
1261 tatttttact attttatcta cttgttcttg agtataacat tcaaactctc tttgagcttt
1321 tcttgctctc tcaatataac cattaatgta ttccttactt aaatcttcaa agttttccac
1381 tgctttttcc at

### 7. Rubrerythrins

In one embodiment, a *Clostridium* spore protein comprises a rubrerythrin protein (CD 1524) described by the amino acid sequence of SEQ ID NO: 35 (Accession Numbers YP_001088025; GI: 126699128): 
1 MKKFVCTVCG YIHEGDAAPA QCPVCKVGAD KFEEMKGEMV WADEHRIGVA
51 QGVDAEIIEG LRANFTGECT EVGMYLAMSR QADREGYPEV AEAYKRIAFE
101 EAEHAAKFAELLGEVVVADTKENLRVRVDAEYGATDGKLKLAKRAKELGL
151 DAIHDTVHEM CKDEARHGKA FLGLLNRHFG K
 encoded by a *Clostridium* rubrerythrin nucleic acid SEQ ID NO: 36:

1 atgaaaaaat ttgtttgtac agtatgtgga tatatacatg aaggagatgc tgcaccagca
61 caatgtccag tatgtaaagt tggagctgat aaatttgaag aaatgaaagg cgaaatggtt
121 tgggctgatg aacatagaat aggagtagct caaggtgtag atgcagaaat aatcgaagga
181 ttaagagcta actttactgg tgagtgtaca gaagtaggaa tgtatttagc aatgagtaga
241 caagctgata gagaaggtta tccagaagta gctgaagcgt ataagagaat agcttttgaa
301 gaggctgaac atgctgctaa atttgcagaa cttcttggag aagttgtagt tgcagataca
361 aaagaaaact taagagttag agttgatgct gagtatggtg caactgatgg aaaattaaaa
421 ttagctaaga gagctaaaga attaggatta gatgctatac atgatacagt acatgaaatg
481 tgtaaagacg aagctagaca tggtaaagca ttcttaggat tattaaacag acattttgga
541 aaataa

### 8. Flavoprotein Alpha Subunits

In one embodiment, a *Clostridium* spore protein comprises a flavoprotein alpha subunit protein (CD 1056) described by the amino acid sequence of SEQ ID NO: 37 (Accession Numbers YP_001087535; GI: 126698640): 
1 MGNVLVVIEQ RENVIQTVSL ELLGKATEIA KDYDTKVSAL LLGSKVEGLI
51 DTLAHYGADE VIVVDDEALA VYTTEPYTKA AYEAIKAADP IVVLFGATSI
101 GRDLAPRVSARIHTGLTADCTGLAVAEDTKLLLMTRPAFGGNIMATIVCK
151 DFRPQMSTVR PGVMKKNEPD ETKEAVINRF KVEFNDADKL VQVVQVIKEA
201 KKQVKIEDAK ILVSAGRGMG GKENLDILYE LAEIIGGEVS GSRATIDAGW
251 LDKARQVGQT GKTVRPDLYI ACGISGAIQH IAGMEDAEFI VAINKNPEAP
301 IFKYADVGIV GDVHKVLPEL ISQLSVAKEK GEVLAN
 encoded by a *Clostridium* flavoprotein alpha subunit nucleic acid SEQ ID NO: 38:

1 atgggtaacg ttttagtagt aatagaacaa agagaaaatg taattcaaac tgtttcttta
61 gaattactag gaaaggctac agaaatagca aaagattatg atacaaaagt ttctgcatta
121 cttttaggta gtaaggtaga aggtttaata gatacattag cacactatgg tgcagatgag
181 gtaatagtag tagatgatga agctttagca gtgtatacaa ctgaaccata tacaaaagca
241 gcttatgaag caataaaagc agctgaccct atagttgtat tatttggtgc aacttcaata
301 ggtagagatt tagctcctag agtttctgct agaatacata caggtcttac tgctgactgt
361 acaggtcttg cagtagctga agatacaaaa ttattattaa tgacaagacc tgcatttggt
421 ggaaatataa tggcaacaat agtttgtaaa gatttcagac ctcaaatgtc tacagttaga
481 ccaggggtta tgaagaaaaa tgaacctgat gaaactaaag aagctgtaat taaccgtttc
541 aaggtagaat ttaatgatgc tgataaatta gttcaagttg tacaagtaat aaaagaagct
601 aaaaaacaag ttaaaataga agatgctaag atattagttt ctgctggacg tggaatgggt
661 ggaaaagaaa acttagacat actttatgaa ttagctgaaa ttataggtgg agaagtttct
721 ggttctcgtg ccactataga tgcaggttgg ttagataaag caagacaagt tggtcaaact
781 ggtaaaactg taagaccaga cctttatata gcatgtggta tatctggagc aatacaacat
841 atagctggta tggaagatgc tgagtttata gttgctataa ataaaaatcc agaagctcca
901 atatttaaat atgctgatgt tggtatagtt ggagatgttc ataaagtgct tccagaactt
961 atcagtcagt taagtgttgc aaaagaaaaa ggtgaagttt tagctaacta a

### 9. 2-Ketoisovalerate Ferrodoxin Reductases

In one embodiment, a *Clostridium* spore protein comprises a 2-ketoisovalerate ferrodoxin reductase protein (CD 116) described by the amino acid sequence of SEQ ID NO: 39 (Accession Numbers YP_001086585; GI: 126697688): 
1 MAKILMKGNE AFGKAAIEAG CKYFFGYPlT PQSELPEYLS RELPKIGGAF
51 VQAESEVSAI NMVYGGAGAG ARVMTSSSSP GVALKQEGIT YAVGAEVPCV
101 VLNVMRGGPG LGSIQPSQAD YFMSTRGGGN GDYRTPVFAP ATVQEAVDMI
151 MEAFDVADYY RSPVMVVADG MIGQMMEPVE FRAPEKKREL PPKDWATVGT
201 KGKRKPNVIN SLYLEPEVLE DHCWHLQEKF DAMEKNEVQY EMYKTEDAEF
251 VFAAYGTTSR VVKSAIDILR EEGIKAGLIR PKVLWPFPFE AFNQIPNARN
301 ILTVEMSMGQ MVEDVKMAVE GKLPVYFHGR PGGMTPTPAE IVEKAKKIIA
351 GELVAGGAR
 encoded by a *Clostridium* flavoprotein alpha subunit nucleic acid SEQ ID NO: 40::

1 atggctaaga tacttatgaa aggtaacgaa gcatttggaa aagcagctat agaagctggt
61 tgcaaatatt tcttcggtta cccaataact ccacaaagtg aattaccaga atatttatca
121 agagagttac ctaaaatagg tggagcgttc gttcaagctg aatcagaagt ttctgcgata
181 aacatggttt atggtggagc aggtgcagga gcaagagtta tgacttcttc atcttcacca
241 ggagttgctt taaagcaaga aggtataaca tatgcagtag gagcagaagt tccttgtgtt
301 gtacttaatg taatgagagg tggtccagga cttggaagta tacaaccttc acaagctgac
361 tactttatgt ctacaagagg tggaggaaat ggagactaca gaactccagt attcgcacca
421 gctacagttc aagaagctgt tgatatgata atggaagctt ttgacgttgc agattattac
481 agatcaccag ttatggtagt tgcagatggt atgataggtc aaatgatgga gccagttgaa
541 tttagagctc ctgagaagaa aagagaattg cctcctaaag attgggcaac tgttggaact
601 aaaggaaaaa gaaaacctaa tgtaataaac tctctatact tagagccaga agtactagaa
661 gaccattgtt ggcatttaca agaaaaattt gatgcaatgg aaaagaatga agtacaatac
721 gaaatgtaca aaacagaaga tgctgaattt gtatttgcag cttatggaac tacttcaaga
781 gtagtaaaaa gcgcaattga tatattaaga gaagaaggaa taaaagcagg tcttataaga
841 cctaaagtat tatggccatt cccattcgaa gcattcaacc aaattcctaa tgctagaaac
901 atattgactg ttgaaatgag tatgggacaa atggtggaag atgttaaaat ggcagtagaa
961 ggtaaattac cagtttactt ccatggaaga ccaggaggaa tgactccaac accagcagaa
1021 atagttgaaa aagctaaaaa aattattgcc ggagaattgg ttgcaggagg tgctagataa

### II. Antibody Inhibition Of Microbial Spore Binding

Also described are mehtods of inhibiting microbial spore binding and/or attachment to inanimate and/or animate surfaces using antibodies. In one embodiment, the antibodies inhibit *Clostridium* spore binding. These antibodies include, but are not limited to, polyclonal and monoclonal antibodies. In addition, chimeric antibodies may be produced, for example, by splicing mouse antibody genes to human antibody genes. Single chain antibodies are also contemplated as well as antibody fragments generated by pepsin digestion of the antibody molecule or by reduction ofthe disulfide bridges of the Fab fragments.

### A. Antibody Generation Methods

Isolated antibodies or fragments thereof can be provided. (i.e., for example, polyclonal, monoclonal, and/or Fab's). In one embodiment, there are provided monoclonal antibodies that specifically inhibit the binding of microbial spore surface proteins to various surfaces (i.e., for example, animate surfaces and/or inanimate surfaces). An antibody against a protein described may be any monoclonal or polyclonal antibody, as long as it can recognize the protein. Antibodies can be produced by using a protein as the antigen according to a conventional antibody or antiserum preparation process. Any suitable method may be used to generate the antibodies used in the described methods and compositions including but not limited to, those disclosed herein. For example, for preparation of a monoclonal antibody, protein, as such, or together with a suitable carrier or diluent is administered to an animal (e.g., a mammal) under conditions that permit the production of antibodies (i.e., for example, immunization). Normally, the protein is administered once every 2 weeks to 6 weeks, in total, about 2 times to about 10 times. Animals suitable for use in such methods include, but are not limited to, primates, rabbits, dogs, guinea pigs, mice, rats, sheep, goats, etc. and may be immunized by injection with a full-length protein or any portion, fragment or oligopeptide which retains immunogenic properties. Depending on the host species, various adjuvants may be used to increase immunological response. For enhancing the antibody production capability, complete or incomplete Freund's adjuvant may be administered. Such adjuvants include, but are not limited to, Freund's, mineral gels such as aluminum hydroxide, and surface active substances such as lysolecithin, pluronic polyols, polyanions, peptides, oil emulsions, keyhole limpet hemocyanin, and dinitrophenol. BCG (Bacillus Calmette-Guefin) and Cornebacterium parrum are potentially useful adjuvants.

Monoclonal antibodies may be prepared using any technique which provides for the production of antibody molecules by continuous cell lines in culture. These include, but are not limited to hybridoma techniques. Koehler et al. Nature 256:495-497(1975*);* Kosbor et al., Immunol Today 4:72 (1983); Cote et al., Proc NatZAcad Sci 80:2026.-2030 (1983); Cole et al., In: Monoclonal Antibodies and Cancer Therapy, Alan R Liss Inc, New York, N.Y., pp 77-96 (1985).

For preparing monoclonal antibody-producing cells, an individual animal (e.g., a mouse) whose antibody titer has been confirmed is selected, and 2 days to 5 days after the final immunization, its spleen or Ymph node is harvested and antibody-producing cells contained therein are fused with myeloma cells to prepare the desired monoclonal antibody producer hybridoma. Measurement of the antibody titer in antiserum can be carried out, for example, by reacting the labeled protein, as described hereinafter and antiserum and then measuring the activity of the labeling agent bound to the antibody. The cell fusion can be carried out according to known methods, for example, the method described by Koehler and Milstein (Nature 256:495 [1975]). As a fusion promoter, for example, polyethylene glycol (PEG) or Sendai virus (HVJ), preferably PEG is used.

Examples of myeloma cells include NS-1, P3U1, SP2/0, AP-1 and the like. The proportion of the number of antibody producer cells (i.e., for example, spleen cells) and the number of myeloma cells to be used is preferably about 1:1 to about 20: 1. PEG (preferably PEG IOOO-PEG 6000) is preferably added in concentration of about 10% to about 80%. Cell fusion can be carried out efficiently by incubating a mixture ofboth cells at about 20°C to about 40°C, preferably about 30°C to about 37°C for about 1 minute to 10 minutes.

Various methods may be used for screening for a hybridoma producing the antibody (e.g., against a tumor antigen or autoantibody). For example, where a supernatant of the hybridoma is added to a solid phase (e.g., microplate) to which antibody is adsorbed directly or together with a carrier and then an anti-immunoglobulin antibody (if mouse cells are used in cell fusion, anti-mouse immunoglobulin antibody is used) or Protein A labeled with a radioactive substance or an enzyme is added to detect the monoclonal antibody against the protein bound to the solid phase. Alternately, a supernatant ofthe hybridoma is added to a solid phase to which an anti-immunoglobulin antibody or Protein A is adsorbed and then the protein labeled with a radioactive substance or an enzyme is added to detect the monoclonal antibody against the protein bound to the solid phase.

Selection of the monoclonal antibody can be carried out according to any known method or its modification. Normally, a medium for animal cells to which HAT (hypoxanthine, aminopterin, thymidine) are added is employed. Any selection and growth medium can be employed as long as the hybridoma can grow. For example, RPMI 1640 medium containing 1% to 20%, preferably 10% to 20% fetal bovine serum, GIT medium containing 1% to 10% fetal bovine serum, a serum free medium for cultivation of a hybridoma (SFM-I01, Nissui Seiyaku) and the like can be used. Normally, the cultivation is carried out at 20°C to 40°C., preferably 37°C for about 5 days to 3 weeks, preferably 1 week to 2 weeks under about 5% CO₂ gas. The antibody titer of the supernatant of a hybridoma culture can be measured according to the same manner as described above with respect to the antibody titer of the anti-protein in the antiserum.

Separation and purification of a monoclonal antibody can be carried out according to the same manner as those of conventional polyclonal antibodies such as separation and purification of immunoglobulins, for example, salting-out, alcoholic precipitation, isoelectric point precipitation, electrophoresis, adsorption and desorption with ion exchangers (e.g., DEAE), ultracentrifugation, gel filtration, or a specific purification method wherein only an antibody is collected with an active adsorbent such as an antigenbinding solid phase, Protein A or Protein G and dissociating the binding to obtain the antibody.

Polyclonal antibodies may be prepared by any known method or modifications of these methods including obtaining antibodies from patients. For example, a complex of an immunogen (an antigen against the protein) and a carrier protein is prepared and an animal is immunized by the complex according to the same manner as that described with respect to the above monoclonal antibody preparation. A material containing the antibody against is recovered from the immunized animal and the antibody is separated and purified.

As to the complex of the immunogen and the carrier protein to be used for immunization of an animal, any carrier protein and any mixing proportion of the carrier and a hapten can be employed as long as an antibody against the hapten, which is crosslinked on the carrier and used for immunization, is produced efficiently. For example, bovine serum albumin, bovine cycloglobulin, keyhole limpet hemocyanin, etc. may be coupled to an hapten in a weight ratio of about 0.1 part to about 20 parts, preferably, about 1 part to about 5 parts per 1 part ofthe hapten.

In addition, various condensing agents can be used for coupling of a hapten and a carrier. For example, glutaraldehyde, carbodiimide, maleimide activated ester, activated ester reagents containing thiol group or dithiopyridyl group, and the like find use with the described methods. The condensation product as such or together with a suitable carrier or diluent is administered to a site of an animal that permits the antibody production. For enhancing the antibody production capability, complete or incomplete Freund's adjuvant may be administered. Normally, the protein is administered once every 2 weeks to 6 weeks, in total, about 3 times to about 10 times.

The polyclonal antibody is recovered from blood, ascites and the like, of an animal immunized by the above method. The antibody titer in the antiserum can be measured according to the same manner as that described above with respect to the supernatant ofthe hybridoma culture. Separation and purification of the antibody can be carried out according to the same separation and purification method of immunoglobulin as that described with respect to the above monoclonal antibody.

The protein used herein as the immunogen is not limited to any particular type of immunogen. For example, a protein expressed resulting from a virus infection (further including a gene having a nucleotide sequence partly altered) can be used as the immunogen. Further, fragments of the protein may be used. Fragments may be obtained by any methods including, but not limited to expressing a fragment ofthe gene, enzymatic processing ofthe protein, chemical synthesis, and the like.

For the production of antibodies in the context of the described methods, any antigenic portion of a microbial spore protein amino acid sequence contemplated herein (i.e., for example, SEQ ID NOs:1, 3, 5, 7, or 9) can be used either alone ,or fused with amino acids of another protein (i.e., for example, glutathione to produce an antibody against a chimeric molecule). Such antibodies include, but are not limited to, polyclonal, monoclonal, chimeric, single chain, Fab fragments and fragments produced by a Fab expression library.

Neutralizing antibodies, i.e., those which inhibit dimer formation, are especially preferred for diagnostics and therapeutics.

Polypeptides to be used for antibody induction need not retain biological activity; however, the protein fragment, or oligopeptide should be antigenic. Peptides used to induce specific antibodies may have an amino acid sequence comprising at least five amino acids, preferably at least 10 amino acids. Preferably, they should mimic a portion of the amino acid sequence of the natural protein and may contain the entire amino acid sequence of a small, naturally occurring molecule. Short stretches of an amino acid sequence may be fused with those of another protein including, but not limited to, keyhole limpet hemocyanin and antibody produced against a chimeric molecule.

Antibodies can also be used to detect polypeptides in samples including, but not limited to, body fluids, cell extracts, tissue extracts, or surface swipes using techniques including, but not limited to, enzyme linked immunosorbent assay (ELISA), radioimmunoassay (RIA) and fluorescent activated cell sOliing (FACS). Reporter molecules can be joined to the polypeptides and antibodies to facilitate detection of polypeptide-antibody binding.

### B. Methods For Antibody Inhibition Of Microbial Spore Surface Protein Binding

In one embodiment, there is contemplated a screening method comprising a monoclonal and/or polyclonal antibody having affinity to a *Clostridium* spore surface protein.

It has been reported that mice immunized with a 1:1 mixture of *B. cereus T* spores and *Clostridium sporogenes* PA3679 spores resulted in the generation of 397 hybridomas that were screened for their reactivities with 10⁷ *B. cereus* spores and for their reactivities with 10⁷ C. *sporogenes* spores. Apparently, nine hybridomas produced antibodies specific for *B*. *cereus T* spores while fifteen hybridomas produced antibodies specific for C. *sporogenes* spores. Seven hybridomas produced antibodies which cross-reacted with *B. cereus T* and C. *sporogenes* spores. Quinlan et al., "Monoclonal antibodies for use in detection of Bacillus and Clostridium spores" Appl Environ Microbiol. 63:482-487 (1997). Two of the hybridomas producing antibodies specific for C. *sporogenes* spores were subcloned and the resulting IgM antibodies were designated C33 and C225.

The antibodies were examined qualitatively (visually) for their reactivities with a range of *Bacillus*, *Clostridium,* and *Desulfotomaculum* spores by using a dot blot format. Antibody C33 reacted strongly with C. *sporogenes* spores and weakly with C. *perfringens* spores. Antibody C225 reacted weakly with *B. megaterium* spores and strongly with spores of *B. stearothermophilus,* C. *perfringens,* and C. *sporogenes.* Immunocytochemical localization ofthe antigen in C. *sporogenes* indicated that it is present on the exosporium and the outer cortex region of the spore.

While less information is available about C. *sporogens* spore antigens, it has been demonstrated that specific spore antigens are distinct from vegetative antigens. Norris J., "Bacterial spore antigens: a review" J. Gen. Microbiol. 28:393-408 (1962); and Princewell, T., "Spore antigens of Clostridium sporogenes" J. Med. Microbiol. 12: 29-41 (1979). Although it is not necessary to understand the mechanism of an invention, it is believed that polyclonal antibodies raised to *Clostridium* spore antigens are common to more than one specific of *Clostridium.* Foegeding et al., "Polyclonal antibodies in an enzyme-linked immunosorbent assay (ELISA) to detect bacterial spores" J. Rapid Methods Automat. Microbiol. 2:135-150 (1993).

Also described is a method whereby a monoclonal and/or a polyclonal antibody has affinity to a *Clostridium* spore surface protein (i.e., for example, CD1581) is used to disrupt the binding ofa *Clostridium* spore to a surface. In one embodiment, the surface may be selected from the group including, but not limited to, plastic, ceramic, stainless steel, iron steel, vinyl, tile, wood laminate, skin (i.e., for example, pigskin), internal body tissues, clothing, or environmental protection suits.

### C. Antibody Inhibition Of Microbial Spore Binding

A modified cup scrub method was used to evaluate the ability of C. *difficile* spores to bind to a human skin surrogate (i.e., for example, pigskin). See, Example X. The pigskin was first washed using a non-antimicrobial soap for 30 s followed by water rinse for 30 s. After washing the pigskin, up to 50 µl of spores were added to a 20 mm diameter area on the surface ofthe skin. The spores were allowed to dry for up to an hour and then removed according to the cup scrub method. This experiment determined the ability of C. *diffcile* spores to bind to the skin surrogate. Preliminary data has shown that up to 80% of spores can be removed from the surface of the pigskin. In subsequent experiments, spores were first reacted with C. *difficile* spore surface antibodies prior to being applied to the skin surface. The ability to remove the spores from the skin was again determined using the cup scrub method. Preliminary data has shown the ability to remove greater than 80% of the spores from the pigskin, suggesting the antibody is preventing the spores from binding to the skin.

### III. Microbial Spore Surface Protein Inhibition Of Microbial Spore Binding

Also described is a method comprising inhibiting microbial spore binding using an infection control agent, wherein the agent comprises at least a portion of a microbial spore protein. Although it is not necessary to understand the mechanism of an invention, it is believed that the infection control agent competes for surface binding sites to which the microbial spore has similar affinity. It is further believed that the resultant competitive binding either displaces previously bound microbial spores from the surface attachment site and/or is sterically prevented from binding due to the presence of the at least a portion of the microbial spore surface protein.

In one embodiment, the spore surface protein may be derived from microbial organisms selected from the group comprising bacteria, viruses, fungi, algae, or molds. In one embodiment, the method further comprises preventing attachment of a microbial spore to a surface with the spore surface protein. In one embodiment, the spore surface proteins are formulated with non-spore surface protein compounds are selected from the group comprising small molecules, proteins, or nucleic acids. In one embodiment, the spore surface proteins are formulated with surface acting agents which may include, but are not limited to, surfactants, soaps, detergents, foaming agents, wetting agents, and aerosolizing agents. In one embodiment, the compounds are selected form the class of compounds known as solvents.

### A. Full Length Spore Surface Proteins

In one embodiment, at least a portion of a microbial spore surface protein comprises a full length microbial spore surface protein. In one embodiment, the full length microbial spore surface protein comprises a *Clostridium* spore surface protein. In one embodiment, the *Clostridium* full length spore surface protein comprises a C. *difficile* full length spore surface protein. In one embodiment, the C. *difficile* full length spore surface proteins may be selected from the group comprising SEQ ID NO: 1, SEQ ID NO:3, SEQ ID NO:5, SEQ ID NO: 7 SEQ ID NO: 9 .

In one embodiment, the full length microbial spore surface protein comprises a *Bacillus* spore surface protein. In one embodiment, the *Bacillus* full length spore surface protein comprises a *B. anthracis* full length spore surface protein. In one embodiment, the *B. anthracis* full length spore surface proteins may be selected from the group comprising SEQ ID NO:41, SEQ ID NO: 43 SEQ ID NO: 45 SEQ ID NO: 47 SEQ ID NO:49.

### B. Truncated Spore Surface Proteins

In one embodiment, the at least a portion of a microbial spore surface protein comprises a truncated microbial spore surface protein. Although it is not necessary to understand the mechanism of an invention, it is believed that such truncated microbial spore surface proteins maybe constructed by recombinant protein expression platforms *(infra)* wherein a portion of a nucleic acid sequence encoding a microbial spore surface protein is integrated within a vector and placed in operable combination with a compatible promoter. Alternatively, such truncated microbial surface proteins may be created by digesting full length microbial spore surface proteins using anyone, or more, of peptidase enzymes previously reported. Such peptidase enzymes may comprise enzymes including but not limited to endopeptidases, exopeptidases, amidopeptidases, and/or carboxypeptidases.

In one embodiment, the truncated microbial spore surface protein is derived from a *Clostridium* spore surface protein. In one embodiment, the *Clostridium* truncated spore surface protein comprises a C. *difficile* truncated spore surface protein. In one embodiment, the C. *difficile* truncated spore surface protein may be derived from a *Clostridium* hypothetical protein (CD 1581) as listed in Table III:

**Table III. Clostridium difficile Spore Surface Protein Truncated Peptide Fragments**

| Peptide Fragment | Sequence Identification Number |
|---|---|
| MENKKCYSED | SEQ ID NO: 67 |
| WYERGESTAK. | SEQ ID NO:: 68 |
| WFQNDREEYE | SEQ IDNO: 69 |
| REAYDEDRER | SEQ ID NO: 70 |
| RGSNCGCSDS | SEQ ID NO: 71 |
| GENRPRNCER | SEQ ID NO: 72 |
| FRREAEIRER | SEQ ID NO: 73 |
| EAREAFCESS | SEQ ID NO:: 74 |
| EKKKEALAYE | SEQ ID NO: 75 |
| CEARKLWEEA | SEQ ID NO: 76 |
| EKYWDEYSKY | SEQ ID NO: 77 |
| NYKGIEYLAE | SEQ ID NO: 78 |
| AARLFDEGME | SEQ ID NO: 79 |
| CEARRNGNNG | SEQ ID NO: 80 |
| GNNNNCCHKC | SEQ ID NO: 81 |
| HKCNCNCCRK | SEQ ID NO: 82 |

In one embodiment, the C. *difficile* truncated spore surface protein may be derived from a *Clostridium* hypothetical protein (CD 1067) as listed in Table IV:

**Table IV. Clostridium difficile Spore Surface Protein Truncated Peptide Fragments**

| Peptide Fragment | Sequence Identification Number |
|---|---|
| MQDYKKNKRR | SEQ ID NO: 83 |
| MMNQPMSTMN | SEQ ID NO: 84 |
| EEEVYTDEIN | SEQ ID NO: 85 |
| SEDMRGFKKS | SEQ ID NO: 86 |
| HHHNGCNTDN | SEQ ID NO: 87 |
| KCECHDDCNP | SEQ ID NO: 88 |
| CNPCNPCKPN | SEQ ID NO: 89 |
| PCNPCKPNPC | SEQ ID NO:: 90 |
| DDNCGCHDNC | SEQ ID NO: 91 |
| KCDCEPCEMD | SEQ ID NO: 92 |
| SDECFENKCG | SEQ ID NO: 93 |
| PECCNPISPR | SEQ ID NO:: 94 |
| NFSVSNAVPF | SEQ ID NO: 95 |
| AIEANRIFDT | SEQ ID NO: 96 |
| MQFQTFTDAT | SEQ ID NO: 97 |
| GPNGEPLTFE | SEQ ID NO: 98 |
| TEVVEVFGSV | SEQ ID NO: 99 |
| PSAGQASVTI | SEQ ID NO: 100 |
| EKICLSNDGI | SEQ ID NO: 101 |
| VIDTGMTTLE | SEQ ID NO: 102 |
| DFDLDPLGDI | SEQ ID NO: 103 |
| VGRNCETTFE | SEQ ID NO: 104 |
| FAVCGERNSE | SEQ ID NO: 105 |
| CCRQGKGKSV | SEQ ID NO: 106 |
| AYKQRGLTVA | SEQ ID NO: 107 |
| VRNLVLELRG | SEQ ID NO: 108 |
| RCGCTEFVAL | SEQ ID NO: 109 |
| AFPAVRAGGG | SEQ ID NO: 110 |
| CKRRVDYVEF | SEQ ID NO: 111 |
| TFNTLSAPIC | SEQ ID NO: 112 |
| LPADGRAVTL | SEQ ID NO: 113 |
| RQEYQTNLTV | SEQ ID NO: 114 |
| DCIGKSILKL | SEQ ID NO: 115 |
| ECNECCEPFY | SEQ ID NO: 116 |
| ELIIPNDIDL | SEQ ID NO: 117 |
| VLCLQETVST | SEQ ID NO: 118 |
| LISEQIVVLA | SEQ ID NO: 119 |
| SPNPIQPRLV | SEQ ID NO: 120 |
| DTFSKVCDFS | SEQ ID NO: 121 |
| QCGPNHGSGK | SEQ ID NO: 122 |
| PSCHR | SEQ ID NO: 123 |

In one embodiment, the C. *difficile* truncated spore surface protein may be derived from a *Clostridium* hypothetical protein (CD 3620) as listed in Table V:

**Table V. Clostridium difficile Spore Surface Protein Truncated Peptide Fragments**

| Peptide Fragment | Sequence Identification Number |
|---|---|
| MYDDYKYNKC | SEQ ID NO: 124 |
| NKCYNDYEEM | SEQ ID NO: 125 |
| THVHEYSESV | SEQ ID NO: 126 |
| KLAEECEDRH | SEQ ID NO: 127 |
| NHRAAGVTGE | SEQ ID NO: 128 |
| AIPINGGTNH | SEQ ID NO: 129 |
| VHKINDNVDF | SEQ ID NO: 130 |
| LDHFHKICVT | SEQ ID NO: 131 |
| TGPAIRIPGT | SEQ ID NO: 132 |
| DKHIHLICGE | SEQ ID NO: 133 |
| TTVNDGHCHK | SEQ ID NO: 134 |
| FLFTTQIEAPLV | SEQ ID NO: 135 |

In one embodiment, the C. *difjicile* truncated spore surface protein may be derived from a *Clostridium* hypothetical protein (CD 1613) as listed in Table VI:

**Table VI. Clostridium difficile Spore Surface Protein Truncated Peptide Fragments**

| Peptide Fragment | Sequence Identification Number |
|---|---|
| MENNKCREDF | SEQ ID NO: 136 |
| RFTQEYEEDY | SEQ ID NO: 137 |
| PNTNERYYEN | SEQ ID NO: 138 |
| YQVADRYYNY | SEQ ID NO: 139 |
| PNKYKEPKIK | SEQ ID NO: 140 |
| QCCCKKSMRE | SEQ ID NO: 141 |
| ALELLRYDAL | SEQ ID NO: 142 |
| RPFVNFNQFA | SEQ ID NO: 143 |
| FISDFFIVGA | SEQ ID NO: 144 |
| NLVGIDLSAP | SEQ ID NO: 145 |
| PKDNLSGLDG | SEQ ID NO: 146 |
| TFERFSACNC | SEQ ID NO: 147 |
| DLIDIAGRVS | SEQ ID NO: 148 |
| YPIPVPLTLE | SEQ ID NO: 149 |
| GLINTIGTIP | SEQ ID NO: 150 |
| GVAELIALID | SEQ ID NO: 151 |
| AVIPPTIDLG | SEQ ID NO: 152 |
| AILDAILAAI | SEQ ID NO: 153 |
| IDFILAASTP | SEQ ID NO: 154 |
| LANVDLASLC | SEQ ID NO: 155 |
| NLKAVAFDIT | SEQ ID NO: 156 |
| PADYEDFIAS | SEQ ID NO: 157 |
| LGYYLDKKHY | SEQ ID NO: 158 |
| KECNCNCDCD | SEQ ID NO: 159 |
| DCCCNKGILD | SEQ ID NO: 160 |
| NLYMSNINNQ | SEQ ID NO: 161 |
| VTWAGSLVL | SEQ ID NO: 162 |
| TGVEVLGKKN | SEQ ID NO: 163 |
| DVIVLGNSND | SEQ ID NO: 164 |
| SRIYFVCVDS | SEQ ID NO: 165 |
| IDYIA | SEQ ID NO: 166 |

In one embodiment, a *Clostridium* truncated spore surface protein may be derived from a hypothetical protein (CD 1711) as listed in Table VII:

**Table VII. Clostridium difficile Spore Surface Protein Truncated Peptide Fragments**

| Peptide Fragment | Sequence Identification Number |
|---|---|
| MLIVTTEKVE | SEQ ID NO: 167 |
| GKKISKVLGL | SEQ ID NO: 168 |
| VRGSTIRAKH | SEQ ID NO: 169 |
| VGKDIGASFK | SEQ ID NO: 170 |
| NLVGGELTGY | SEQ ID NO: 171 |
| NEMLTEARQI | SEQ ID NO: 172 |
| AIGRMVEDAE | SEQ ID NO: 173 |
| AKGANAVIAF | SEQ ID NO: 174 |
| RLSSASVMQG | SEQ ID NO: 175 |
| AAEMLAYGTA | SEQ ID NO: 176 |
| WLEDDNSILEK | SEQ ID NO: 177 |

In one embodiment, a *Clostridium* truncated spore surface protein may be derived from a hypothetical protein (CD 0881) as listed in Table VIII:

**Table VIII. Clostridium difficile Spore Surface Protein Truncated Peptide Fragments**

| Peptide Fragment | Sequence Identification Number |
|---|---|
| MGTKIVLSIV | SEQ ID NO: 178 |
| LIWWAISL | SEQ ID NO: 179 |
| TCIRVIKQSK | SEQ ID NO: 180 |
| VGIIMRLGKF | SEQ ID NO: 181 |
| QKVAETGVHF | SEQ ID NO: 182 |
| LIPFLDKMAY | SEQ ID NO: 183 |
| VIDLREIVID | SEQ ID NO: 184 |
| FPPQPVITKD | SEQ ID NO: 185 |
| NVTMQIDTW | SEQ ID NO: 186 |
| YYKVTDPVRY | SEQ ID NO: 187 |
| VFEIANPIAA | SEQ ID NO: 188 |
| IENLTATTLR | SEQ ID NO: 189 |
| NIIGELDLDE | SEQ ID NO: 190 |
| TLTSRDIINV | SEQ ID NO: 191 |
| KMRTILDEAT | SEQ ID NO: 192 |
| DKWGIKVNRV | SEQ ID NO: 193 |
| ELKMMPPQD | SEQ ID NO: 194 |
| IQVAMEKQMR | SEQ ID NO: 195 |
| AERERREAIL | SEQ ID NO: 196 |
| QAEGNKSAAI | SEQ ID NO: 197 |
| LQAEGEKQSA | SEQ ID NO: 198 |
| ILTAEAKKEA | SEQ ID NO: 199 |
| MVRVAEGEKE | SEQ ID NO: 200 |
| SAILVAEGEA | SEQ ID NO: 201 |
| EAIRQTAIAK | SEQ ID NO: 202 |
| AQGEAEMIKR | SEQ ID NO: 203 |
| TQMATAEGLK | SEQ ID NO: 204 |
| LVFSAMKEAD | SEQ ID NO: 205 |
| IDNMLALKS | SEQ ID NO: 206 |
| MEALEKMAEG | SEQ ID NO: 207 |
| KSTKLVLPSE | SEQ ID NO: 208 |
| AVNFLGTFKG | SEQ ID NO: 209 |
| IKEVMSDDNK | SEQ ID NO: 210 |
| EVLDIKEVLN | SEQ ID NO: 211 |
| DNESLKK | SEQ ID NO: 212 |

In one embodiment, a *Clostridium* truncated spore surface protein may be derived from a hypothetical protein (eDI511) as listed in Table IX:

**Table IX. Clostridium difficile Spore Surface Protein Truncated Peptide Fragments**

| Peptide Fragment | Sequence Identification Number |
|---|---|
| MIDNQKYVIL | SEQ ID NO: 213 |
| SLELHLFFSR | SEQ ID NO: 214 |
| IMKEHALFLE | SEQ ID NO: 215 |
| AGFTNKNYNL | SEQ ID NO: 216 |
| AMEADHYKKQ | SEQ ID NO: 217 |
| FEDLLSYTVS | SEQ ID NO: 218 |
| ASNGIIRPDI | SEQ ID NO: 219 |
| LYSEELVTTL | SEQ ID NO: 220 |
| TSVAEQKTEE | SEQ ID NO: 221 |
| FTGIEINKNI | SEQ ID NO: 222 |
| TTRELNLQSG | SEQ ID NO: 223 |
| VNPQVGQDLV | SEQ ID NO: 224 |
| NYVAQLNSDA | SEQ ID NO: 225 |
| IRLLDGLINF | SEQ ID NO: 226 |
| KERVLDGVLS | SEQ ID NO: 227 |
| CTIFTSNYPL | SEQ ID NO: 228 |
| LLEHIIHEAN | SEQ ID NO: 229 |
| LYRSYVVDLE | SEQ ID NO: 230 |
| NKIDIESKNA | SEQ ID NO: 231 |
| KEIELFWDHI | SEQ ID NO: 232 |
| MMEHALFMRG | SEQ ID NO: 233 |
| LLDPSEGELI | SEQ ID NO: 234 |
| NTSNDFAIKF | SEQ ID NO: 235 |
| NELIEKTNEM | SEQ ID NO: 236 |
| TDSNIKNITE | SEQ ID NO: 237 |
| ETLNETVEFK | SEQ ID NO: 238 |
| DFKEAGASGI | SEQ ID NO: 239 |
| EQCKIKSIIL | SEQ ID NO: 240 |
| PLLADHVLRE | SEQ ID NO: 241 |
| ANHYIRILES | SEQ ID NO: 242 |
| YKNM | SEQ ID NO: 243 |

In one embodiment, the C. *difficile* truncated spore surface protein may be derived from a collagen-like protein as listed in Table X.

**Table X. Clostridium difficile Spore Surface Protein Truncated Peptide Fragments**

| Peptide Fragment | Sequence Identification Number |
|---|---|
| MRNIILYLND | SEQ ID NO: 244 |
| DTFISKKYPD | SEQ ID NO: 245 |
| KNFSNLDYCL | SEQ ID NO: 246 |
| IGSKCSNSFV | SEQ ID NO: 247 |
| KEKLIIFFKV | SEQ ID NO: 248 |
| RIPDILKDKS | SEQ ID NO: 249 |
| ILKAELFIHI | SEQ ID NO: 250 |
| DSNKNHIFKE | SEQ ID NO: 251 |
| KVDIEIKRIS | SEQ ID NO: 252 |
| EYYNLRTITW | SEQ ID NO: 253 |
| NDRVSMENIR | SEQ ID NO: 254 |
| GYLPIGISDT | SEQ ID NO: 255 |
| SNYICLNITG | SEQ ID NO: 256 |
| nKAWAMNKY | SEQ ID NO: 257 |
| PNYGLALSLN | SEQ ID NO: 258 |
| YPYQILEFTS | SEQ ID NO: 259 |
| SRGCNKPYIL | SEQ ID NO: 260 |
| VTFEDRIIDN | SEQ ID NO: 261 |
| CYPKCECPPI | SEQ ID NO: 262 |
| RITGPMGPRG | SEQ ID NO: 263 |
| ATGSTGPMGV | SEQ ID NO: 264 |
| TGPTGSTGAT | SEQ ID NO: 265 |
| GSIGPTGPTG | SEQ ID NO: 266 |
| NTGATGSIGP | SEQ ID NO: 267 |
| TGVTGPTGST | SEQ ID NO: 268 |
| GATGSIGPTG | SEQ ID NO: 269 |
| VTGPTGNTGV | SEQ ID NO: 270 |
| TGSIGPTGAT | SEQ ID NO: 271 |
| GPTGNTGVTG | SEQ ID NO: 272 |
| SIGPTGVTGP | SEQ ID NO: 273 |
| TGNTGEIGPT | SEQ ID NO: 274 |
| GATGPTGVTG | SEQ ID NO: 275 |
| SIGPTGATGP | SEQ ID NO: 276 |
| TGEIGPTGAT | SEQ ID NO: 277 |
| ATGPTGATGV | SEQ ID NO: 278 |
| TGEIGPTGEI | SEQ ID NO: 279 |
| GVTGATGPTG | SEQ ID NO: 280 |
| VTGEIGPTGA | SEQ ID NO: 281 |
| TGPTGNTGVT | SEQ ID NO: 282 |
| GEIGPTGATG | SEQ ID NO: 283 |
| PTGNTGVTGE | SEQ ID NO: 284 |
| IGPTGATGPT | SEQ ID NO: 285 |
| GVTGEIGPTG | SEQ ID NO: 286 |
| NTGATGSIGP | SEQ ID NO: 287 |
| TGVTGPTGAT | SEQ ID NO: 288 |
| GSIGPTGATG | SEQ ID NO: 289 |
| ATGVTGPTGP | SEQ ID NO: 290 |
| TGATGNSSQP | SEQ ID NO: 291 |
| VANFLVNAPS | SEQ ID NO: 292 |
| PQTLNNGDAI | SEQ ID NO: 293 |
| TGWQTIIGNS | SEQ ID NO: 294 |
| SSITVDTNGT | SEQ ID NO: 295 |
| FTVQENGVYY | SEQ ID NO: 296 |
| ISVSVALQPG | SEQ ID NO: 297 |
| SSSINQYSFA | SEQ ID NO: 298 |
| ILFPILGGKD | SEQ ID NO: 299 |
| LAGLTTEPGG | SEQ ID NO: 300 |
| GGVLSGYFAG | SEQ ID NO: 301 |
| FLFGGTTFTI | SEQ ID NO: 302 |
| NNFSSTTVGI | SEQ ID NO: 303 |
| RNGQSAGTAA | SEQ ID NO: 304 |
| TLTIFRIADTVMT | SEQ ID NO: 305 |

In one embodiment, the C. *difficile* truncated spore surface protein may be derived from a collagen-like protein as listed in Table XI.

**Table XI. Clostridia difficile Spore Surface Protein Truncated Peptide Fragments**

| Peptide Fragment | Sequence Identification Number |
|---|---|
| MLLIMSRNKY | SEQ ID NO: 306 |
| FGPFDDNDYN | SEQ ID NO: 307 |
| NGYDKYDDCN | SEQ ID NO: 308 |
| NGRDDYNSCD | SEQ ID NO: 309 |
| CHHCCPPSCV | SEQ ID NO: 310 |
| GPTGPMGPRG | SEQ ID NO: 311 |
| RTGPTGPTGP | SEQ ID NO: 312 |
| TGPGVGATGP | SEQ ID NO: 313 |
| TGPTGPTGPT | SEQ ID NO: 314 |
| GNTGNTGATG | SEQ ID NO: 315 |
| LRGPTGATGA | SEQ ID NO: 316 |
| TGPTGATGAI | SEQ ID NO: 317 |
| GFGVTGPTGP | SEQ ID NO: 318 |
| TGATGATGAD | SEQ ID NO: 319 |
| GVTGPTGPTG | SEQ ID NO: 320 |
| ATGADGITGP | SEQ ID NO: 321 |
| TGATGATGFG | SEQ ID NO: 322 |
| VTGPTGPTGA | SEQ ID NO: 323 |
| TGVGVTGATG | SEQ ID NO: 324 |
| LIGPTGATGT | SEQ ID NO: 325 |
| PGATGPTGAI | SEQ ID NO: 326 |
| GATGIGITGP | SEQ ID NO: 327 |
| TGATGATGAD | SEQ ID NO: 328 |
| GATGVTGPTG | SEQ ID NO: 329 |
| PTGATGADGV | SEQ ID NO: 330 |
| TGPTGATGAT | SEQ ID NO: 331 |
| GIGITGPTGP | SEQ ID NO: 332 |
| TGATGIGITG | SEQ ID NO: 333 |
| ATGLIGPTGA | SEQ ID NO: 334 |
| TGTPGATGPT | SEQ ID NO: 335 |
| GATGPTGVGV | SEQ ID NO: 336 |
| TGATGATGAT | SEQ ID NO: 337 |
| GADGATGVTG | SEQ ID NO: 338 |
| PTGATGATGA | SEQ ID NO: 339 |
| NGLVGPTGAT | SEQ ID NO: 340 |
| GAAGTPGATG | SEQ ID NO: 341 |
| PTGATGPTGV | SEQ ID NO: 342 |
| GITGATGATG | SEQ ID NO: 343 |
| ATGPTGADGA | SEQ ID NO: 344 |
| TGPTGATGNT | SEQ ID NO: 345 |
| GADGVAGPTG | SEQ ID NO: 346 |
| ATGNTGADGA | SEQ ID NO: 347 |
| TGPTGATGAT | SEQ ID NO: 348 |
| GVTGATGPTG | SEQ ID NO: 349 |
| PTGATGATGA | SEQ ID NO: 350 |
| TGASAIIPFA | SEQ ID NO: 351 |
| SGIPLSLTTI | SEQ ID NO: 352 |
| AGGLVGTPGF | SEQ ID NO: 353 |
| VGFGSSAPGL | SEQ ID NO: 354 |
| SNGGVIDLT | SEQ ID NO: 355 |
| NAAGTLTNFA | SEQ ID NO: 356 |
| FSMPRDGTIT | SEQ ID NO: 357 |
| SISAYFSTTA | SEQ ID NO: 358 |
| ALSLVGSTIT | SEQ ID NO: 359 |
| ITATLYQSTA | SEQ ID NO: 360 |
| PNNSFTAVPG | SEQ ID NO: 361 |
| ATVTLAPPLT | SEQ ID NO: 362 |
| GILSVGSISS | SEQ ID NO: 363 |
| GIVTGLNIAA | SEQ ID NO: 364 |
| TAQTPDRQYAI | SEQ ID NO: 365 |

In one embodiment, the C. *difficile* truncated spore surface protein may be derived from a collagen-like protein as listed in Table XII.

**Table XII. Clostridia difficile Spore Surface Protein Truncated Peptide Fragments**

| Peptide Fragment | Sequence Identification Number |
|---|---|
| MSDISGPSLY | SEQ ID NO: 366 |
| QDVGPTGPTG | SEQ ID NO: 367 |
| ATGPTGPTGP | SEQ ID NO: 368 |
| RGATGATGAN | SEQ ID NO: 369 |
| GITGPTGNTG | SEQ ID NO: 370 |
| ATGANGITGP | SEQ ID NO: 371 |
| TGNMGATGPN | SEQ ID NO: 372 |
| GTTGSTGPTG | SEQ ID NO: 373 |
| NTGATGANGI | SEQ ID NO: 374 |
| TGPTGNTGAT | SEQ ID NO: 375 |
| GANGITGPTG | SEQ ID NO:-376 |
| NKGATGANGI | SEQ ID NO: 377 |
| TGSTGPTGNT | SEQ ID NO: 378 |
| GATGANGITG | SEQ ID NO: 379 |
| PTGNTGATGA | SEQ ID NO: 380 |
| TGPTGLTGAT | SEQ ID NO: 381 |
| GATGANGITG | SEQ ID NO: 382 |
| PTGNTGATGA | SEQ ID NO: 383 |
| NGVTGATGPT | SEQ ID NO: 384 |
| GNTGATGPTG | SEQ ID NO: 385 |
| SIGATGATGT | SEQ ID NO: 386 |
| TGATGPIGAT | SEQ ID NO: 387 |
| GATGADGEVG | SEQ ID NO: 388 |
| PTGAVGATGP | SEQ ID NO: 389 |
| DGLVGPTGPT | SEQ ID NO: 390 |
| GPTGATGANG | SEQ ID NO: 391 |
| LVGPTGPTGA | SEQ ID NO: 392 |
| TGANGLVGPT | SEQ ID NO: 393 |
| GATGATGVAG | SEQ ID NO: 394 |
| AIGPTGAVGA | SEQ ID NO: 395 |
| TGPTGADGAV | SEQ ID NO: 396 |
| GPTGATGATG | SEQ ID NO: 397 |
| ANGATGPTGA | SEQ ID NO: 398 |
| VGATGANGVA | SEQ ID NO: 399 |
| GPIGPTGPTG | SEQ ID NO: 400 |
| ENGVAGATGA | SEQ ID NO: 401 |
| TGATGANGAT | SEQ ID NO: 402 |
| GPTGAVGATG | SEQ ID NO: 403 |
| ANGVAGAIGP | SEQ ID NO: 404 |
| TGPTGANGAT | SEQ ID NO: 405 |
| GATGATGATG | SEQ ID NO: 406 |
| ANGATGPTGA | SEQ ID NO: 407 |
| TGATGVLAAN | SEQ ID NO: 408 |
| NAQFTVSSSS | SEQ ID NO: 409 |
| LVNNTLVTFN | SEQ ID NO: 410 |
| SSFINGTNIT | SEQ ID NO: 411 |
| FPTSSTINLA | SEQ ID NO: 412 |
| VGGIYNVSFG | SEQ ID NO: 413 |
| 1RATLSLAGF | SEQ ID NO: 414 |
| MSITTNFNGV | SEQ ID NO: 415 |
| TQNNFIAKAV | SEQ ID NO: 416 |
| NTLTSSDVSV | SEQ ID NO: 417 |
| SLSFLVDARA | SEQ ID NO: 418 |
| AAVTLSFTFG | SEQ ID NO: 419 |
| SGTTGTSAAG | SEQ ID NO: 420 |
| YVSVYRIQ | SEQ ID NO: 421 |

### C. Spore Surface Protein Mimetics

Also described are compositions and methods for making and using infection control compositions comprising microbial spore surface protein mimetics. In one embodiment, the protein mimetic comprises an amino acid sequence. In one embodiment, the amino acid sequence comprises a circular tertiary structure. In one embodiment, the protein mimetic comprises a small organic molecule.

Small organic molecules mimicking the necessary confonnation for recognition and docking to the active site binding to the peptides are contemplated. For example, mimetics of microbial spore surface proteins and/or peptide fragments are contemplated. A variety of designs for such mimetics are possible. For example, cyclic and/or linear proteins and/or peptide fragments, in which the necessary conformation for binding is stabilized by nonpeptides, are specifically contemplated. United States Patent No. 5,192,746 to Lobl, et al., United States Patent No. 5,169,862 to Burke, Jr., et al., United States Patent No. 5,539,085 to Bischoff, et al., United States Patent No. 5,576,423 to Aversa, et al., United States Patent No. 5,051,448 to Shashoua, and United States Patent No. 5,559,103 to Gaeta, et al., describe multiple methods for creating such compounds.

Synthesis of non-peptide compounds that mimic peptide sequences is also known in the art. For example, some non-peptide antagonists mimic the Arg-Gly-Asp sequence. Eldred, et al., (J. Med. Chem. 37:3882 (1994)). Likewise, Ku, et al., (J. Med. Chem. 38:9 (1995)) give further elucidation of the synthesis of a series of such compounds. Such non-peptide compounds that mimic microbial spore surface proteins and/or peptide fragments are specifically contemplated.

Also described are synthetic mimicking compounds that are multimeric compounds that repeat relevant peptide sequences. As is known in the art, peptides can be synthesized by linking an amino group to a carboxyl group that has been activated by reaction with a coupling agent, such as dicyclohexylcarbodiimide (DCC). The attack of a free amino group on the activated carboxyl leads to the formation of a peptide bond and the release of dicyclohexylurea. It can be necessary to protect potentially reactive groups other than the amino and carboxyl groups intended to react. For example, the α-amino group of the component containing the activated carboxyl group can be blocked with a tertbutyloxycarbonyl group. This protecting group can be subsequently removed by exposing the peptide to dilute acid, which leaves peptide bonds intact.

With this method, peptides can be readily synthesized by a solid phase method by adding amino acids stepwise to a growing peptide chain that is linked to an insoluble matrix, such as polystyrene beads. The carboxyl-terminal amino acid (with an amino protecting group) of the desired peptide sequence is first anchored to the polystyrene beads. The protecting group of the amino acid is then removed. The next amino acid (with the protecting group) is added with the coupling agent. This is followed by a washing cycle. The cycle is repeated as necessary.

In one embodiment, the mimetics are peptides having sequence homology to the above-described microbial spore surface proteins and/or peptide fragments. One common methodology for evaluating sequence homology, and more importantly statistically significant similarities, is to use a Monte Carlo analysis using an algorithm written by Lipman and Pearson to obtain a Z value. According to this analysis, a Z value greater than 6 indicates probable significance, and a Z value greater than 10 is considered to be statistically significant. W.R. Pearson and DJ. Lipman, Proc. Nat. Acad. Sci. (USA), 85:2444-2448 (1988); D.J. Lipman and W.R. Pearson, Science, 227:1435-1441 (1985). In the disclosure, synthetic polypeptides useful in solving the problems of microbial decontamination and microbial infection are those peptides with statistically significant sequence homology and similarity (Z value of Lipman and Pearson algorithm in Monte Carlo analysis exceeding 6).

### IV. Screening For Infection Control Compositions

Embodiments comprise methods comprising screening for infection control compositions that inhibit, antagonize, interfere, displace, and/or disrupt the binding of a microbial spore to an inanimate and/or animate surface. In one embodiment, the present invention contemplates methods comprising screening for infection control compositions that inhibit, antagonize, interfere, displace, and/or disrupt the binding of a microbial spore surface protein to an inanimate and/or animate surface. In one embodiment, the animate surface may be selected from the group comprising skin, human body tissues, and/or human cells. In one embodiment, the inanimate surface may comprise hard surfaces including, but not limited to, ceramic, tile, granite, and/or stainless steel. In one embodiment, the microbial spore comprises a *Clostridium* spore. In one embodiment, the *Clostridium* spore comprises a C. *difficile* spore. In one embodiment, the screened infection control compositions are capable of binding to a microbial spore surface protein. Although it is not necessary to understand the mechanism of an invention, it is believed that the infection control compositions bind to a surface thereby blocking binding of a microbial spore surface protein. Although it is not necessary to understand the mechanism of an invention, it is believed that such infection control compositions may include, but are not limited to, natural compounds isolated from viral, bacterial, fungal, plant, and animal sources, as well as synthetic compounds. It is further believed that neither the source, chemical structure, or mechanism of action of the infection control composition is essential to this invention.

### A. Assays For Detecting Microbial Spore Binding To A Surface

### 1. Standard Growth Assays

In one embodiment, methods are described for detecting microbial spores and/or microbial spore proteins comprising: a) providing; i) a substrate surface suspected of contamination by at least one microbial spore; ii) a spore collection device comprising a plurality of infection control compositions capable of attaching the at least one microbial spore; and iii) a spore growth plate capable of supporting growth of the at least one microbial spore; b) contacting the spore collection device with the substrate surface wherein the at least one microbial spore becomes attached to the device; c) contacting the microbial collection device with the spore growth plate; and d) detecting at least one microbial spore on the spore growth plate. In one embodiment, the infection control composition comprises a microbial spore protein peptide fragment. In one embodiment, the microbial spore protein peptide fragment is selected from the group comprising a *Clostridium* spore protein peptide fragment. In one embodiment, the microbial spore is a *Clostridium difficile* spore.

Although it is not necessary to understand the mechanism of an invention, it is believed that a growth assay can be performed using any microbial spore. By way of illustration, C. *sporogenes* spores are used below in a method to detect the growth of spores after their collection from a contaminated surface, but is should be understood that any microbial spore can be substituted wherein collection and detection each respective microbial spore will be successfully performed (i.e., for example, *Clostridium difficile* antibodies).

### a. Cup Scrub Assays

One described technique is suitable for the recovery of resident and transient microorganisms from human or animal skin. For example, the technique may be used in situ within clinical protocols or in vitro for studies using isolated skin or skin equivalents. ASTM International, "Standard Test Method for Recovery of Microorganisms From Skin using the Cup Scrub Technique" E1874 - 09 (2009). Briefly, resident microorganisms or transient microorganisms (i.e., for example, microorganisms previously applied to a test site), are recovered from the site by pressing a rigid cylinder against the skin with sufficient pressure to form a seal and instilling recovery liquid into the cylinder. The surface of the skin is then mechanically 'scrubbed' with a glass rod, rubber policeman, or some other suitable device for a prescribed period of time. The fluid is pipetted from the cylinder into a test tube, or other suitable receptacle, for further analysis.

A cub-scrub procedure can be incorporated into any method describes herein to evaluate test materials containing infection control compositions that are intended to reduce significantly the number of microbial spores on a test surface (i.e., for example, intact skin). It also may be used to provide an indication of residual antimicrobial activity.

### b. Cell Culture Assays

Aliquots of C *difficile* endospore suspension are anaerobically incubated at 35°C on prereduced (i.e., for example, oxygen removed) BCDC, TCCFA, and TSA. Control and experimental plates are inoculated identically with a spore suspension, and one set of control plates are allowed to incubate and grow undisturbed for the duration of each experiment.

Attachment experiments are done by placing a circle (i.e., for example, a target area) on the bottom of the agar-filled Petri dish, and inoculating the plate with 5 or 10 µl of C. *difficile* spore suspensions directly within a target area, incubating these spores in the anaerobic hood (Forma Scientific, Marietta, Ohio) on pre-reduced plates for 27 min, 99 min, 150 min, and 360 min. After timed incubation, the plates are removed, and 4 ml of sterile distilled water added to each culture plate. The plates are then shaken at 300 rpm for 10 min, after which the agitation solution is aseptically pipetted off the agar surface, and the plates re-incubated anaerobically for 24, 48, and 72 h. To determine if the plates had become contaminated, light microscopy smears on glass slides are made from randomized colonies and photographed on a photomicroscope, and plates are inoculated and incubated under aerobic conditions to identify any aerobic contaminants.

To determine if spores are not yet attached to the agar, and establish if they could be easily detached and washed off the agar surface, the water agitation solution (removed after shaking the plate) is also tested for the presence of spores. Aliquots of the water is:(i) screened by phase microscopy; and (ii) used to streak agar plates; and (iii) injected into BacTec NR6A broth (anaerobic blood culture broth, BD Microbiology Systems Cockeyesville, MD) and the plates and broth read at 24 and 48 h for the presence of bacterial growth.

### 2. Anti-Spore Surface Antibody Assays

In one embodiment, there is described a method for detecting microbial spores comprising: a) providing; i) a substrate surface suspected of contamination by at least one microbial spore; and ii) an anti-spore surface antibody capable of binding to the at least one microbial spore; b) contacting the anti-spore surface antibody with the substrate surface or spore surface under conditions such that the antibody binds to the at least one microbial spore, wherein an antibody/spore complex is created; and c) detecting the antibody/spore complex. In one embodiment, the antibody comprises a polyclonal antibody (i.e.., for example, F1997) In one embodiment, the antispore surface antibody comprises a label. In one embodiment, the label comprises a fluorescent label. In one embodiment, the microbial spore is a *Clostridium difficile* spore.

There are described methods for detecting microbial spores comprising: a) providing; i) a substrate surface suspected of contamination by at least one microbial spore; ii) an anti-spore surface antibody capable ofbinding to the at least one microbial spore; and iii) a growth assay plate; b) combining the anti-spore surface antibody with the antibody, wherein an antibody/spore complex is created; c) contacting the antibody/spore complex with the substrate surface; d) removing a sample of the antibody/spore complex (i;e., for example, by the cup-scrub method) from the surface; e) streaking the sample on the growth assay plate. In one embodiment, the antibody comprises a polyclonal antibody (i.e.., for example, FI997). In one embodiment, the substrate surface comprises a pigskin surface. In one embodiment, the label comprises a fluorescent label. In one embodiment, the microbial spore is a *Clostridium difficile* spore.

### a. Antibody Development

Although it is not necessary to understand the mechanism of an invention, it is believed that an anti-spore surface antibody can be directed towards any epitope on a spore surface (i.e., whether a protein or other antigenic structure). By way of illustration, C. *sporogenes* spores are used below in a method to generate anti-spore surface monoclonal and polyclonal antibodies, but is should be understood that any microbial spore can be substituted wherein antibodies against each respective microbial spore will be successfully generated (i.e., for example, *Clostridium difficile* antibodies).

Mouse polyclonal antiserum and monoclonal antibodies against C. *sporogenes* spores were obtained by several injections of 108 inactivated spores (incubated in 4% (v/v) formaldehyde for 4 h at 37°C). Monoclonal antibodies were produced according to the procedures of Kohler and Milstein (Kohler and Milstein, 1975). Briefly, the mice were boosted on days 21 and 42 and bled on day 50 for polyclonal antibodies, or boosted on day 50 and sacrificed for fusion on day 53 for monoclonal antibodies. Spore stocks are prepared by growing C. *sporogenes* cells in cooked meat medium at 35°C, and *Clostridium perjringens* cells in fluid thioglycolate medium at 37°C under anaerobic conditions.

Antigen prepared by the above formalin inactivation of C. *sporogenes* PA3679 spore stocks were free of vegetative cell material as observed by phase-contrast microscopy. Phillips et aI., "Assessment of immunofluorescence measurements of individual bacteria in direct and indirect assays for Bacillus anthracis and Bacillus cereus spores" J Appl. Bacteriol. 53 :223-231 (1982). A total of 1 x 107 C. *sporogenes* spores are used as the antigen.

Monoclonal antibody production is done according one of many procedures. Kohler et al., "Continuous cultures of fused cells secreting antibody of predefined specificity" Nature 296:495--497 (1975)(herein incorporated by reference in its entirety). Hybridomas are screened for reactivity with C. *sporogenes* spores. Hybridomas found to be reactive are also screened for reactivity with the vegetative cells.

Isotyping of monoclonal antibodies are performed by using a dot blot immunoassay format. Spores are applied to an Immobilon-P® membrane (Millipore Corp., Bedford, Mass.) in a dot blot apparatus. The spores are then incubated with 100 ml of the appropriate tissue culture supernatants. Monoclonal antibody bound to spores are isotyped by using rabbit anti-mouse subclass specific antiserum obtained from BioRad ® (Hercules, Calif.).

*Clostridium sporogenes* spores were then applied to an Immobilon-P® membrane, followed by incubation with above generated antibodies. Similarly, 10⁶ of the *Clostridium* vegetative cells are applied to the membrane, followed by incubation with the indicated antibodies. Reactivity is detected with a goat anti-mouse immunoglobulin G (IgG)-horseradish peroxidase conjugate (Sigma A4416) or a goat anti-mouse IgMhorseradish peroxidase conjugate (Sigma A8786). Color development maybe performed with the insoluble substrate3-amino-9-ethyl-carbazole (Biomeda Corp.).

Culture supernatants are then diluted 1:32 and 1:128 with Tris-buffered saline (TBS) (0.01 M Tris, 0.15 M NaCl, pH 7.4). Aliquots of diluted culture supernatants are mixed with an equal volume of the spore antigen (4 x 109 spores/ml) by gentle inversion overnight at 48°C. Diluted culture supernatants mixed with sterile distilled water serve as a positive control. Antibodies adsorbed to spores are removed by centrifugation (ca. 4,000 3g, 10 min). The supernatant fluid is used in immunoblot assays as detailed above.

Antibodies are concentrated by ammonium sulfate precipitation oftissue culture supernatants. Halow et al., "Storing and purifying antibodies" In: Antibodies: A Laboratory Manual. p. 283-318. E. Halow and D. Lane (ed.), Cold Spring Harbor Laboratory, Cold Spring Harbor, N.Y. (1988). The concentrated protein is biotinylated by incubation with N-hydroxysuccinimidobiotin (Sigma Chemical Co.) (ca. 100 mg/mg of antibody in the sample) at room temperature for 4 h. One molar ammonium chloride (20 ml/250 mg of biotin) is added to stop the reaction, and the biotinylated protein is dialyzed extensively against phosphate-buffered saline (0.1 M sodium phosphate, 0.8% NaCl) (PBS), pH 7.0. For immunocytochemical localization studies.

IgG antibodies are purified out of tissue culture supernatants by using a protein A column (Pierce Chemical Co., Rockford, Ill.) according to the manufacturer's directions. IgM antibodies are purified from the culture supernatant by using a goat anti-mouse IgM antibody column (Pierce Chemical Co.). The tissue culture supernatant is diluted 1: 1 in PBS and applied to the goat anti-mouse IgM column, which is equilibrated with PBS. Washing and elution of antibody off the column is performed according to the manufacturer's directions. Fractions containing protein are pooled and concentrated with a Centricon 10 microconcentrator (Amicon, Danvers, Mass.) and centrifugation at 3,000 g for 30 min.

### 3. Anti-Spore Surface Protein Antibody Assays

There are described methods for detecting microbial spores comprising: a) providing; i) a substrate surface suspected of contamination by at least one microbial spore, said spore comprising at least one surface protein; and ii) a anti-surface protein antibody capable of binding to the at least one surface protein; b) contacting the anti- surface protein antibody with the substrate surface under conditions such that the antibody binds to the at least one surface protein, wherein an antibody/protein complex is created; and c) detecting the antibody/protein complex. In one embodiment, the spore surface protein includes, but is not limited to, CD 1067, CD 1581, CD 3520, CD 0372, or CD 1613. In one embodiment, the anti- surface protein antibody comprises a label. In one embodiment, the label comprises a fluorescent label. In one embodiment, the surface protein is a *Clostridium difficile* surface protein. In one embodiment, the surface protein is a *Bacillus anthracis* surface protein.

Although it is not necessary to understand the mechanism of an invention, it is believed that a microbial surface protein antibody can be directed towards a specific epitope on a spore surface protein (i.e., whether an amino acid sequence or other antigenic structure). By way of illustration, a specific C. *difficile* spore surface protein is used below in a method to generate polyclonal and monoclonal antibodies, but is should be understood that any microbial spore surface protein can be substituted wherein polyclonal and/or monoclonal antibodies directed against each respective microbial spore surface protein will be successfully generated.

Mouse polyclonal antiserum and monoclonal antibodies against C. *difficile* hypothetical protein (SEQ ID NO: 1) are obtained by several injections of isolated hypothetical protein (i.e., for example, approximately 108 proteins per injection). Monoclonal antibodies are produced according to the procedures of Kohler and Milstein (Kohler and Milstein, 1975). Briefly, the mice can be boosted on days 21 and 42 and bled on day 50 for polyclonal antibodies, or boosted on day 50 and sacrificed for fusion on day 53 for monoclonal antibodies. Hybridomas may be screened for reactivity with C. *difficile* spores. Hybridomas found to be reactive are also screened for reactivity with the vegetative cells.

Isotyping of monoclonal antibodies are performed by using a dot blot immunoassay format. Spores are applied to an Immobilon-P® membrane (Millipore Corp., Bedford, Mass.) in a dot blot apparatus. The spores are then incubated with 100 ml of the appropriate tissue culture supernatants. Monoclonal antibody bound to spores are isotyped by using rabbit anti-mouse subclass specific antiserum obtained from BioRad ® (Hercules, Calif.).

There are described methods for detecting microbial spores comprising: a) providing; i) a substrate surface suspected of contamination by at least one microbial spore, said spore comprising at least one surface protein; and ii) a anti-surface protein monoclonal antibody capable of binding to the at least one surface protein; b) contacting the anti-surface protein monoclonal antibody with the substrate surface under conditions such that the monoclonal antibody binds to the at least one surface protein, wherein a monoclonal antibody/protein complex is created; and c) detecting the monoclonal antibody/protein complex. In one embodiment, the spore surface protein includes, but is not limited to, CD 1067, CD 1581, CD 3520, CD 0372, or CD 1613. In one embodiment, the anti-surface protein monoclonal antibody comprises a label. In one embodiment, the label comprises a fluorescent label. In one embodiment, the surface protein is a *Clostridium difficile* surface protein. In one embodiment, the surface protein is a *Bacillus anthracis* surface protein.

### B. Assays For Detecting Microbial Spore Surface Protein Binding To A Surface

Several methods are provided herein for detecting spore surface protein binding in order to screen potential infection control compositions.

In order to provide methods and devices for screening compounds for developing infection control compositions, the described method generally incorporates model *in vitro* systems which mimic a given microbial system *in vitro* for which infection control compositions are desired. The range of systems against which these infection control compositions can be screened is extensive. For example, potential infection control compositions are optionally screened for effects in blocking, slowing or otherwise inhibiting key events associated with infectious microbial organisms. For example, potential infection control test compositions are optionally screened for their ability to block systems that are responsible, at least in part, for sporulation or for the binding of spores to a surface, including, e.g., skin, bodily tissues, granite, stainless steel, ceramic etc.. Infection control test compositions which show promising results in these screening assay methods can then be subjected to further testing to identify effective pharmacological agents for the treatment of disease or symptoms of microbial infections

### 1. Binding Screens

In their simplest forms, the infection control composition screening models employed in the methods and apparatuses described will screen for an interaction of a infection control composition with a component of a biochemical system, e.g., receptor-ligand interaction, enzyme-substrate interaction, and the like. In this form, the screening model will typically include two interacting components of the system (i.e., for example, a bacterial spore protein and an infection control composition or a microbial enzyme and an infection control composition).

In one embodiment, the method comprises determining whether an infection control test composition has an effect on microbial spore protein binding interactions with a surface by contacting the microbial spore protein with the infection control test composition and assaying for residual binding of the microbial spore to the surface. The residual surface binding amount is then compared to a control surface binding, e.g., the same binding assay performed in the absence of the infection control composition. Typically, such binding assays involve the measurement of a parameter of the screening system. By "parameter of the screening system" is meant some measurable evidence, e.g., the presence or absence of a labeled group or a change in molecular weight (e.g., in binding reactions, transport screens), the presence or absence of a reaction product or substrate (in substrate turnover measurements), or an alteration in electrophoretic mobility (typically detected by a change in elution time of a labeled compound).

Although described above in terms of a two-component screening systems, the methods and apparatuses may also be used to screen for effectors ofinuch more complex systems, where the result or end product ofthe system is known and assayable at some level, e.g., enzymatic pathways, spore surface signaling pathways and the like. Alternatively, the methods and apparatuses described herein are optionally used to screen for compounds that interact with a single component of a biochemical system, e.g., compounds that specifically bind to a particular biochemical compound, e.g., a receptor, ligand, enzyme, nucleic acid, structural macromolecule, etc.

In some embodiments, assays may involve the interaction of two or more components. Assays involving interactions between two components can be viewed as assays for enhancers or inhibitors of binding between members of "binding pairs". Prefened binding pairs include, but are not limited to, microbial spore proteins/small organic molecules, microbial spore proteins/nucleic acids, microbial spore proteins/infection control compositions (i.e., for example, *Clostridium* spore surface protein fragments). In one embodiment, either member ofthe binding pair can be immobilized (i.e., for example, attached to the surface) while the other member is in a solution contacted to the surface. In another embodiment, both members can be attached to different surfaces which are then juxtaposed to perform the assay.

Accordingly, the methods described will be useful in screening for infection control compositions that form a binding pair with a receptor molecule, wherein the binding pair affects an interaction between the receptor molecule and a surface. As used herein, the term "receptor" generally refers to one member of the binding pair (i.e., for example, a microbial spore surface protein). The other member of the binding pair is termed "an infection control composition. Thus, a receptor/ligand pair may include a typical protein receptor, usually membrane associated, and its natural ligand, e.g., another protein or small molecule. Receptor/ligand pairs may also include antibody/antigen binding pairs, complementary nucleic acids, nucleic acid associating proteins and their nucleic acid ligands.

Traditionally, methods for screening for effectors of a receptor/ligand interaction (i.e., for example, a microbial spore surface protein/infection control composition interaction) have involved incubating a receptor/ligand binding pair in the presence of a test compound. The level ofbinding of the receptor/ligand pair is then compared to negative and/or positive controls. Where a decrease in normal binding is seen, the test compound is determined to be an inhibitor of the receptor/ligand binding. Where an increase in that binding is seen, the test compound is determined to be an enhancer or inducer of the interaction. Hhowever, it is contemplated that the receptor/ligand interaction is assayed by measuring the amount of residual binding of the receptor to a surface in the presence ofthe infection control composition.

In one embodiment, high throughput screening assays may comprise multiwell reaction plates, e.g., multi-well microplates, which allow for the simultaneous, parallel screening of large numbers of potential infection control compositions.

A similar, and perhaps overlapping, set of biochemical systems may include the interactions between microbial enzymes and their substrates. The term "enzyme" as used herein, generally refers to a protein which acts as a catalyst to induce a chemical change in other compounds or "substrates." Typically, effectors of an enzyme's activity toward its substrate are screened by contacting the enzyme with a substrate in the presence and absence of the compound to be screened and under conditions optimal for detecting changes in the enzyme's activity. After a set time for reaction, the mixture is assayed for the presence of reaction products or a decrease in the amount of substrate. The amount of substrate that has been catalyzed is them compared to a control, i.e., enzyme contacted with substrate in the absence of test compound or presence of a known effector. As above, a compound that reduces the enzymes activity toward its substrate is termed an "inhibitor," whereas a compound that accentuates that activity is termed an "inducer."

Depending upon the particular embodiment being practiced, the infection control test compositions are provided in various different media including, but not limited to, by injection, free in solution, attached to a carrier, attached to a solid support (i.e., for example, beads). A number of suitable solid supports are employed for immobilization of the test compounds. Examples of suitable solid supports include agarose, cellulose, dextran (commercially available as, i.e., Sephadex, Sepharose) carboxymethyl cellulose, polystyrene, polyethylene glycol (PEG), filter paper, nitrocellulose, ion exchange resins, plastic films, glass beads, polyaminemethylvinylether maleic acid copolymer, amino acid copolymer, ethylene-maleic acid copolymer, nylon, silk, etc. Additionally, for the methods and apparatuses described herein, test compounds are screened individually, or in groups. Group screening is particularly useful where hit rates for effective test compounds are expected to be low such that one would not expect more than one positive result for a given group. Alternatively, such group screening is used where the effects of different test compounds are differentially detected in a single system.

### 2. Immunoassays

In one embodiment, there are disclosed immunological tests where a reaction takes place between two or more related immunological agents (i.e., for example, an antigen and antibody). Thus, for example, when it is sought to determine whether and how much of a particular antigen or antibody resides in a sample one must attempt to react the sample suspected of containing an antigen with its immunological binding pair. Although it is not necessary to understand the mechanism of an invention, it is believed that if a reaction takes place, then the visualization of that reaction is evidence of the presence of the antigen or antibody in the originally tested fluid.

In one embodiment, an antibody/antigen binding pair may be visualized using an indicator or carrier particle. One suitable system comprises a polymeric particle technique in which synthetic resin particles, surfaces, or matrices have been used as an adsorbent onto which the appropriate antigen (i.e., for example, a microbial spore protein fragment) or antibody (i.e., for example, having affinity to a microbial spore protein fragment) has been adsorbed.

Formats for immunoassays include, but are not limited to, competitive (e.g., ELISA, hapten inhibition, etc.) and noncompetitive assays. Various immunoassay formats have been reported. See, U.S. Pat. Nos. 4,366,241; 4,376,110; 4,517,288; and 4,837,168; Asai (1993) Methods in Cell Biology Volume 37: Antibodies in Cell Biology, Academic Press, Inc. New York, Stites & Terr, eds. (1991) Basic and Clinical Immunology 7th Edition, etc.) and the references cited therein).

### 3. Nucleic Acid Binding Assays

In another embodiment, the materials and methods of this invention are used to iinmobilize components in nucleic acid binding protein assays. Typically such assays detect binding of a nucleic acid by one or more binding proteins. The assays are generally used to screen for agents that improve (specificity or affinity) or inhibit nucleic acid binding by one or more particular nucleic acid binding proteins. Nucleic acid binding proteins include, but are not limited to endonucleases, polymerases, nuclear transcription factor receptors, API proteins (i.e., for *example,fos andjun)*. Nucleic acid binding assays are facilitated by attachment to a surface of either the nucleic acid containing the protein binding site or the binding protein itself.

### 4. Binding Assays in Physiologically Compatible Solutions

It will be appreciated that may of the foregoing assays require particular conditions for the subject assay components (e.g., enzymes) to maintain their desired activity. Typically such assays are optimized to maintain physiologically compatible conditions for elements (e.g., pH, ion composition) critical to component activity.

It is a significant advantage that the infection control compositions of this invention are stable to physiological concentrations of most ionic species (e.g., Na+, Ca2+, Mg2+, etc.) Thus, the assays can be run in standard physiologically compatible compositions (e.g., phosphate buffered saline (PBS) standard ringers solutions, and the like.).

The infection control compositions of this invention can also be used to purify the moiety (e.g., polypeptide(s)) to which they for binding pairs. Specifically, the infection control compositions can be used in conjunction with virtually any anion or cation exchange resin. Suitable anion and cation exchange resins are commercially available. Cation exchange resins, for example include, but are not limited to, carboxymethylcellulose, while anion exchange resins include, but are not limited to, DEAE cellulose, DEAE SEPHAROSE gel filtration ion exchange media, heparin, and the like.

The interaction of an infection control composition with a microbial spore surface protein can be exploited for purification purposes. This accomplished in a manner analogous to a cation exchange resin or in a manner analogous to the polyhistidine/nickel separation systems substituting mica for the cation exchange resin or the nickel resin, respectively. In one embodiment, the mica is provided as a bed (either of mica flakes or powder) through which the sample is tlowed. The mica bed is housed in any of a variety of suitable vessels, including cartridges that are attachable to syringes, chromatography columns, and the like. The microbial spore surface protein binds to the mica bed, while other components ofthe sample are washed away. Non- specifically binding components can be eluted away using salt solutions at concentrations sufficient to release undesired components while retaining the microbial spore surface protein. Once the infection control composition is bound to the microbial spore surface protein, the infection control composition can then be released by application of sufficient salt concentrations and/or other appropriate solvent mixtures as desired.

### 5. Continuous Flow Assay Systems

In one embodiment, the methods and apparatuses of the invention are used in screening infection control test compositions using a continuous flow assay system. The use of such continuous flow assay systems are not limited to screening for spore surface protein binding to surfaces but can also be readily used in screening for inhibitors or inducers of enzymatic activity, or for agonists or antagonists of receptor-ligand binding.

In brief, the continuous flow assay system involves the continuous flow of the particular biochemical system along a fluid pathway (i.e., for example, a microarray, a chromatography column, or a micro fabricated channel). As used herein, the term "continuous" generally refers to an unbroken or contiguous stream of the particular composition that is being continuously flowed. For example, a continuous flow may include a constant fluid flow having a set velocity, or alternatively, a fluid flow which includes pauses in the flow rate of the overall system, such that the pause does not otherwise interrupt the flow stream.

### 6. Detectable Labels

In one embodiment, a functioning screening system comprises producing a detectable event or signal. Such detectable signals may include, but are not limited to, optically detectable chromophoric or fluorescent signals. For enzyme systems, such signals can be produced by products of the enzyme's catalytic action, e.g., on a chromogenic or fluorogenic substrate. For binding systems, e.g., receptor ligand interactions or microbial spore surface binding to surfaces, signals will typically involve the association of a labeled ligand with the receptor, or vice versa.

A wide variety of other detectable signals and labels can also be used in the embodiments of the presently contemplated invention. In addition to the chromogenic and fluorogenic labels described above, radioactive decay, electron density, changes in pH, solvent viscosity, temperature and salt concentration are also conveniently measured.

More generally, labels are commonly detectable by spectroscopic, photochemical, biochemical, immunochemical, or chemical means. For example, useful nucleic acid labels include 32p , 35S, fluorescent dyes, electron-dense reagents, enzymes (e.g., as commonly used in an ELISA), biotin, dioxigenin, or haptens and proteins for which antisera or monoclonal antibodies are available. A wide variety oflabels suitable for labeling biological components are known and are reported extensively in both the scientific and patent literature, and are generally applicable for the labeling of biological components. Suitable labels include, but are not limited to, radionucleotides, enzymes, substrates, cofactors, inhibitors, fluorescent moieties, chemiluminescent moieties, magnetic particles, and the like. Labeling agents optionally include e.g., monoclonal antibodies, polyclonal antibodies, proteins, or other polymers such as affinity matrices, carbohydrates or lipids.

Detection of labels may proceed by a variety of methods, including but not limited to, spectrophotometric or optical tracking of radioactive or fluorescent markers, or other methods which track a molecule based upon size, charge or affinity. A detectable moiety can be of any material having a detectable physical or chemical property. Such detectable labels exist in the fields of gel electrophoresis, column chromatography, solid substrates, spectroscopic techniques, and the like, and in general, labels useful in such methods can be applied.

Thus, a label may comprise any composition detectable by spectroscopic, photochemical, biochemical, immunochemical, electrical, optical thermal, or chemical means. In some embodiments, labels may include, but are not limited to, fluorescent dyes (e.g., fluorescein isothiocyanate, Texas red, rhodamine, and the like), radiolabels (e.g., ³H, ¹²⁵I, ³⁵S, ¹⁴C, ³²P or ³³P), enzymes (e.g., LacZ, CAT, horse radish peroxidase, alkaline phosphatase and others, commonly used as detectable enzymes, either as marker products or as in an ELISA), nucleic acid intercalators (e.g., ethidium bromide) and colorimetric labels such as colloidal gold or colored glass or plastic (e.g. polystyrene, polypropylene, latex, etc.) beads.

Also described are fluorescent labels that are typically characterized by one or more of the following: high sensitivity, high stability, low background, low environmental sensitivity and high specificity in labeling. Such fluorescent moieties, which are incorporated into the labels of the invention may include, but are not limited to, 1- and 2-aminonaphthalene, p,p'-diaminostilbenes, pyrenes, quaternary phenanthridine salts, 9-aminoacridines, p,p'-diaminobenzophenone imines, anthracenes, oxacarbocyanine, merocyanine, 3-aminoequilenin, perylene, bisbenzoxazole, bis-p-oxazolyl benzene, 1,2-benzophenazin, retinol, bis-3-aminopyridinium salts, hellebrigenin, tetracycline, sterophenol, benzimidazolylphenylamine, 2-oxo-3- chromen, indole, xanthen, 7-hydroxycoumarin, phenoxazine, calicylate, strophanthidin, porphyrins, triarylmethanes and flavin. Individual fluorescent compounds which have functionalities for linking to an element detectable by embodiments of the invention, or which can be modified to incorporate such functionalities, include but are not limited to, dansyl chloride; fluoresceins such as 3,6-dihydroxy-9-phenylxanthhydrol; rhodamineisothiocyanate; N-phenyll -amino-8-sul fonatonaphthalene; N-phenyI2-amino6-sulfonatonaphthalene; 4-acetamido-4-isothiocyanato-stilbene-2,2'-disulfonic acid; pyrene-3-sulfonic acid; 2-toluidinonaphthalene-6-sulfonate; N-phenyl-N-methyl-2aminoaphthalene- 6-sulfonate; ethidium bromide; stebrine; auromine-0,2-(9'anthroyl) palmitate; dansyl phosphatidylethanolamine; N,N'-dioctadecyl oxacarbocyanine: N,N'-dihexyl oxacarbocyanine; merocyanine, 4-(3'pyrenyl)stearate; d-3-aminodesoxyequilenin; 12-(9'-anthroyl)stearate; 2-methylanthracene; 9-vinylanthracene; 2,2'(vinylenep- phenylene)bisbenzoxazole; p-bis(2-(4-methyl-5-phenyl-oxazolyl»benzene; 6dimethylamino- 1,2-benzophenazin; retinol; bis(3'-aminopyridinium) 1,1 O-decandiyl diiodide; sulfonaphthylhydrazone ofhellibrienin; chlorotetracycline; N-(7- dimethylamino-4-methyl-2-oxo-3-chromenyl)maleimide; N-(p-(2-benzimidazolyl)phenyl) maleimide; N-(4-fluoranthyl)maleimide; bis(homovanillic acid); resazarin; 4chloro- 7-nitro-2,1 ,3-benzooxadiazole; merocyanine 540; resorufin; rose bengal; and 2,4diphenyl- 3(2H)-furanone.

Many fluorescent tags are commercially available, for example, from SIGMA chemical company (Saint Louis, Mo.), Molecular Probes, R&D systems (Minneapolis, Minn.), Pharmacia LKB Biotechnology (Piscataway, N.J.), CLONTECH Laboratories, Inc. (Palo Alto, Calif.), Chern Genes Corp., Aldrich Chemical Company (Milwaukee, Wis.), Glen Research, Inc., GIBCO BRL Life Technologies, Inc. (Gaithersberg, Md.), Fluka Chemica-Biochemika Analytika (Fluka Chemie AG, Buchs, Switzerland), and Applied Biosystems (Foster City, Calif.).

Desirably, fluorescent labels absorb light above about 300 nm, preferably about 350 nm, and more preferably above about 400 nm, usually emitting at wavelengths greater than about 10 nm higher than the wavelength of the light absorbed. It should be noted that the absorption and emission characteristics of the bound label may differ from the unbound label. Therefore, when referring to the various wavelength ranges and characteristics of the labels, it is intended to indicate the labels as employed and not the label which is unconjugated and characterized in an arbitrary solvent. Fluorescent labels are detectable because by irradiating a fluorescent label with light, one can obtain a plurality of emissions. Thus, a single label can provide for a plurality of measurable events. Detectable signal may also be provided by chemiluminescent and bioluminescent sources.

Chemiluminescent sources include a compound which becomes electronically excited by a chemical reaction and may then emit light which serves as the detectible signal or donates energy to a fluorescent acceptor. A diverse number of families of compounds have been found to provide chemiluminescence under a variety or conditions. One of compounds is 2,3-dihydro-1,4-phthalazinedione. The most popular compound is luminol, which is a 5-amino compound. Other members of the include the 5-amino- 6,7,8-trimethoxy- and the dimethylamino[ca]benz analog. These compounds can be made to luminesce with alkaline hydrogen peroxide or calcium hypochlorite and base. Another of compounds is the 2,4,5-triphenylimidazoles, with lophine as the common name for the parent product. Chemiluminescent analogs include para-dimethylamino and -methoxy substituents. Chemiluminescence may also be obtained with oxalates, usually oxalyl active esters, e.g., p-nitrophenyl and a peroxide, e.g., hydrogen peroxide, under basic conditions. Other useful chemiluminescent compounds are also known and available, including -N-alkyl acridinum esters (basic H20 2) and dioxetanes. Alternatively, luciferins maybe used in conjunction with luciferase or lucigenins to provide bioluminescence.

A label may be coupled directly or indirectly to a molecule to be detected (a product, substrate, enzyme, or the like) according to many methods. As indicated above, a wide variety oflabels are used, with the choice oflabel depending on the sensitivity required, ease of conjugation of the compound, stability requirements, available instrumentation, and disposal provisions. Non-radioactive labels are often attached by indirect means. Generally, a ligand molecule (e.g., biotin) is covalently bound to a polymer. The ligand then binds to an anti-ligand (e.g., streptavidin) molecule which is either inherently detectable or covalently bound to a signal system, such as a detectable enzyme, a fluorescent compound, or a chemiluminescent compound. A number of Ligands and anti-ligands can be used. Where a ligand has a natural anti-ligand, for example, biotin, thyroxine, and cortisol, it can be used in conjunction with labeled, antiligands. Alternatively, any haptenic or antigenic compound can be used in combination with an antibody. Labels can also be conjugated directly to signal generating compounds, e.g., by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescent compounds include luciferin, and 2,3-dihydrophthalazinediones, e.g., luminol.

Means of detecting labels are dependent upon the type of label. Thus, for example, where the label is a radioactive label, a means for detection may include, but is not limited to, a scintillation counter or photographic film as in autoradiography. Where the label is a fluorescent label, a means for detection may include, but is not limited to, exciting the fluorochrome with the appropriate wavelength of light and detecting the resulting fluorescence, e.g., by microscopy, visual inspection, via photographic film, by the use of electronic detectors such as digital cameras, charge coupled devices (CCDs) or photomultipliers and phototubes, and the like. When using fluorescent labels and detection techniques, microscopy and spectroscopy techniques are often integrated into the detection system. Similarly, enzymatic labels may be detected by providing appropriate substrates for the enzyme and detecting the resulting reaction product. Finally, simple colorimetric labels are often detected simply by observing the color associated with the label. For example, conjugated gold often appears pink, while various conjugated beads appear the color of the bead.

In one embodiment, a continuous flow screening system generates a constant signal which varies only when a test compound is introduced that affects the system. Specifically, as the system components flow through the system, they will produce a relatively constant signal level at a detection zone. When test compounds (i.e., for example, infection control compositions) are periodically introduced into the channel and mixed with the system components, the test compounds cause a deviation from the constant signal level at the detection zone. This deviation may then be correlated to the particular test compound screened.

### D. Detection Of Microbial Spore Surface Protein Binding To An Epidennal Protein

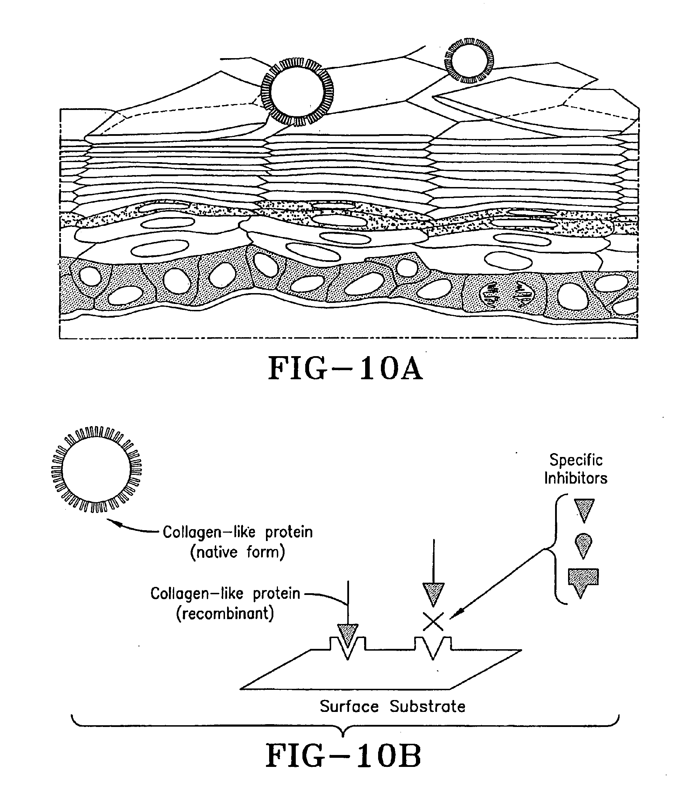

There is described a method for detecting microbial spore surface protein binding to an epidermal layer. In one embodiment, the epidermal layer is derived from skin. In one embodiment, the epidermal layer is derived from an internal organ. In one embodiment, the epidermal layer is derived from a mucosal membrane. In one embodiment, the epidermal layer comprises a skin protein. In one embodiment, the skin protein comprises involucrin. In one embodiment, the skin protein comprises loricrin. In one embodiment, the skin protein comprises cytokeratin. See, Figure 10.

Enzyme-linked immunosorbent assay plates were coated with 5 mg of human keratin in 100 µl of PBS/well overnight at 4°C. Wells were then washed three times with PBS and then blocked with 1% bovine serum albumin in PBS for 1 h before the addition of spores. Spores (c. *difficile* prepared as described above) were diluted to a concentration having an absorbance of 1.0 at 600 urn in PBS (approximately 5x10⁷ spores/ml) before being labeled with FITC. 100 µl oflabeled spores were added per well, and the plates were incubated at 37°C for 1 h. The total fluorescence (Fₜₒₜₐₗ per well was measured after a 1-h incubation using a Fluoroskan II fluorescence reader (Labsystems, Beverly, MA), with λₑₓ = 485 urn and λₑₘ = 535 urn. The wells were washed with PBS three times to remove unbound spores and the remaining fluorescence (Fₜₑₛₜ) measured. Adherence was calculated as follows: adherence = F_{fest}/Fₜₒₜₐₗ. Adherence oflabeled cells in the absence of antibodies or infection control compositions was normalized to 100%. Bovine serum albumin-coated wells were used as negative controls. For inhibition assays, FITC-labeled spores were first incubated with anti- spore antibodies or infection control compositions for 1 hat 37°C before addition of the mixture to the keratin-coated wells.

Alternatively, EpiDerm™ Skin Model plates (24-well or 96-well) were blocked with 1% bovine serum albumin in PBS for 1 h before the addition of spores. Spores (*C. difficile* prepared as described above) were diluted to a concentration having an absorbance of 1.0 at 600 urn in PBS (approximately 5x10⁷ spores/ml) before being labeled with FITC. 100 µl oflabeled spores were added per well, and the plates were incubated at 37°C for 1 h. The total fluorescence (Ftotal) per well was measured after a 1- h incubation using a Fluoroskan II fluorescence reader (Labsystems, Beverly, MA), with λₑₓ = 485 nm and λₑₘ = 535 nm. The wells were washed with PBS three times to remove unbound spores and the remaining fluorescence (Ftest) measured. Adherence was calculated as follows: adherence = Fₜₑₛₜ/Fₜₒₜₐₗ. Adherence of labeled cells in the absence of antibodies or infection control compositions was normalized to 100%. Bovine serum albumin-coated wells were used as negative controls. For inhibition assays, FITC-labeled spores were first incubated with anti- spore antibodies for 1 h at 37°C before addition of the mixture to the EpiDerm™ Skin Model plates.

### E. High-Throughput Screening of Infection Control Compositions

There are described methods comprising high throughput assays for screening ofinfection control compositions. For example, high throughput binding assays for proteins and/or nucleic acids have been developed; i) high throughput screening methods for proteins (U.S. Pat. No. 5,559,410); ii) high throughput screening methods for nucleic acid binding (i.e., in arrays) (U.S. Pat. No. 5,585,639); and iii) high throughput methods of screening for ligand/antibody binding (U.S. Pat. Nos. 5,576,220 and 5,541,061)(all patents herein incorporated by reference). In addition, high throughput screening systems are commercially available (see, e.g., Zymark Corp., Hopkinton, Mass.; Air Technical Industries, Mentor, Ohio; Beckman Instruments, Inc. Fullerton, Calif.; Precision Systems, Inc., Natick, Mass., etc.). These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols the various high throughput. Thus, for example, Zymark Corp. provides technical bulletins describing screening systems for detecting the modulation of gene transcription, ligand binding, and the like.

Conventionally, new chemical entities with useful properties are generated by identifying a chemical compound (called a "lead compound") with some desirable property or activity, creating variants of the lead compound, and evaluating the property and activity of those variant compounds. However, the current trend is to shorten the time scale for all aspects of drug discovery. Because of the ability to test large numbers quickly and efficiently, high throughput screening (HTS) methods are replacing conventional lead compound identification methods.

In one embodiment, high throughput screening methods involve providing a library containing a large number ofpotential therapeutic compounds (candidate compounds). Such "combinatorial chemical libraries" are then screened in one or more assays, as described herein, to identitY those library members (particular chemical species or subclasses) that display a desired characteristic activity. The compounds thus identified can serve as conventional "lead compounds" or can themselves be used as potential or actual therapeutics.

### 1. Combinatorial Chemical Libraries

Recently, attention has focused on the use of combinatorial chemical libraries to assist in the generation of new chemical compound leads. A combinatorial chemical library is a collection of diverse chemical compounds generated by either chemical synthesis or biological synthesis by combining a number of chemical "building blocks" such as reagents. For example, a linear combinatorial chemical library such as a polypeptide library is formed by combining a set of chemical building blocks called amino acids in every possible way for a given compound length (i.e., the number of amino acids in a polypeptide compound). Millions of chemical compounds can be synthesized through such combinatorial mixing of chemical building blocks. For example, a systematic, combinatorial mixing of 100 interchangeable chemical building blocks may result in the theoretical synthesis of 100 million tetrameric compounds or 10 billion pentameric compounds.

Combinatorial chemical libraries include, but are not limited to, peptide libraries. U.S. Pat. No. 5,010,175*,* Furka et al., Int. J Pept. Prot. Res., 37: 487-493 (1991); and Houghton et al., Nacure 354:84-88 (1991) . Peptide synthesis is by no means the only approach envisioned and intended for use with the described methods. Other chemistries for generating chemical diversity libraries can also be used. Such chemistries include, but are not limited to: peptoids *(*PCT Publication No WO 91/19735, 26 Dec. 1991), encoded peptides *(*PCT Publication WO 93120242, 14 Oct. 1993), random bio-oligomers *(*PCT Publication WO 92100091, 9 Jan. 1992), benzodiazepines *(*US. Pat. No. 5,288, 514), diversomers such as hydantoins, benzodiazepines and dipeptides (Hobbs et al., Proc. Nat. Acad. Sci. USA 90:6909-6913 (1993), vinylogous polypeptides (Hagihara et al., J Amer. Chem. Soc. 114:6568 (1992), nonpeptidal peptidomimetics with a Beta-D-Glucose scaffolding (Hirschmann et al., J. Amer. Chem. Soc. 114:9217-9218 (1992), analogous organic syntheses of small compound libraries (Chen et al., J. Amer. Chem. Soc. 116:2661 (1994), oligocarbamates (Cho et al., Science 261:1303 (1993), and/or peptidyl phosphonates (Campbell et al., J. Org. Chem. 59 658 (1994); nucleic acid libraries, peptide nucleic acid libraries (U.S. Pat. No. 5,539,083) antibody libraries (Vaughn et al., Nature Biotechnology 14(3): 309-314 (1996), and PCT/US9611 0287), carbohydrate libraries (Liang et al. Science 274:1520-1522 (1996), and U.S. Pat. No. 5,593,853), and small organic molecule libraries - benzodiazepines (Baum C&EN, January 18, page 33 (1993); isoprenoids (U.S. Pat. No. 5,569,588); thiazolidinones and metathiazanones, U.S. Pat. No. 5,549,974; pyrrolidines, U.S. Pat. Nos. 5,525,735 and 5,519,134; morpholino compounds, U.S. Pat. No. 5,506,337; benzodiazepines, 5,288,514; and the like).

Devices for the preparation of combinatorial libraries are commercially available (See, e.g., 357 NIPS, 390 NTS, Advanced Chern Tech, Louisville Ky., Symphony, Rainin, Woburn, Mass., 433A Applied Biosystems, Foster City, Calif., 9050 Plus, Millipore, Bedford, Mass.).

A number of robotic systems have also been developed for solution phase chemistries. These systems include automated workstations like the automated synthesis apparatus developed by Takeda Chemical Industries, LTD. Osaka, Japan) and many robotic systems utilizing robotic arms (Zymate II, Zymark Corporation, Hopkinton, Mass.; Orca, Hewlett-Packard, Palo Alto, Calif.) which mimic the manual synthetic operations performed by a chemist. Any of the above devices are suitable for use with the described methods. **In** addition, numerous combinatorial libraries are themselves commercially available. ComGenex, Princeton, N.J.; Asinex, Moscow, Ru; Tripos, Inc., St. Louis, Mo.; ChemStar, Ltd, Moscow, RU; 3D Pharmaceuticals, Exton, Pa.; or Martek Biosciences, Columbia, Md..

### 2. High Throughput Assays of Chemical Libraries

Any of the assays for compounds inhibiting the attachment of bacterial spores as described herein are amenable to high throughput screening. As described above, in a preferred embodiment, the assays screen for agents that interact with a microbial spore surface protein.

High throughput implementation of the assays described herein can be implemented with, at most, routine modification of the assays format (e.g., for compatibility with robotic manipulators, large plate readers, and the like). Various high throughput screening systems (e.g., for protein binding, nucleic acid binding, etc.) are described. U.S. Pat. Nos. 5,559,410*,* 5,585,639*,* 5,576,220*, and* 5,541,061.

In addition, high throughput screening systems are commercially available. Zymark Corp., Hopkinton, Mass.; Air Technical Industries, Mentor, Ohio; Beckman Instruments, Inc. Fullerton, Calif.; or Precision Systems, Inc., Natick, Mass. These systems typically automate entire procedures including all sample and reagent pipetting, liquid dispensing, timed incubations, and final readings of the microplate in detector(s) appropriate for the assay. These configurable systems provide high throughput and rapid start up as well as a high degree of flexibility and customization. The manufacturers of such systems provide detailed protocols the various high throughput. Thus, for example, Zymark Corp. provides technical bulletins describing screening systems for detecting the modulation of gene transcription, ligand binding, and the like.

### V. Evaluation Of Infection Control Test Compounds

In one embodiment, there is described a method for detecting and/or quantifying of an amount, or rate, ofinteraction between a microbial spore surface protein and an infection control test compound. In some embodiments, detecting methods include, but are not limited to, video microscopy; DAPI fluorescence changes, fluorescence resonance energy transfer or centrifugation.

### A. Microscopy

In one embodiment, an infection control test compound is detected by microscopy (i.e., for example, by direct visual observation or using a video or photographic recording device). Assays involving microscopic visualization may utilize *Clostridium* spore surface peptides and/or another microbial spore surface protein comprising a labeled (e.g., fluorescently labeled). In one embodiment, the peptides and/or proteins are placed adjacent to a solid support (e.g., a glass slide), and exposed to a solution containing an infection control test compound. Attachment of the peptides and/or proteins to the solid support can be directly visualized using a microscope (i.e., for example, a scanning electron microscope).

A microscope can optionally be equipped with a still camera or a video camera and may be equipped with image acquisition and analysis software to quantify the relative abundance of protein and/or peptide attachment.

Any label that can be visualized in a microscope can be used in conjunction with this type of detection. Such labels include, but are not limited to, fluorescent labels (e.g., fluorescein, rhodamine, etc.), calorimetric labels, and radioactive labels (with appropriate scintillation screen), and the like. In some embodiments of the assays of this invention, the microtubules can be visualized without any label (e.g., via differential interference contrast microscopy).

### B. DAPI Fluorescence Changes.

In another embodiment, the interaction of an infection control test compound with a *Clostridium* spore surface peptide and/or another microbial spore surface protein can be determined by changes in fluorescence utilizing a label comprising DAPI. A decreased rate or amount in DAPI fluorescence is indicative of an interaction between a test compound and a microbial spore surface protein. A change in fluorescence with a test compound is compared to that observed in a negative and/or positive control reaction.

Other labels that can be used include, but are not limited to, anilinonapthalene sulfonate (ANS) (e.g., Molecular Probes Catalogue Nos: A-47, A-50, T-53, etc.), bisANS (Molecular Probes Catalogue No: B-153), N-phenyl-1-naphthylene (NPN) (Molecular Probes Catalogue No: P65), DCV] (Molecular Probes Catalogue No: D3923), ruthenium red, and cresol violet.

### C. Fluorescence Resonance Energy Transfer

The degree ofinteraction between a *Clostridium* spore surface peptide and/or another microbial spore surface protein and an infection control test compound can also be determined by fluorescent resonance energy transfer (FRET). Fluorescence resonance energy transfer, a phenomenon that occurs when two fluorophores with overlapping absorption and emission spectra are located close together (e.g., <7 mm apart). Stryer et al., Ann. Rev. Biochem., 47:819-846 (1978). In one embodiment, there is described a mehtodo of detecting protein-protein interactions using FRET. Taylor et al., J Cell Biol. 89:362-367 (1981).

In one embodiment, equimolar proportions of differently labeled microbial spore surface peptide and an infection control test peptide compound are combined (e.g., fluorescein labeled and rhodamine-labeled, respectively). Fluorescence may be quenched upon the interaction of the microbial spore surface peptide with the peptide test compound, indicating that the respective bound fluorochromes come in close enough proximity for energy transfer to occur. The rates and/or amount of fluorescence generated by a reaction with a test compound and a control can be compared.

### VI. Recombinant Expression Of Microbial Spore Surface Proteins And Peptides

In one embodiment, *Clostridium* spore surface peptides and/or another microbial spore surface proteins are synthesized using recombinant DNA methodology. Generally this involves creating a DNA sequence that encodes a protein, placing the DNA in an expression cassette under the control of a particular promoter, expressing the protein in a host, isolating the expressed protein and, if required, renaturing the protein. DNA encoding a peptide and/or protein as contemplated by this invention can be prepared by many suitable methods, including, for example, cloning and restriction of appropriate sequences or direct chemical synthesis by methods such as the phosphotriester method ofNarang et al., Meth. Enzymol. 68: 90-99 (1979); the phosphodiester method of Brown et al., Meth. Enzymol. 68: 109-151 (1979); the diethylphosphoramidite method of Beaucage et al.., Tetra. Lett., 22: 1859-1862 (1981); and the solid support method of U.S. Pat. No. 4,458,066 (all above reference herein incorporated by reference in their entirety).

Generally, the nomenclature used hereafter and the laboratory procedures in cell culture, molecular genetics, and nucleic acid chemistry and hybridization described below will be understood by those having ordinary skill in the art. Standard techniques are used for recombinant nucleic acid methods, polynucleotide synthesis, and microbial culture and transformation (e.g., electroporation, lipofection). Generally, enzymatic reactions and purification steps are performed according to the manufacturer's specifications supplied with commercial kits. The techniques and procedures are generally performed according to conventional methods in the art and various general references. Sambrook et aI., In: Molecular Cloning: A Laboratory Manual, 2d ed. Cold Spring Harbor Laboratory Press, Cold Spring Harbor, N.Y., (1989); and In: Current Protocols in Molecular Biology John Wiley and Sons, Inc., N.Y. (1996).

There are disclsoed nucleic acid sequence which encodes a spore surface polypeptide sequence or its variants; these nucleic acid sequences are used to make recombinant molecules which express a spore surface protein. For example, the redundancy of the genetic code permits many nucleic acid sequences that encode a spore surface polypeptide. Thus, codons which are different from SEQ ID NOs: 2,4, 6 or 8 may be used to increase the rate of expression of a spore surface nucleotide sequence. Additionally, alternative codons may also be used in eukaryotic expression hosts to generate splice variants of recombinant RNA transcripts which have other properties (i.e., for example, longer or shorter half-life). In addition, different codons may also be desirable for the purpose of altering restriction enzyme sites or, in eukaryotic expression hosts, of altering glycosylation patterns in translated polypeptides.

Variants of microbial spore surface protein nucleotide sequences (i.e., for example, a *Clostridium* spore surface protein) are also included within the scope of this invention. These variants include, but are not limited to, nucleotide sequences having deletions, insertions or substitutions of different nucleotides or nucleotide analogs as long as the biological activity of the translation product of the nucleotide sequence is maintained. This invention is not limited to the *Clostridium* spore surface protein nucleic acid sequences (i.e., for example, SEQ ID NO: 2) but specifically includes nucleic acid homologues which are capable ofhybridizing to SEQ ID NO: 2 (i.e., for example, *Bacillus* homologues; SEQ ID NOs: 3, 5, and 7), and to portions, variants and derivatives thereof, under different hybridization stringencies. For example, higher stringencies may reduce or eliminate nonspecific binding between SEQ ID NO:2 and other nucleic acid sequences. Alternatively, lower stringencies may detect a larger number of nucleic acid sequences having different homologies to SEQ ID NO:2. Fragments ofSEQ ID NO:2 (i.e., also referred to as a portion) are also specifically contemplated to be within the scope of this invention. In one embodiment, a fragment has a length equal to or greater than 10 nucleotides and shows greater than 50% homology to SEQ ID NO:2.

Thre are disclosed antisense molecules comprising the nucleic acid sequence complementary to at least a portion of a polynucleotide of SEQ ID NO:2 or at least a portion of a polynucleotide encoding a spore surface protein. Also disclosed isf a fusion gene comprising at least a portion, variant, derivative and/or homologue of a nucleic acid selected from the group comprising SEQ ID NO:2 or a polynucleotide encoding a spore surface protein ligated to one or more heterologous sequences. Although it is not necessary to understand the mechanism of an invention, it is believed that such a fusion gene may detect expression of nucleic acid sequences. Examples of a heterologous sequence include, but are not limited to, a reporter sequence encoding enzymes such as ∼-galactosidase or luciferase. Fusion genes may also facilitate purification of the expressed protein. For example, a heterologous sequence of protein A allows purification of a fusion protein on an immobilized immunoglobulin. Other affinity traps can also be utilized to purify expressed fusion protein. For example, pGEX vectors (Promega, Madison, Wis.) may be used to express a spore surface protein as a fusion protein with glutathione S-transferase (GST). In general, such fusion proteins are soluble and can easily be purified from lysed cells by adsorption to glutathione-agarose beads followed by elution in the presence of free glutathione. Proteins made in such systems are designed to include heparin, thrombin, or factor XA protease cleavage sites so that the cloned polypeptide of interest can be released from the GST moiety at will.

### A. Recombinant Expression Of Representative Spore Surface Proteins

There is described a method for expression recombinant microbial spore surface proteins. In one embodiment, the method comprises constructing a plasmid encoding at least a portion of a microbial spore surface protein. In one embodiment, the method comprises transfecting a suitable organism with the plasmid. In one embodiment, the suitable organism is *E. coli.*

The data presented herein demonstrates the successful cloning of a transfected E. coli with a plasmid comprising nucleic acids encoding at least a portion of a C. *difficile* spore surface protein. In one embodiment, the spore surface protein is CDI067. In one embodiment, the spore surface protein is CD3620. The following experiment is merely intended to be illustrative, and is not intended to be limiting, of the present embodiment as similar results may be obtained with other spore surface protein nucleic acid constructs using the basic technique described below.

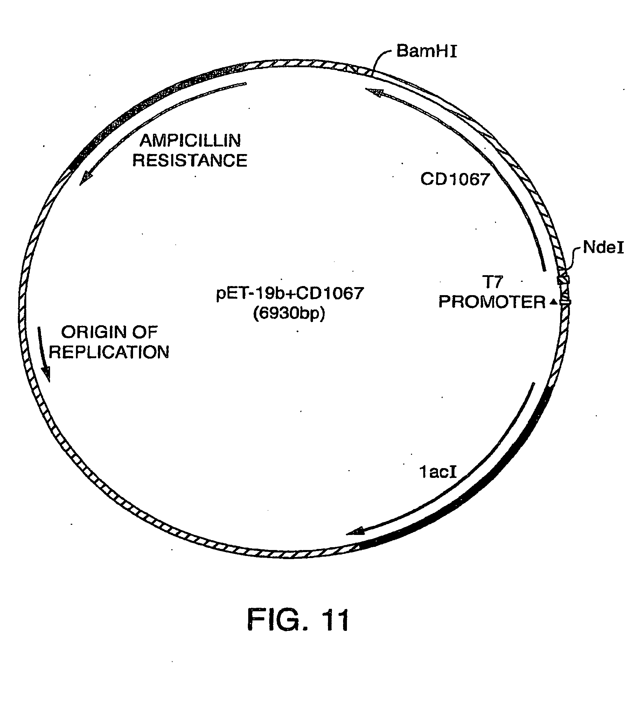

Cloning of C. *difficile* suspected surface proteins was performed in *E. coli Rosetta-Gami* expression strain via an pET-19b plasmid (Novagen, product number 69677-3) having a ten (10) amino acid His tag.. Specifically, pET-19b+CD1067 (pJEB02) or pET-19b+CD3620 (pJEB03) was incubated in an overnight culture (i.e., for example, 12 hr) comprising *E*. *coli Rosetta-Gami* cells. See, Figure 11.

Approximately 2 ml of the transfected *E. coli* cell culture was transferred to an expression culture medium comprising 50 ml Luria Broth (LB) + 100 Ilg/ml adenosine monophosphate (AMP) and 34 Ilg/ml chloramphenical. After the culture was grown to approximately OD₆₂₅ to 0.5-0.6, expression of the pET plasmid was induced with 1mM IPTG. Following IPTG induction, 1 ml medium samples were taken at approximate one hour intervals. The samples were centrifuged (i.e., for example, approximately 10,000 x g for 1 min), and the resulting pellet was resuspended in 100 µl Novex solution (45 µl 2x SDS-PAGE Sample Buffer + 45 µl H20 + 10 µl 2M dithiothreitol) and boiled for approximately 5 minutes. Approximately 20 µl of the boiled pellet suspension was initially run on a 4-12% Tris-Glycine SDS-PAGE gel, whereupon approximately 10 µl of the suspected isolated recombinant protein was used for a Western Blot analysis.

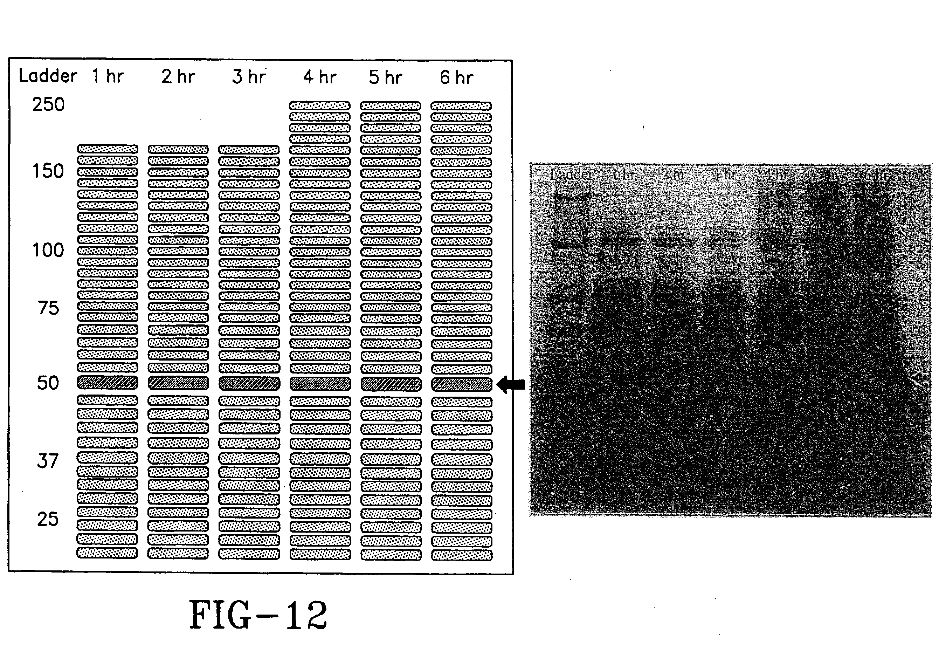

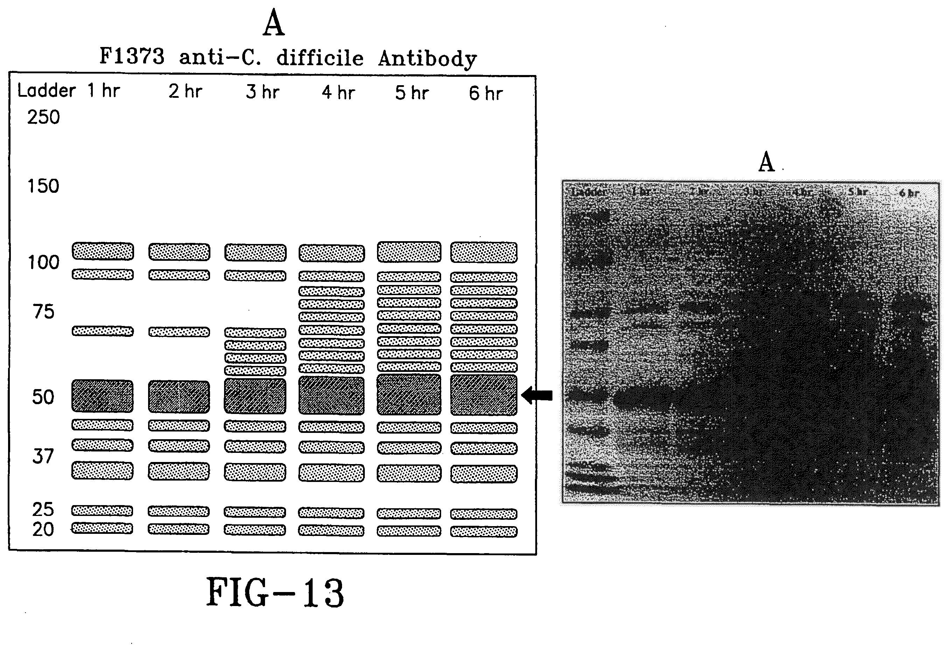

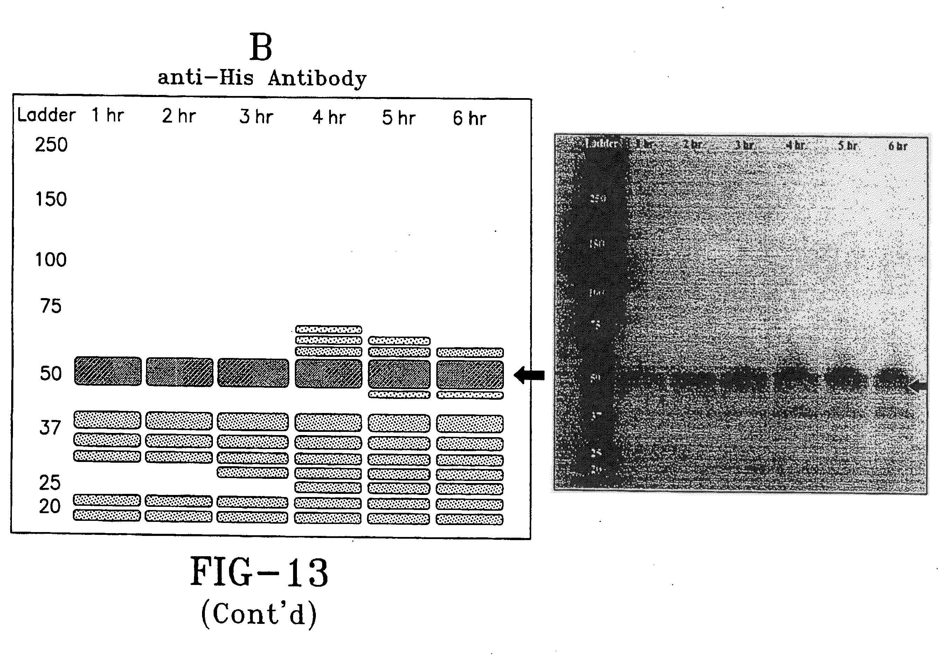

The pJEB02 plasmid was expected to express a protein having 405 amino acids with an approximate molecular weight of 44 kDa. SDS-PAGE gel electrophoresis separation confirms this expectation showing that expression of a protein of approximately 50 kDa appeared within one hour after IPTG induction and increased in intensity in subsequent samples (i.e., 2 - 6 hour samples). See, Figure 12. This preliminary separation was confirmed using Western Blot analysis detected by either F1373 anti-C. *difficile* antibody or anti-His antibody. See, Figure 13A and Figure 13B, respectively.

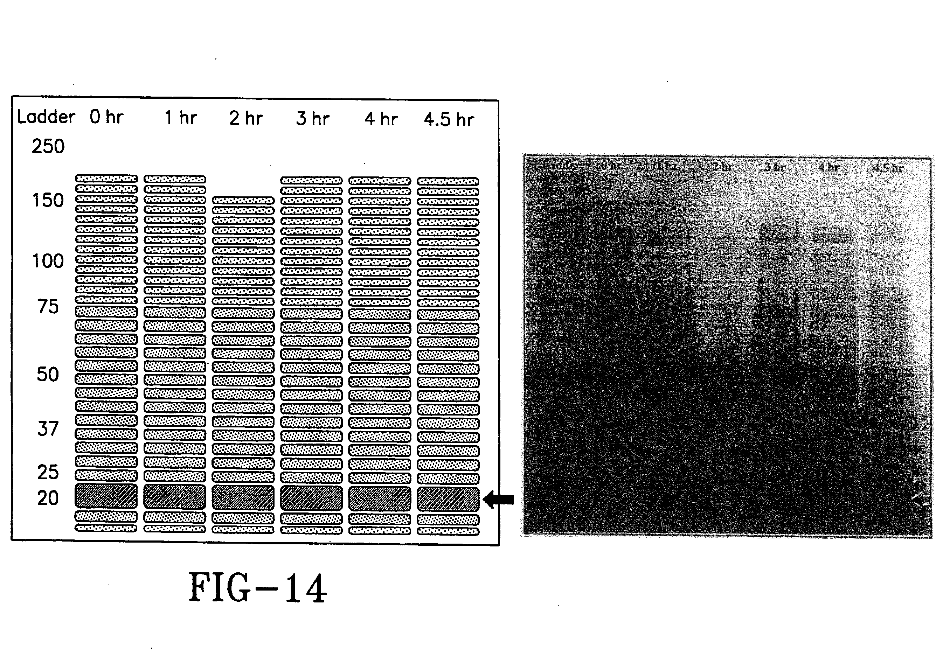

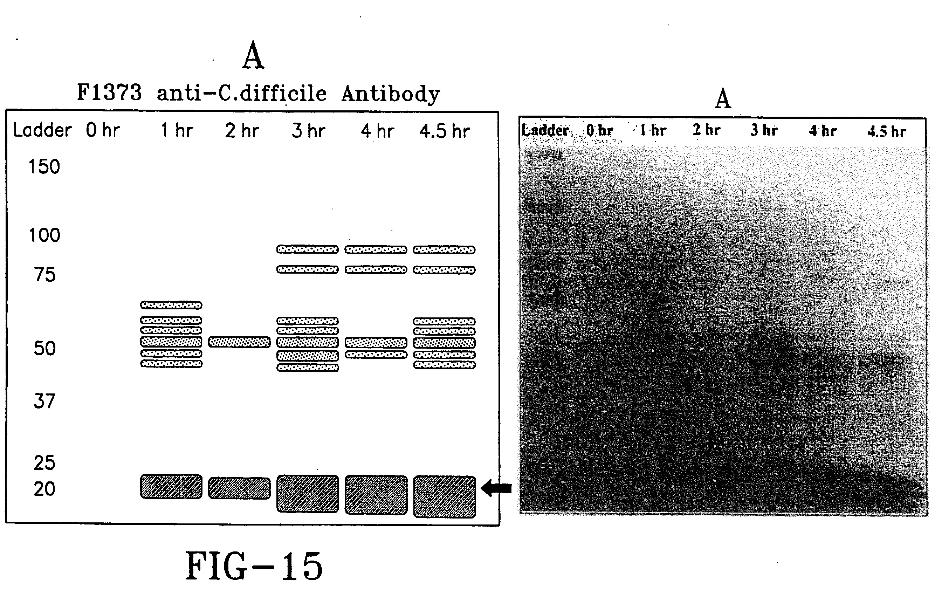

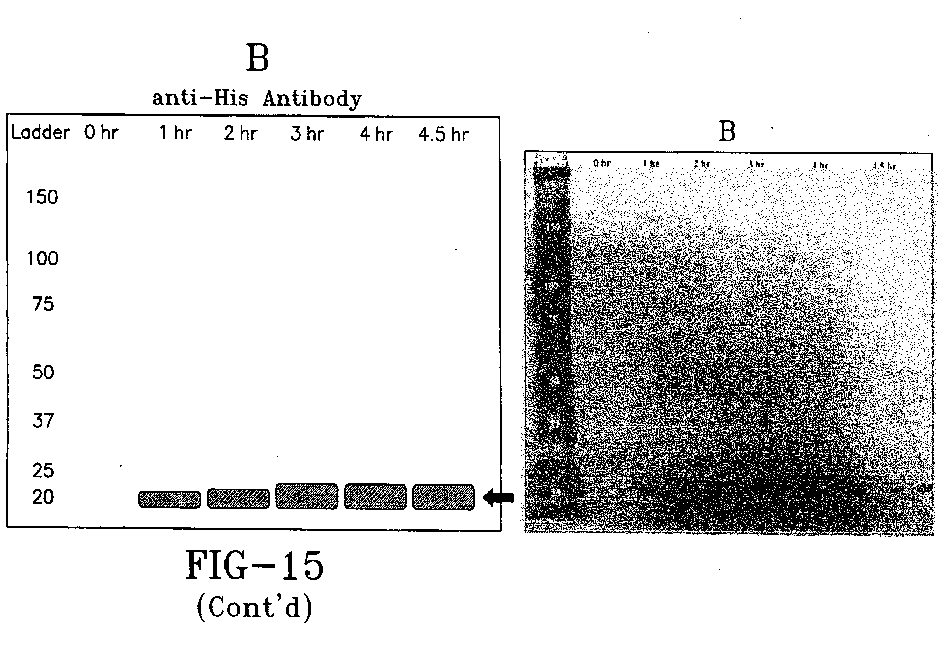

The pJEB03 plasmid was expected to express a protein having 122 amino acids with an approximate molecular weight of 14 kDa. SDS-PAGE gel electrophoresis separation confirms this expectation showing that expression of a protein of approximately 20 kDa appeared within one hour after IPTG induction and increased in intensity in subsequent samples (i.e., 2 - 4.5 hour samples). See, Figure 14. This preliminary separation was confirmed using Western Blot analysis detected by either F1373 anti-C *difficile* antibody or anti-His antibody. See, Figure 15A and Figure 15B, respectively.

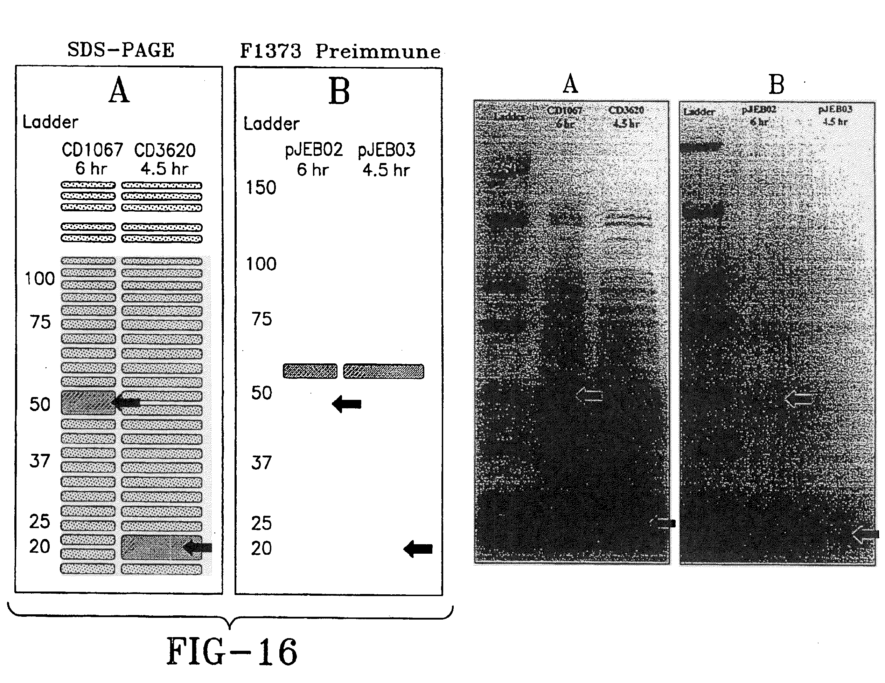

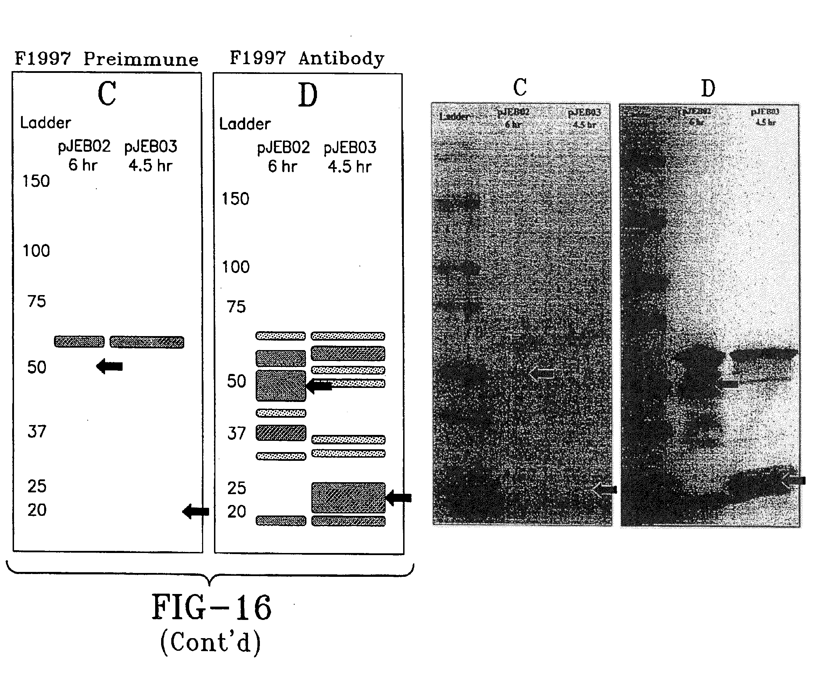

Preimmune Western Blot control samples for both the F1373 anti-C *difficile* antibody or the F1997 anti-C *difficile* antibody (e.g., commercial customized preparations directed against spore surfaces; Strategic Diagnostics, Inc.) demonstrated an absence of binding specificity in both pJEB02 and pJEB03 expression profiles. See, Figure 16A-D. These data show that when CDI067 and CD3620 are cloned into E. coli, a preimmune control detection is not reactive with either a recombinant CDI 067 protein or a recombinant CD3620 protein. In contrast, a C *difficile* spore surface antibody (i.e., for example, F1373 or F1997) reacts specifically with a recombinant CDI067 protein and a recombinant CD3620 protein. Specific expression of these proteins was confirmed by the detection of a HislO tag attached to the expression plasmid construct that corresponds with the MW of both a recombinant CDI067 protein and a CD3620 recombinant protein.

### B. Nucleic Acid Sequence Synthesis Techniques

Synthetic chemistry techniques may also be used to generate nucleic acid sequences. Oligonucleotides can be synthesized on an Applied BioSystems oligonucleotide synthesizer according to specifications provided by the manufacturer. Sinha et al., Nucleic Acids Res. 12:4539 (1984). Following synthesis, complementary oligonucleotides can be annealed by heating them to 90°C in a solution of 10 mM Tris-HCI buffer (pH 8.0) containing NaCI (200 mM) and then allowing them to cool slowly to room temperature. For binding and turnover assays, duplex DNA is purified from native polyaclylamide (15% w/v) gels. The band corresponding to double-stranded DNA is excised and soaked overnight in 0.30 M sodium acetate buffer (pH 5.0) containing EDTA (1 mM). After soaking, the supernatant is extracted with phenol/chloroform (1/1 v/v) and precipitated with ethanol. DNA substrates are radiolabeled on their 5'-OH group by treatment with ³²P-ATP and T4 polynucleotide kinase. Salts and unincorporated nucleotides are removed by chromatography on Sephadex G columns.

A synthesized nucleotide sequence may be confirmed using commercially available kits comprising enzymes including, but not limited to, Klenow fragment of DNA polymerase I, Sequenase®, Taq DNA polymerase, or thermostable T7 polymerase. Capillary electrophoresis may also be used to analyze a nucleic acid sequence size and confirm sequences nucleic acid synthesis products. Synthesized sequences may also be amplified by: i) polymerase chain reaction (PCR) (Mullis et al., U.S. Pat. No. 4,683,195*;* and Mullis et al., U.S. Pat. No. 4,683,202*,* the ligase chain/amplification reaction (LCR/LAR); Barany et al.., Froc. Natl. Acad. Sci., 88:189 (1991); Barany et al., PCR Methods and Applic., 1:5 (1991); and Wu et al., Genomics 4:560 (1989).

A spore surface protein nucleotide sequence may be used in a variety of ways. For example, fragments of a sequence of at least about 10 bp, more usually at least about 15 bp, and up to and including the entire (i.e., full-length) sequence can be used as probes for the detection and isolation of complementary genomic DNA sequences from *Bacillus, Clostridia,* as well as other bacteria. Genomic sequences are isolated by screening genomic library containing bacterial DNA with all or portion of a spore surface protein nucleic acid sequence. In addition to screening genomic libraries, a spore surface protein nucleic acid sequence can also be used to screen cDNA libraries made using bacterial RNA. A spore surface protein nucleic sequence is also useful in directing the synthesis of a spore surface protein. spore surface protein antibodies may also be used to antagonize the interaction of spore surface proteins to mammalian cells, mammalian skin, hard surfaces etc. A spore surface protein finds use in producing a spore surface protein antibodies for diagnostic purposes such as detecting infections with bacteria which express a spore surface protein. A spore surface protein nucleic acid sequence is also useful for the screening ofbinding antagonists and the detection ofmicrobes which contain spore surface proteins.

Chemical synthesis usually produces a single stranded oligonucleotide. This may be converted into double stranded DNA by hybridization with a complementary sequence, or by polymerization with a DNA polymerase using the single strand as a template. Chemical synthesis ofDNA is routinely performed for sequences of about 100 bases, but longer sequences may be obtained by the ligation of shorter sequences.

Alternatively, subsequences may be cloned and the appropriate subsequences cleaved using appropriate restriction enzymes. The fragments may then be ligated to produce the desired DNA sequence.

In one embodiment, a microbial spore surface protein nucleic acid can be cloned using DNA amplification methods such as polymerase chain reaction (PCR). Thus, for example, a nucleic acid sequence or subsequence is PCR amplified, using a sense primer containing one restriction site (e.g., NdeI) and an antisense primer containing another restriction site (e.g., HindIII). This will produce a nucleic acid encoding the desired protein comprising terminal restriction sites. This nucleic acid can then be easily ligated into a vector containing a nucleic acid encoding the second molecule and having the appropriate corresponding restriction sites.

Suitable PCR primers can be determined using the sequence information provided herein. Appropriate restriction sites can also be added to the nucleic acid encoding the microtubule depolymerizing or severing proteins by site-directed mutagenesis. The plasmid containing the microtubule depolymerizing or severing protein encoding nucleic acid is cleaved with the appropriate restriction endonuclease and then ligated into the vector encoding the second molecule according to standard methods.

Nucleic acid sequences encoding *Clostridium* spore surface peptides and/or other microbial spore surface proteins may be expressed in a variety of host cells, including *E. coli,* other bacterial hosts, yeast, and various higher eukaryotic cells such as the COS, CRO and HeLa cells lines and myeloma cell lines. A recombinant protein gene will usually be operably linked to appropriate expression control sequences for each host. For *E. coli,* preferred promoters include, but are not limited to, T7, trp, or lambda promoters. Further, a ribosome binding site and a transcription termination signal is part of the expression vector. For eukaryotic cells, the control sequences will include a promoter and preferably an enhancer derived from immunoglobulin genes, SV40, cytomegalovirus, etc., and a polyadenylation sequence, and may include splice donor and acceptor sequences.

The plasmids of the invention can be transferred into the chosen host cell by methods including, but not limited to, calcium chloride, calcium phosphate, or electroporation. Cells transformed by the plasmids can be selected by resistance to antibiotics conferred by genes contained on the plasmids, such as the *amp, gpt, neo* and *hyg* genes.

Once expressed, the recombinant proteins can be purified according to standard procedures including, but not limited to, ammonium sulfate precipitation, affinity columns, column chromatography, gel electrophoresis and the like. R. Scopes, In: Protein Purification, Springer-Verlag, N.Y. (1982); and Deutscher et al., In: Methods in Enzymology Vol. 182 (1990). Substantially pure compositions of at least about 90 to 95% homogeneity are preferred, and 98 to 99% or more homogeneity are most preferred. Once purified, partially or to homogeneity as desired, the polypeptides may then be used e.g., as immunogens for antibody production).

### VII. Clostridium Infections

*Clostridium difficile,* a gram-positive anaerobic bacterium, frequently causes morbidity in hospitalized patients and is believed an etiological agent of antibiotic- associated diarrhea and pseudomembranous colitis. Borriello et aI., "Virulence factors of Clostridium difficile" Rev. Infect. Dis. 12:S185-S191 (1990); Cerquetti et al., "Characterization of surface layer proteins from different Clostridium difficile clinical isolates" Microb. Pathog. 28:363-372 (2000); and Kuipers et al., "Quorum sensingcontrolled gene expression in lactic acid bacteria" J Biotechnol. 64: 15-21 (1998). Two factors have been evaluated for their involvement in the pathogenesis of these conditions: i) the suppression of the resident intestinal flora by the administration of antibiotics (Freeman et al., "Antibiotics and Clostridium difficile" Microbes Infect. 1:377-384 (1999); and George W., "Antimicrobial agent-associated colitis and diarrhea: historical background and clinical aspects" Rev. Infect. Dis. 6:S208-S213 (1984); and ii) the production by the bacterium oftwo high-molecular-weight (MW) toxins (toxins A and B) (Pothoulakis et al., "Microbes and microbial toxins: paradigms for microbial-mucosal interactions. II. The integrated response of the intestine to Clostridium difficile toxins" Am. J Physiol. -Gastroint. Liver Physiol. 280:G178-G183 (2001); and Wren B., "Molecular characterisation of Clostridium difficile toxins A and B" Rev. Med. Microbiol. 3:21-27 (1992).

As with other enterotoxigenic pathogens, delivery of toxins follows C. *difficile* colonization ofthe gut, which requires bacterial adherence to the mucosa. In a Syrian hamster model of clindamycin-induced disease, a correlation was reported between variable efficiencies of gut colonization by different C. *difficile* strains and their abilities to associate with regions of the gastrointestinal tract spanning from the jejunum to the colon. At least part of the variation was suggested to be due to factors other than toxin production. However, the adherence of a poorly virulent strain to the small bowel mucosa was increased by the administration of a crude toxin preparation, possibly due to the unmasking of receptor sites following cell damage.

Two virulence factors from C. *difficile* have been shown to be exotoxins, toxin A (TcdA) and toxin B (TcdB) 3, little is known about other factors involved in the adherence and colonization processes. Voth et al.., "Clostridium difficile toxins: mechanism of action and role in disease" Clin. Microbiol. Rev. 18, 247-263 (2005). This is in contrast to other enteric organisms such as *Escherichia coli*, *Salmonella*, and *Shigella spp.* Possibly, this might be partly explained by the lack of well-developed mutagenesis systems for studying C. *difficile.* One approach to possibly understanding C. *difficile* involves identifying the genomic locations of potential factors involved in pathogenicity. The complete genome sequence of C. *difficile strain* 630 (epidemic type X; virulent and multidrug resistant) has been reported. Wust et al., "Investigation of an outbreak of antibiotic-associated colitis by various typing methods" Clin. Microbiol 16:1096-1101 (1982).

The genome of C. *difficile* strain 630 comprises a circular chromosome of 4,290,252 bp and a plasmid, pCD630, of 7,881 bp. The chromosome encodes 3,776 predicted coding sequences (CDSs); in common with other low-G+C content Gram positive bacteria, the chromosome has a strong coding bias, with 82.1% of the CDSs encoded on the leading strand. The plasmid carries 11 CDSs, none of which has any obvious function. Reciprocal FASTA analysis against the four sequenced clostridial genomes, C. *acetobutylicum*, C. *botulinum,* C. *perfringens,* and C *tetani,* showed that only 567 (15%) of C *difficile* CDSs are shared with all the sequenced clostridia, whereas 1,893 (50%) are unique to *C. diffcile.* The conserved clostridial CDSs mainly encode essential functions, whereas the C. *difficile* unique CDSs encode many accessory functions and mobile elements. Sebaihia et aI., "The multi-drug-resistant human pathogen Clostridium difficile has a highly mobile, mosaic genome" Nature Genetics 38:779-786 (2006).

Binding of purified *C*. *difficile* surface layer proteins (SLPs) has been observed in both human epithelial cells and gastrointestinal tissues. Calabi et aI., "Binding of Clostridium difficile surface layer proteins to gastrointestinal tissues" Infect Immun 70:5770-5778 (2002). Binding to both cells and tissues is observed with acid-extracted SLPs and with the soluble recombinant high-MW subunit. In contrast, much less or no less binding is observed with a soluble recombinant low-MW SLP subunit, expressed and purified under the same conditions as a recombinant high-MW SLP subunit. Because an anti-high-MW subunit serum partially blocked adherence of C. *difficile* to HEp-2 cells it is possible that SLPs are involved in the adherence of whole bacteria to epithelial cells. It is believed that the natural conformation ofthese proteins are retained during the adhesion because; i) both native and recombinant SLP preparations retained peptidoglycan hydrolase activities; ii) acid-extracted SLPs are capable ofpolymerizing into higher order structures upon the addition of calcium; and iii) antisera raised against both recombinant subunits react at high titers with whole C. difficile cells.

*Clostridium perfringens* is suspected to cause gas gangrene and is a severe form of gangrene (tissue death; also referred to as necrotizing subcutaneous infection. Gas gangrene can also result from infections from *Streptococcus spp.* and *Vibrio vulnificus.*

Gas gangrene usually occurs as a result of infection with a *Clostridium perfringens* bacteria that, under anaerobic (low oxygen) conditions, produce toxins that cause tissue death and associated symptoms. In the United States, gas gangrene is rare, with only 1,000-3,000 cases yearly. Gas gangrene generally occurs at the site of trauma or a recent surgical wound but may also occur spontaneously. Patients who develop gas gangrene spontaneously often have a high risk factor including, but not limited to, an underlying blood vessel disease (i.e., for example, atherosclerosis or hardening of the arteries), diabetes, or colon cancer.

The onset of gas gangrene is generally sudden and dramatic. Inflammation begins at the site ofinfection as a pale-to-brownish-red and extremely painful tissue swelling. Gas may be felt in the tissue as a crackly sensation when the swollen area is pressed with the fingers. The edges ofthe infected area expand so rapidly that changes are visible over a few minutes. The involved tissue is completely destroyed.

*Clostridium perfringens* bacteria produce many different toxins, four of which (i.e., for example, alpha, beta, epsilon, and iota) can cause potentially fatal syndromes. In addition, they cause tissue death (i.e., for example, necrosis), destruction of blood cells (i.e., for example, hemolysis), local decrease in circulation (i.e., for example, vasoconstriction), and leaking of the blood vessels (i.e., for example, increased vascular permeability).

*Clostridium perfringens* toxins are responsible for both the local tissue destruction and the systemic symptoms (the other symptoms that occur throughout the body). Systemic symptoms develop early in a gas gangrene infection and include, but are not limited to, sweating, fever, and anxiety. Ifleft untreated, the subject develops a shock like syndrome including, but not limited to, decreased blood pressure (i.e., for example, hypotension), kidney failure, coma, and finally death. Other gas gangrene symptoms may include, but are not limited to, moderate to severe pain around a skin injury, progressive swelling around a skin injury, moderate to high fever, skin color initially pale, later dusky progressing to dark red or purple, jaundice, vesicle formation, coalescent vesicles combining into large blisters, blisters filled with brown-red fluid, drainage from the tissues, foul-smelling brown-red or bloody fluid (i.e., for example, serosanguineous discharge), increased heart rate (i.e., for example, tachycardia), sweating, subcutaneous emphysema (i.e., for example, air under the skin), or crepitus (i.e., for example, air in the tissues).

Alternatively, the subject may be in shock comprising general pallor, cold extremities, low blood pressure, or rapid heart rate. Ultimately a systemic infection may develop involving the entire body (i.e., for example, systemic toxicity or sepsis). Other methods of determining the presence of gas gangrene include, but are not limited to, a gram stain of fluid from the infected area may shows Gram-positive rods (i.e., for example, a *Clostridium* species); a culture may grow the bacteria causing the infection; blood cultures may grow the infecting bacteria; an anaerobic tissue and/or fluid culture may reveal at least on *Clostridium* species; or an X-ray, computerized tomography scan, or a magnetic resonance image of the affected area may show gas in the tissues

Treatment of gas gangrene generally involves a prompt surgical removal of dead, damaged, and infected tissue (i.e., for example, debridement). Amputation of an arm or leg may be indicated to control the spread of infection. Conventional antibiotics (i.e., for example, of the penicillin) may be intravenously administered as an initial bolus dose followed by a long term oral regimen.

### VIII. Prophylactic And Therapeutic Uses

### A. Hospital Decontamination

Hospital pathogens (i.e., for example, C. *difficile)* are responsible for the vast majority of bacterial infections in both hospital staff and the resident patient population. Such infections may cause symptoms including, but not limited to, severe diarrhea but may also result in death following the infection of a compromised patient (i.e., for example, an immunocompromised patient). Consequently, improved antiseptic maintenance ofboth the hospital facility and staff should facilitate patient recovery and reduce overall health care costs due to shorter hospitalization durations.

Some bacteria (i.e., for example, *C. difficile)* produce spores which have proved highly resistant to common skin and surface disinfectants. Currently available cleansers for both hospital facility surfaces and hospital staff skin (i.e., epidermal layers) are ineffective for killing and/or removing bacterial spores. Anaerobic bacteria (i.e., for example, C. *difficile)* require specialized equipment and handling techniques to work with and manipulate. Consequently, there are currently no easy ways to develop an anaerobic bacteria specific "spore removal handwash".

In fact C. *difficile* is responsible for 15-25% of all cases of antibiotic-associated diarrhea. Recurrences ofthis type ofdiarrhea is a serious, difficult, and still unresolved management problem and increase both the length and overall cost of hospitalization. Most studies demonstrate that hospital recurrences of C. *difficile* infections are split 50:50 between a reinfection with the same, or different, bacterial subtype. Adequately controlling these types of recurrences depends upon the quality of antimicrobial procedures such as handwashing, environmental decontamination, and enteric isolation. Contamination of the environment and persistence of spores have been demonstrated and implicated in cross infections. Barbut et aI., "Epidemiology of recurrences or reinfections of Clostridium difficile-Associated diarrhea" J Clin Microbiol 38: 2386-2388 (2000*).*

In one embodiment, there is disclsoed a composition comprising at least one outermost protein component of an anaerobic bacterial spore. In one embodiment, the anaerobic bacteria comprises a *C*. *difficile* spore. In one embodiment, the protein component is derived from an exosporium layer. In one embodiment, the protein component comprises a spore surface protein. Although it is not necessary to understand the mechanism of an invention, it is believed that the outermost protein component of anaerobic bacterial spore directs the attachment of the spores to tissues of an organism (i.e., for example, skin or gut) or to inanimate hard surfaces (i.e., for example, stainless steel, granite, leather, vinyl, ceramic etc).

In one embodiment, there is descibed a method comprising screening for compositions capable of disrupting attachment interactions between anaerobic spore proteins and tissues or inanimate surfaces. In one embodiment, an outermost protein component of an anaerobic bacterial spore serves as a molecular target for screening such compositions.

A spore surface protein derived from a C. *difficile* exosporium can be expressed and purified by standard methods *(supra).* Consequently, using purified and isolated spore surface protein, simple screening assays may be used to develop anaerobic spore removing products (e.g., handwashes, inanimate surface cleansers, whole body decontamination solutions) without the need to work with the intact C. *difficile* organism. This eliminates the above mentioned need for large scale anaerobic equipment and effectively miniaturizes the screening process.

### B. Emergency First Responders

There are described methods of prophylactic and therapeutic treatment of emergency personnel responding to microbial contamination (i.e., for example, first responders). In one embodiment, emergency first responders may include, but are not limited to, emergency medical technicians (EMTs), fireman, firewomen, policeman, policewomen, journalists, police dogs, or police horses.

The collection and identification ofunknown substances can be a dangerous and generally time consuming procedure. In particular, microbial analysis typically requires specialized equipment in a laboratory. With the recent increase in global terrorism where there is an increased high risk of encountering toxic substances (i.e., for example anthrax) there is a need for quickly preventing and/or treating emergency first responder contamination. Further, screening and/or testing protocols should be readily available without specialized equipment.

With the recent acceleration of terrorist activities around the world, first responders need a readily available composition comprising compounds that provide specific protection against microbial infection. The need for such protection for emergency first responders extends beyond terrorist attacks to events including, but not limited to, natural disasters, truck crashes, and industrial accidents. These events can disperse microbial substances and make prompt identification nearly impossible by removing or destroying written descriptions, warning labels, etc. of said microbial sources.

Without knowing a microbe's identity, emergency first responders put themselves, their families, and others at high risk of being contaminated as well as the person they are treating. It is also necessary that a first responder know what the person they are treating has been exposed to because they cannot properly treat a person without knowing what is causing the person's condition and if first responders do not know what substance a person has been exposed to they might treat that person with a drug which will react with the unknown substance in a negative way.

Emergency first responders are not the only people who need quick and efficient analysis, protection, and treatment of/from unknown substances. Other examples of people who need to have prophylaxis, treatment, and/or quick analysis of an unknown substances include, but are not limited to, a waste clean up person cleaning up an industrial site, a mail room clerk who sees a package leaking a fluid, a police officer who has found an unknown substance in a suspect's car, and a teacher who has found a child unconscious next to an unmarked bottle of chemicals.

Current methods used to test an unknown substance are often times only available at a specialized laboratory. Precious time is wasted waiting for sample transport, assembly oftrained personnel to perform the testing. Consequently, there is disclosed a kit comprising an array oflabeled microbial DNA, a sample DNA extraction solution, and a detector capable of quantitating the label. Such a kit would allow an on-site determination of the type of microbial contamination.

Non-specific decontamination is labor intensive and a time consuming task which may not be effective if microbial spores are present. There is contemplated a readily available decontamination composition comprising a plurality of peptide fragments derived from at least one spore surface protein. Such fragments would displace bacterial spores from solid surfaces (i.e., for example, building walls, ceiling, staircases, office furnishings etc) as well as from the outer epithelial layers (i.e., for example, skin) of emergency first responders. Further, either parenteral, intranasal, pulmonary, or oral compositions suitable for human and/or animal administration would displace bacterial spores from bodily tissues (i.e., for example, mouth, throat, nasal passages, lungs, stomach, intestine, bladder, or colon). Consequently, there is disclosed a composition comprising a compound having an affinity for a spore surface protein, wherein the protein is responsible for the attachment of a microbial spore to a surface (i.e., for example, a solid artificial surface or a skin surface).

### C. Military Applications

There is described prophylactic and therapeutic treatment of military personnel responding to microbial contamination (i.e., for example, first responders). In one embodiment, military personnel may include, but are not limited to, infantry, pilots, cavalry, naval, marine, special forces, logistics, contractors, or journalists.

There is described a readily available decontamination composition comprising a plurality of peptide fragments derived from at least one spore surface protein. Such fragments would displace bacterial spores from solid surfaces (i.e., for example, tents, jeeps, tanks, weapons, uniforms, etc.) as well as from the outer epithelial layers (i.e., for example, skin) of military personnel. Further, either parenteral, intranasal, pulmonary, or oral compositions suitable for human and/or animal administration would displace bacterial spores from bodily tissues (i.e., for example, mouth, throat, nasal passages, lungs, stomach, intestine, bladder, or colon). Consequently, there is described a composition comprising a compound having an affinity for a spore surface protein, wherein the protein is responsible for the attachment of a microbial spore to a surface (i.e., for example, a solid artificial surface or a skin surface).

There is described a method of routine decontamination of ballistic fabrics. At present, garments made of ballistic fabrics are washed by hand with cold water and mild detergent, rinsing thoroughly to remove all traces of detergent. Proper rinsing prohibits the accumulation of residual soap film, which can absorb water and reduce the ballistic resistance of certain types of ballistic fabric. While such a process is time consuming and laborious, it is necessary since machine washing or drying, can be damaging to the fabric, ultimately affecting its ballistic performance. Furthermore, some detergents, dry cleaning solvents, bleach and starch may reduce the garment's level ofballistic resistance and most manufacturers strongly recommend against their use. Furthermore, most manufacturers of ballistic fabric strongly recommend that ballistic fabrics never be submerged in water or dried outdoors, even in the shade, because ultraviolet light causes degradation of certain types of ballistic fabric.

Similarly, the care and cleaning of protective apparel, such as military turnout gear and other protective items such as knee pads, shin guards, shoulder pads and the like, is laborious and time consuming. Repeated cleaning can reduce the useful life of such garments.

A problem common to both protective gear and garments made of ballistic fabric is sweat from the wearer. Ifthe sweat is not removed, fungal and/or bacterial growth can result over time. This fungal and/or bacterial growth ultimately degrades the protective properties of the article, results in noxious odors, and possibly even results in long-term health issues for the wearer. With regard to military turnout gear in particular, all fabrics and components used in the construction of military protective clothing must pass minimum performance requirements. Thus, the inner lining of protective garments designed for military personnel where there is a danger of exposure to weapons fire and/or flame and high temperatures are usually made from aramid fibers and yams.

In one embodiment, there is described a method relating to decontaminating ballistic fabric articles and other military protective gear, wherein the articles and gear comprise components including, but not limited to, aramid, polybenzazole, or high performance polyethylene fiber. In one embodiment, the articles and/or gear are decontaminated by a composition comprising a compound having affinity for a spore surface protein. The composition can be applied to the article directly or to the fiber or as a fabric finish easily and in a cost-effective way.

### IX. Antimicrobial Peptides

Infection control research now includes searching for novel anti-microbial peptides as well as anti-microbial organic chemicals. Antimicrobial peptides have been isolated from plants, insects, fish, amphibians, birds, and mammals. Gallo, J Invest Dermatol., 111:739 -743 (1998); and Ganz et al, Pharmac. Ther., 66:191-205 (1998). These peptides are apparently a primary component of innate host protection against microbial pathogenesis functioning to create pores in the cytoplasmic membrane of microorganisms. Oren et al Biopolymers, 47(6):451-463 (1998). Furthermore, antimicrobial peptides also act on animal cells by stimulating them to change behaviors such as syndecan expression, chemotaxis, and chloride secretion. Gallo, J Invest Dermatol, 111 :739 -743 (1998). After contact with microorganisms, vertebrate skin, trachea and tongue epithelia have been reported to produce peptide antibiotics. Russell et al., Infect Immun, 64(5): 1565-1568 (1996). Antimicrobial peptides, however, have not generally been observed to be effective against microbial spores.

Antimicrobial peptides of higher eukaryotes, though long recognized as components of the innate immune system, were initially considered primitive and of little clinical significance. However, the relative simplicity of these peptides belies their importance, not only in the prevention ofprimary microbial infection, but also in subsequent immunomodulation. Bowman, "Peptide antibiotics and their role in innate impunity," Annu. Rev. Immunol., 13:61-92 (1995). Further, the small size of the molecules suggests a decreased sensitivity to many of the mechanisms of microbial resistance.

Antimicrobial peptides are generally lethal to bacteria and some fungi. They exhibit differential toxicity towards mammalian cells. Although it is not necessary to understand the mechanism of an invention, it is believed that anti-microbial peptides interact with the lipid bilayer and may thus compromise the integrity of the bacterial membrane. Hwang et al., Biochem Cell Biol, 76:235-246 (1998).

SMAP 29 is an ovine antimicrobial peptide of the cathelicidin originally identified through 3' RACE analysis of sheep bone marrow RNA. Mahoney, FEBS Lett, 377:519522 (1995). RCAP 18 is a lupine antimicrobial peptide of the cathelicidin originally identified from granulocytes. Hirata et al., Infect. Immun. 62:421-1426 (1994). It is believed that SMAP 29 and RCAP 18 peptides provide protection against some drug resistant bacterial strains. For example, SMAP 29 compositions are capable of controlling antibiotic resistant infections by *Pseudomonas aeruginosa, Alcaligenes xylosoxidans*, *and Stenotrophomonas maltophila.*

The antibacterial potency of the full-length antimicrobial peptides are apparently correlated directly with a hydrophobicity gradient along their backbone, inversely with their relative abundance of anionic residues, but not with the extent of helix formation in trifluoroethanol.

This invention thus contemplates methods to screen for, and identity, compositions that are capable of inhibiting microbial spore interaction, attachment, and/or stabilization to a solid surface. It is further contemplated that these screening methods would also identity compounds capable ofinhibiting microbial spore interaction, attachment, and/or stabilization to a host organism (i.e., for example, a human, domestic animal, livestock animal, etc).

### X. Combination Vegetative Cell/Spore Antimicrobial Compositions

There are described methods to solve problems related to antimicrobial resistance to common antibiotic drugs. One reason for apparent antimicrobial resistance is that common antimicrobial agents are ineffective against microbial spores. Consequently, when microbial spores are still present after "decontamination" with a conventional antimicrobial compound, the spore will convert into an active microbial growth. This can be (mis)-interpreted as "microbial resistance" when, in fact, the recurring microbial presence is due to an incomplete decontamination technique. In one embodiment, there is described a composition comprising a compound having affinity for a bacterial spore surface peptide in combination with one or more conventional antimicrobial agents or antibiotics.

Although it is not necessary to understand the mechanism, it is believed that a composition comprising a compound having affinity for a microbial surface spore peptide and/or one or more conventional antimicrobial agents or antibiotics will not only effectively kill the active microbes (i.e., for example, vegetative cells) but also detach microbial spores from solid surfaces and or bodily tissues. It is further believed that this combination strategy results in an antiseptic level not possible with decontamination with an antimicrobial agent alone.

A list ofbacterial strains that have been identified with resistance characteristics are listed in Table XIII.

**Table XIII: Mechanisms Of Bacterial Resistance To Antimicrobial Agents**

| Antimicrobial Agent | Mechanisms Causing Resistance | Examples of Organisms |
|---|---|---|
| Aminoglycosides | Modifying enzymes: acetyltransferases, adenylyl-transferases (nucleotidyl-transferases), phosphotransferases Ribosomal resistance (streptomycin, spectinomycin) Inadequate drug transport | Enterobacteriaceae, P. aeruginosa, S. aureus, E. faecalis, E. faecalis, Enterobacteriaceae, M. tuberculosis, P. aeruginosa E. faecalis, P. aeruginosa, anaerobes |
| β-Lactams | Enzymatic inactivation Low affinity PBPs Lack of penetration through outer membrane | S. aureus, E. faecalis, Enterobacteriaceae, P. aeruginosa, Neisseria spp., H. influenzae S. pneumoniae, N. gonorrhoeae, S. aureus, P. aeruginosa, Enterobacteriaceae |
| Chloramphenicol | Acetylation Lack of penetration | Enterobacteriaceae, S. aureus, streptococci, Bacteroides uniformis P. aeruginosa |
| Clindamycin, erythromycin, lincomycin | Ribosomal resistance due to methylation ofrRNA Inactivation by esterase Decreased penetration | Streptococci, E. faecalis, Enterobacteriaceae Enterobacteriaceae, S. hominis |
| Fluoroquinolones | Decreased uptake Altered target site (DNA gyrase) | Enterobacteriaceae, P. aeruginosa, staphylococci Enterobacteriaceae, P. aerugmosa |
| Lincomycin | Inactivation | S. aureus |
| Sulfonamides | Synthesis of an altered or alternative target site (dihydropteroate synthetase) aerugmosa Lack of penetration Overproduction ofPABA | Enterobacteriaceae, Neisseria spp., P. Anaerobes Neisseria, S. aureus |
| Tetracycline | Drug efflux Protection of ribosome from tetracycline Inactivation | Enterobacteriaceae, staphylococci, streptococci Streptococci, E. faecalis, Neisseria spp., Mycoplasma spp. Cryptic gene found in B. fragilis, expressed resistance in E. coli |
| Trimethoprim | Synthesis of an altered or alternative target site (dihydrofolate reductase) Lack of penetration Ability to use alternative pathway H. Overproduction of dihydrofolate reductase | Enterobacteriaceae, V. cholerae, staphylococci P. aeruginosa Enterococci influenzae |
| Vancomycin | ? ?Blocking of target site | Pediococci, Leuconostoc spp. (intrinsic) Enterococci (acquired) |

Lonan, In: Antibiotics in Laboratory Medicine, Satterfield (Ed), Williams & Wilkins, Philadelphia, pp. 558-559 (1991 ).

There is described a composition comprising a compound having affinity for microbial spore surface peptides, wherein the compound comprises an affinity for peptides derived from different species and strains of bacteria. In one embodiment, different strains of bacteria are resistant to a plurality of antibiotic drugs. In one embodiment, the compositions of the present invention comprise stable pharmaceutical formulations. In one embodiment, the formulation may further comprise a pharmaceutically acceptable carrier. It is further contemplated that formulations may be administered to humans or animals, included in food preparations, pharmaceutical preparations, medicinal and pharmaceutical products, cosmetic products, hygienic products, cleaning products and cleaning agents, as well as any material to which the compositions could be sprayed on, or adhered to, wherein inhibition of microbial spore attachment and subsequent growth on such a material is desired.

The proper applied amount of the described compositions necessary to prevent microbial attachment and subsequent growth and proliferation depends upon a number of factors including the types of bacteria that might be present, the environment into which the composition is being introduced, and the time that the composition is envisioned to remain in a given area.

It is further contemplated that the described compositions maybe used in combination with other antimicrobial agents or antibiotics to enhance their activity. Combinations of described compositions with other agents may be useful to allow antibiotics to be used at lower doses due to toxicity concerns, to enhance the activity of antibiotics whose efficacy has been reduced, or to effectuate a synergism between the components such that the combination is more effective than the sum of the efficacy of either component independently.

Antibiotics which may be combined with described compositions in combination therapy include, but are not limited to, penicillin, ampicillin, amoxycillin, vancomycin, cycloserine, bacitracin, cephalolsporin, imipenem, colistin, methicillin, streptomycin, kanamycin, tobramycin, gentamicin, tetracycline, chlortetracycline, doxycycline, chloramphenicol, lincomycin, clindamycin, erythromycin, oleandomycin, polymyxin nalidixic acid, rifamycin, rifampicin, gantrisin, trimethoprim, isoniazid, paraminosalicylic acid, and ethambutol.

There is described a method of reducing the resistance of a microorganism to an antimicrobial agent, as exemplified by reducing the resistance of a bacterium to an antibiotic, or to kill a microorganism or bacterium, by generally contacting the microorganism or bacterium with an effective amount ofthe antibiotic or antimicrobial agent in combination with an amount of a composition having affinity for a microbial spore surface peptide effective to inhibit the attachment and/or subsequent growth of the microorganism or bacterium. The terms "microorganism" and "bacterium" are used for simplicity and it will be understood that the invention is suitable for use against a population of microorganisms.

The microorganism, e.g., bacterium, or population thereof, may be contacted either *in vitro* or *in vivo*. Contacting *in vivo* may be achieved by administering to an animal (including a human patient) that has, or is suspected to have been contaminated with a microbe or bacteria, a therapeutically effective amount of pharmacologically acceptable formulation comprising a composition having a compound having affinity for a microbial spore surface peptide alone, or in combination with, a therapeutic amount of a pharmacologically acceptable formulation of an antibiotic agent. The invention may thus be employed to treat both systemic and localized microbial and bacterial infections by introducing the combination of agents into the general circulation or by applying the combination, e.g., topically to a specific site, such as a wound or bum, or to the eye, ear or other site of infection.

Where a composition of the present invention is used in combination with other antimicrobial agents or antibiotics, an "effective amount of an antimicrobial agent or antibiotic" means an amount, or dose, within the range normally given or prescribed. Such ranges are well established in routine clinical practice and will thus be known to those of skill in the art. Appropriate doses and treatment regimens are further detailed herein. See, Table XIV. Naturally, in confirming the optimal therapeutic dose for compounds having affinity for microbial spore surface peptides, first animal studies and then clinical trials would be conducted, as is routinely practiced in the art. See, Example II.

**Table XIV: Common Antibiotics And Usual Oral Doses Antibiotic Dosage**

| Antibiotic | Dosage |
|---|---|
| Penicillin V | 2 |
| Rugby (generic) | 50 mg qid |
| V-cillin K | |
| Dicloxacillin Glenlawn (generic) Dynapen Cloxacillin (Tegopen) Amoxicillin Rugby (generic) Polyrnox Ampicillin Moore (generic) Polycillin | 250 mg qid |
| Augmentin | tid |
| | 250-mg tablets |
| | chewables (250 mg) |
| | 125-mg (suspension) |
| | chewables (125 mg) |
| Carbenicillin (Geocillin) | 382 mg qid (1 tb) 2 tabs qid |
| Cephalexin Rugby (generic) Keflex | 250 mg qid |
| Rugby (generic) Keflex | 500 mg qid |
| Cefadroxil Rugby (generic) Duricef | 1 gm bid |
| Cephradine 250 mg qid | |
| Rugby (generic) Velosef Rugby (generic) | 500 mg qid |
| Cefaclor Ceclor | 250 mg tid |
| Cefuroxime axetil | 125 mgbid |
| Ceftin | 250mgbid |
| | 500 mgbid |
| Cefixime Suprax | 400 mg q24h |
| Cefprozil Cefzil | 250 mg q12h |
| Loracarbef(Lorabid) | 200 mgbid |
| Cefpodoxime proxetil (Vantin) | 200 mgbid |
| Clindamycin Cleocin | 300 mgq8h |
| TMP/SMZ Bactrim Septra (generic) | 1 double-strength bid |
| Trimethoprim Rugby (generic) Proloprim | 100 mgbid |
| Erythromycin (base) Abbott E-mycin (delayed release) | 250 mg qid |
| Erythromycin stearate Rugby (generic) | 250 mg qid |
| Azithromycin Zithromax | 1 g once only; 500 mg day 1 plus 250 mg day 2- 5 |
| Clarithromycin | 250 mgbid |
| Biaxin | 500 mgbid |
| Tetracycline hydrochloride Mylan Sumycin 250 | 250 mg qid |
| Doxycycline Lederle (generic) Vibramycin | 100 mg qd (with 200- mg initial load) |
| Vancomycin Vancocin HCI | Capsules (oral 125 mg q6h PO soln/powder) |
| Metronidazole Rugby (generic) Flagyl | 250 mg qid |
| Norfloxacin Noroxin | 400 mgbid |
| Ciprofloxacin | 250 mgbid |
| Cipro | 500 mgbid |
| | 750 mg bid |
| Ofloxacin 200 mgbid | |
| Floxin | 300 mgbid |
| | 400 mg bid |
| Lomefloxacin Maxaquin | 400 mg once qid |

qid (4 times daily), tid (3 times daily), bid (twice daily), qd (once daily), q4h (every 4 hours around the clock), q6h (every 6 hours around the clock) and q8h (every 8 hours around the clock).

The LD₅₀/ED₅₀ ratio required for safe use of the proposed compositions and/or combinations of the compositions with other antimicrobial agents are assessed by determining the LD₅₀ (median lethal toxic dosage) and the ED₅₀ (median effective therapeutic dosage) in experimental animals. The optimal dose for human subjects is then defined by fine-tuning the range in clinical trials. In the case of LD₅₀, an inhibitor is usually administered to mice or rats (orally or intraperitoneal) at several doses (usually 4- 5) in the lethal range. The dose in mg/kg is plotted against % mortality and the dose at 50% represents the LD₅₀. The ED₅₀ is determined in a similar fashion.

In a clinical trial, the therapeutic dose would be determined by maximizing the benefit to the patient, while minimizing any side-effects or associated toxicities. In optimizing a therapeutic dose within the ranges disclosed herein, one would not use the upper limit of the range as the starting point in a clinical trial due to patient heterogeneity. Starting with a lower or mid-range dose level, and then increasing the dose will limit the possibility of eliciting a toxic or untoward reaction in any given patient or subset of patients. The presence of some side-effects or certain toxic reactions per se would not, of course, limit the utility of the invention, as it is well known that most beneficial drugs also produce a limited amount of undesirable effects in certain patients.

In the treatment of animals or human patients with combination therapy, there are various appropriate formulations and treatment regimens that may be used. For example, a composition comprising a compound having affinity for a spore surface peptide and at least one additional antimicrobial drug may be administered to an animal simultaneously, e.g., in the form of a single formulation that includes the compound and additional drug(s), or by using at least two distinct formulations. The compounds having affinity for a spore surface peptide could also be administered to an animal prior to the additional drug(s) or *vice versa.*

Further embodiments include therapeutic kits that comprise, in suitable container means, a pharmaceutical composition of at least one compound having affinity for a spore surface peptide and at least one additional antimicrobial drug and/or antibiotic. The compound, antimicrobial agent and/or antibiotic may be contained within a single container means, or a plurality of distinct containers may be employed.

Depending on the circumstances, antimicrobial agents may be employed in oral or parenteral treatment regimens. Methods to achieve appropriate doses are described in various publications. Reese and Betts, In: A Practical Approach to Infectious Diseases, (3rd ed.), Boston, Little Brown, (1993).

### XI. Antibacterial Cleanser Compositions

Antibacterial cleansing compositions are typically used to cleanse the arms and hands of the user and to destroy bacteria and any other microorganisms which might be present on the user's arms or hands. These compositions are widely used in the health care industry by hospital staff and other health care personnel as hand washing cleansers to prevent infection ether between staff personnel or between staff personnel and patients. They are particularly suitable for use by heathcare worker such as surgeons, nurses and other health care professionals who might be subject to contact with bacteria and the like. They are also suitable for use by personnel in the food service and meat processing industries and, generally are used for antimicrobial cleansing of the hands and arms by the public.

There are several active antimicrobial agents currently available for use in cleansing compositions. For example, many antimicrobial cleansing compositions contain a bisbiguanide bacterial substance such as chlorhexidine digluconate (CHG). Other cleansing compositions use phenolic compounds. The antimicrobial activity of these substances, however, are often dependent upon the type(s) of surfactants or other ingredients employed therewith, and therefore, the use of the same ingredients with these antimicrobial substances will not necessary derive the same result. For instance, when CHG is employed as an active antimicrobial agent, the cleansing composition may include, but not be limited to, alkyl polyglucosides and nonionic alcohol ethoxylates as the primary surfactants. However, in compositions containing substituted phenols such as para-chloro-meta-xylenol (PCMX; 4-chloro-3,5-dimethyl phenol) as the active antimicrobial agent, most non-ionic alcohol ethoxylates inhibit, rather than enhance, PCMX antibacterial activity. PCMX may yield high initial reductions of Gram-positive and Gram-negative bacteria as well as fungi, and provide good residual activity for several hours between hand washings. Typically, where PCMX is used in cleansing compositions, it is used in concentrations of from about 0.1 to 4 percent by weight. However, the majority of formulas contain 1 percent or less by weight.

Antimicrobial cleansing compositions containing substituted phenols as the active antimicrobial agent have also included anionic detergents, soaps, surfactants, and the like, as well as other compounds (non-ionic ethoxylated surfactants, polyethylene glycol etc.) which may reduce the antibacterial activity of the phenolic compounds. Substituted phenolic compounds and anionic surface active detergents or soaps in an alcohol and water-based carrier are also useful. Additionally, chelating agents such as ethylenediaminetetraacetic acid (hereinafter, EDTA) may be added. Winicov et al.., U.S. Pat. No. 3,824,190 & RE 28,778.

Antimicrobial compositions exhibiting mildness characteristics may include, but are not limited to, a substituted phenol, namely PCMX, an anionic detergent, a thickener such as ethylene glycol monostearate, and a foam builder such as a fatty acid alkanol amide. The thickener and fatty acid alkanol amides as well as the anionic surfactant are suspected to hinder antibacterial activity of a phenolic compound. Garabedian et al. U.S. Pat. No. 4,632,772 (herein incorporated by reference). Other PCMX compositions comprise anhydrous blends in surfactant mixtures. Corti et aI., U.S. Pat. No. 5, 114, 978.

PCMX may be solubilized in high concentrations of water or aqueous compositions. PCMX compositions include, but are not limited to, diethanolamides of fatty acids and anionic surfactants of the class diethanolammonium salts of alkylpolyoxyethylsulfuric acid.

Alcohol-based antimicrobial compositions may include, but are not limited to, PCMX or CHG, hydropropyl cellulose, an alcohol, an emollient, and water. Although it is not necessary to understand the mechanism of an invention, it is believed that the use of alcohol as a solvent or carrier in the composition is known to defat the skin which may lead to skin dryness and irritation. White et al., U.S. Pat. No. 5,288,486.

Consequently, PCMX is similar to that ofCHG and other substituted phenols against microorganisms in that their efficacy is highly dependent upon both the carrier and other chemical constituents within the antimicrobial cleanser composition. While the antimicrobial activity of PCMX maybe potentiated against *Pseudomonas aeruginosa* by the addition of the chelating agent ethylenediaminetetraacetic acid (EDTA) and/or the sequestering agent sodium hexametaphosphate, PCMX may be reduced in the presence of some organic materials, and/or moderate concentrations of anionic surfactants. Antimicrobial activity may also be lost as a consequence of reversible interactions between PCMX and noninonic surfactants, polyethylene glycol, and polyethylene glycol stearate. Similarly, reduction in the antibacterial efficacy ofPCMX has also been found to be the direct result of its interaction with a variety of macromolecules, namely, methyl cellulose, polyethylene glycol 6000, and polysorbate 80.

The present invention is not limited to standard antibiotic cleanser compositions such as PCMX and CGH. In one embodiment, the present invention contemplates an antibacterial composition containing a substituted phenol which does not include conventional anionic surfactants or other chemical ingredients detrimental to substituted phenol antimicrobial activity. In one embodiment, an antimicrobial cleansing composition comprises a substituted phenol and at least one primary surfactant. In one embodiment, the composition comprises a substituted phenol in amounts ranging from about 0.1 to about 4 percent by weight of the total composition, and preferably in amounts ranging from about 0.3 to 1 percent by weight of the total composition. In one embodiment, the composition comprises a substituted phenol in amounts that are more than 4 percent by weight of the antimicrobial agent. Although it is not necessary to understand the mechanism of an invention, it is believed that the upper limit of the antimicrobial agent amount is related to the amount of substituted phenol which is considered to be non-toxic and non-irritating to the skin.

Any substituted phenol which is soluble in water or other non-alkanol solvent (i.e., for example, triethylene glycol, propylene glycol, hexylene glycol etc.) may be used in the compositions of the present invention. In one embodiment, a substituted phenol may include, but is not limited to, alkyl substituted, halogen substituted, phenol substituted, alkyl halo substituted, benzyl alkyl substituted, phenylethyl alkyl substituted and benzyl halo substituted phenols, and alkyl substituted, halogen substituted and alkyl halo substituted bisphenols, bis(hydroxyphenyl) alkanes, amino substituted phenols, resorcinol derivatives, trihydric phenol derivatives, naphthol derivatives and carboxylic acid substituted phenols (e.g., salicylic acid and esters). In one embodiment, a substituted phenol comprises a chIoro-substituted phenol, namely para-chloro-meta-xylenol (PCMX). Although it is not necessary to understand the mechanism of an invention, it is believed that PCMX may have 60 times the antimicrobial activity of an unsubstituted phenol against a wide spectrum ofbacteria and is also considered to be non-toxic and nonirritating to human skin at levels below about 3 percent by weight. Fendler, et al., "Antimicrobial cleansing compositions" *United States Patent* 5,635,462.

In one embodiment, an infection control cleanser composition comprises at least one primary surfactant selected from the group including, but not limited to, amine oxides, phospholipids, partially neutralized carboxylic acids and diacids, betaines, or ethoxylated methylglucosides, and/or mixtures thereof. Although it is not necessary to understand the mechanism of an invention, it is believed that the amount of primary surfactant(s) to be added to the composition is somewhat dependent upon the number ofprimary surfactants added. In one embodiment, the composition comprises a plurality of primary surfactants wherein the combined amount of the primary surfactants is less than about 20 percent by weight of the composition.

In one embodiment, the composition comprises a primary surfactant including, but not limited to, an amine oxide or an alkyl amine oxide. In one embodiment, an amine oxide is added to the composition in an amount ranging from about 0 (absent) to about 10 percent by weight but more preferably in an amount ranging from about 5 to 7 percent by weight. In one embodiment, a composition comprises an amine oxide as a sole primary surfactant wherein the amine oxide added in amounts ranging from about 1 to about 10 percent by weight. In one embodiment, an amine oxide has a alkyl chain length of from about C₈ to about C₁₆, with chain lengths offrom C₁₂ to C₁₄.

Although it is not necessary to understand the mechanism of an invention, it is believed that amine oxides contemplated by the present invention are considered to be amphoteric surfactants. Examples of amine oxides found to be suitable for the present invention include, but are not limited to, lauramine oxide a C₁₂ alkyl amine oxide (Mackamine LO®) and cocamine oxide a C₁₂ -C₁₄ alkyl amine oxide (Mackamine CO®)(McIntyre Chemical Co., Ltd., Chicago, Ill.), as well as a C14 alkyl amine oxide(Barlox 14®) and a C₁₂ alkyl amine oxide(Barlox 12®)( Lonza, Inc., Fair Lawn, N.J). Alternatively, the compositions contemplated herein are suitable for amidopropyl amine oxides including, but not limited to Standamox LAO® or Mackamine CAO® (Henkel Corp., Hoboken, N.J.).

In addition, alkylamides such as cocamide may be used in conjunction with the amine oxides or other primary surfactants to boost foaming and add to the viscosity of the composition. Viscosity modifiers and/or foam stabilizers may include, but are not limited to, Standamide CD®, Standamide SD®, and Cocamide DEA®(Henkel Corp., Hoboken, N.J.).

Although it is not necessary to understand the mechanism of an invention, it is believed that amine oxides do not significantly reduce the antibacterial activity of a substituted phenol and have been found to be effective in combination with PCMX for killing microorganisms..

In one embodiment, there is described an infection control composition comprising zwitterionic/amphoteric surfactants such as phospholipids and betaines as a primary surfactant. In one embodiment, phospholipids and/or betaines may be used in place of, or in conjunction with, amine oxides. In one embodiment, a composition may comprise phospholipids and betaines in amounts up to about 10 percent by weight.

In one embodiment, an infection control cleanser composition comprises a phospholipid including, but not limited to, an alkyl phosphatidyl PG-dimonium chloride. In one embodiment, an alkyl phosphatidyl PG-dimonium chloride may be selected from the group comprising cocamidopropyl phosphatidyl PG-dimonium chloride (Phospholipid PTC®, MONA Industries, Inc., Paterson, N.J.); stearamidopropyl phosphatidyl PGdimonium chloride (Phospholipid PTS®, MONA Industries, Inc., Paterson, N.J.); linoleamidopropyl phosphatidyl PG-dimonium chloride (Phospholipid EFA®, MONA Industries, Inc., Paterson, N.J.); and stearamidopropyl phosphatidyl PG-dimonium chloride/cetyl alcohol (Phospholipid SV®, MONA Industries, Inc., Paterson, N.J.). In one embodiment, alkyl phosphatidyl PG-dimonium chlorides are incorporated into the composition in an amount ranging from 0 (absent) to about 10 percent by weight of the total composition.

In one embodiment, there is described an infection control cleanser composition comprising a betaine lipid as a primary surfactant either alone or in conjunction with other primary surfactants. In one embodiment, a betaine lipid comprises an alkylammonio carboxylate ranging from 8 to 18 carbon atoms. In one embodiment, a composition comprising an alkylammonio carboxylate includes up to 10 percent by weight of the total composition. In one embodiment, an alkylammonio carboxylate comprises cocobetaine (Mackam CB-35®, Mcintyre Chemical Co., Chicago, Ill.).

In one embodiment, the there is described an infection control cleanser composition comprising a mixture of at least one partially neutralized carboxylic acids and/or diacids (i.e., for example, sodium caproyl 1 actylate) that may be employed as a primary surfactant. In one embodiment, a carboxylic acid and/or diacid is added to the cleansing composition in amounts ranging from 0 (absent) to about 5 percent by weight. Although it is not necessary to understand the mechanism of an invention, it is believed that although partially neutralized carboxylic acids and diacids might contain some anionic surfactants they have considerable protonated acid character and, therefore, these compounds are expressly excluded from the term "conventional anionic surfactants".

In one embodiment, there is desribed an infection control cleanser composition comprising a nonionic primary surfactant. In one embodiment, the nonionic primary surfactant comprises an ethoxylated nonionic methylglucoside. In one embodiment, an ethoxylated nonionic methylglycoside may also be added to a composition in amounts ranging from 0 (absent) to about 10 percent by weight.

The composition may also include other additives such as thickeners, emollients, chelating and sequestering agents, fragrances, coloring agents, opacifying agents, pearlizing agents, vitamins and the like. For example, a composition may include a polymer viscosifier or thickener such as hydroxyethyl cellulose to make the composition more aesthetically pleasing. Examples of other suitable polYmer viscosifiers include, but are not necessarily limited to, hydroxypropyl cellulose, methyl cellulose, and carboxymethyl cellulose.

A glycol may be added in order to quicken the rate of dissolution of the substituted phenol and as an emollient and/or humectant. Glycols may include, but are not limited to, propylene glycol, triethylene glycol, and hexylene glycol. Glycols may be added to a composition in amounts ranging from 0 (absent) to about 10 percent by weight of the total composition, but more preferably in an amount of about 3 percent. In one embodiment, a PCMX and/or a second substituted phenol may be dissolved in at least a portion of a glycol prior to adding other ingredients to the solution. In one embodiment, a composition comprises a 25 percent/75 percent ratio of PCMX:glycol.

In one embodiment, the present invention contemplates an infection control cleanser composition comprising a chelating and/or sequestering agent ranging up to about 1 percent by weight. Although it is not necessary to understand the mechanism of an invention, it is believed that a chelating agent and/or sequestering agent may be added to soften the water-based composition. In one embodiment, a suitable chelating agent for the present invention comprises ethylenediaminetetra-acetic acid (EDTA) (i.e., for example, disodium EDTA).

The compositions may further comprise minor but effective amounts of other conventional additives such as fragrances, color additives, opacifying agents, pearlizing agents, vitamins, etc. An example of a particular pearlizing agent includes, but is not necessarily limited to, ethylene glycol distearate. Generally, these additives are used in amounts which do not affect the essential nature of the composition with respect to its infection control properties.

For optimal antibacterial efficacy, the pH ofthe composition should be between 4 and 8, and preferably between 5.5 and 6.5. To adjust the pH of the composition, any acid compatible with the components of the composition can be used. Acids may include, but are not limited to, lactic acid, citric acid, acetic acid, glycolic acid, or gluconic acid. Typically, less than 1 percent by weight of these acids are used to achieve the proper pH balance.

The balance of the composition is typically water so as to provide 100 percent by weight of the composition but other water soluble compounds are also suitable (i.e., for example, alcohols).

All percents by weight indicated herein are based upon the percent active composition. Thus, for example, where 5 percent by weight lauramine oxide is employed and the lauramine oxide is obtained from the manufacturer or has been diluted into solution so as to comprise a 30 percent active solution, 16.7 percent of the solution will have to be used in order to obtain the 5 percent by weight recommended.

The infection control cleansing compositions are generally prepared by dissolving substituted phenol, namely PCMX, in glycol as noted hereinabove, and adding this solution to one or more of the primary surfactant discussed hereinabove, in water. More particularly, the primary surfactant or surfactants, and other ingredients (i.e., for example, fragrance, chelating agent, pearlizing agent, etc.) are added to the solution with stirring. The pH ofthe composition is adjusted with lactic acid or similar acid. The thickener/viscosifier is then preferably added and the solution is mixed until it is completely homogeneous. The pH is checked and adjusted again if necessary. This process may be employed with or without the application of heat to enhance hydration of the viscosifier, if required.

### XII. Detecting Microbes Comprising Spore Surface Protein Nucleotide Sequences

There are disclosed methods for the detection of microbes which contain a spore surface protein nucleotide sequence (i.e., for example, SEQ ID NO: 2) and/or a spore surface protein nucleotide sequence (i.e., for example, SEQ ID NO's: 4, 6, or 8). Microbes which contain nucleotide sequences may be identified by variety of procedures. These procedures include, but are not limited to, DNA--DNA or DNA-RNA hybridization as well as amplification (e.g., PCR) using DNA probes (e.g., oligonucleotide or oligomer probes or amplimers), mRNA probes and fragments ofthe sequence of interest. These probes and fragments can be made using a wide vmiety of techniques including, but not limited to, chemical synthesis, restriction digestion and expression of the nucleotide sequence, or any portion of it, in an expression vector. Labeling of synthesized or expressed probes and nucleotide sequence fragments can be achieved using oligolabeling, end-labeling or PCR amplification using labeled nucleotide.

Having generated labeled probes, microbes present in a biological sample can be tested for the presence of a nucleotide sequence using Southern or reverse Northern analysis of isolated plasmid total cellular DNA, or using Northern analysis ofmRNA. Microbes can be either grown on filters or spotted onto filters either directly or following overnight culture. Moseley et aI., J Infect. Dis. 142:892-898 (1980); Perine et al., J Infect. Diseases 152(1):59-63 (1985); and Gootz et al.., Antimicrob. Agents and Chemother. 28(1):69-73 (1985). Briefly, a solid support such as nitrocellulose paper or a nylon membrane is inoculated with clinical specimen, or with broth culture of clinical specimen suspected of containing bacteria. The cells lysed onto the nitrocellulose paper and the DNA denatured, for example by treatment with NaOH. The support is treated with pronase and chloroform to lower background non-specific binding ofDNA probes to the colony material. Prehybridization and hybridization of the filters (i.e., support) with oligonucleotides, oligomers, or portions of a nucleotide sequence is then performed using standard techniques. Hybridization is detected using anyone ofmany available methods, such as by imaging radioactive probes using X-ray films (i.e., autoradiography).

Detection of bacteria which harbor a microbial spore surface protein nucleotide sequence in a sample derived from a surface demonstrates that it is beneficial to administer infection control compositions which alter microbial spore surface protein binding to the surface. In addition, such detection also permits monitoring the surface for the presence of the bacteria during and after administration of the infection control composition.

In one embodiment, screening methods (i.e., for example, an assay) contemplated by this invention can be performed in solid phase where one or more components of the assay is attached to a solid surface. In solid phase assays, one or more components of the assay is attached to a solid surface. Virtually any solid surface is suitable, as long as the surface material is compatible with the assay reagents and it is possible to attach the component to the surface without unduly altering the reactivity ofthe assay components. Some components might show reduced activity in solid phase, but this is generally acceptable so long as the activity is sufficient to interact with an infection control test compound.

Solid supports include, essentially any solid surface, including, but not limited to, a glass bead, planar glass, controlled pore glass, plastic, porous plastic metal, or resin to which the molecule may be adhered. Solid supports may be derivatized with functional groups (e.g., hydroxyls, amines, carboxyls, esters, and sulfhydryls) to provide reactive sites for the attachment oflinkers or the direct attachment ofthe component(s).

Adhesion of an assay component (e.g., a spore surface protein) to the solid support can be direct or indirect (i.e., a particular compound or compounds are bound to the support, and an assay component binds to this compound or compounds rather than to the solid support). The component can be immobilized either: i) covalently (i.e., for example, by utilizing single reactive thiol groups of cysteine for anchoring protein components; Colliuod et aI., Bioconjugate Chem. 4:528-536 (1993)); ii) non-covalently but specifically (i.e., for example, via immobilized antibodies or other specific binding proteins; Schuhmann et aI. Adv. Mater. 3:388-391 (1991); and Lu et aI. Anal. Chem. 67:83-87 (1995); iii) by the biotin/streptavidin system; Iwane et aI., Biophys. Biochem. Res. Comm. 230:76-80 (1997); iv) by using metal-chelating Langmuir-Blodgett films; Ng et aI. Langmuir 11 :4048-4055 (1995); and Schmitt et aI., Angew. Chem. Int. Ed. Engl. 35: 317320 (1996); and v) metal-chelating self-assembled monolayers for binding of polyhistidine fusion proteins; Sigal et aI., Analytical Chem. 68:490-497 (1996).

### XIV. Detecting Microbes Comprising Spore Surface Proteins

In one embodiment, there are disclsoed methods comprising detecting a microbial spore protein and/or peptide fragment. In one embodiment, the microbial spore protein and/or peptide fragment comprises a microbial spore surface protein and/or peptide fragment. In one embodiment, the microbial spore surface protein and/or peptide fragment comprises a *Clostridium* spore surface protein and/or peptide fragment. In one embodiment, the *Clostridium* spore surface protein and/or peptide fragment comprises a *Clostridium difficile* spore surface protein and/or peptide fragment.

Proteins and/or peptide fragments, from any source, may be detected by a variety of suitable methods. In some embodiments, proteins and/or peptide fragments are detected by immunohistochemistry. In other embodiments, proteins and/or peptide fragments are detected by their binding to an antibody raised against the protein.

Antibody binding may be detected by many different techniques including, but not limited to, (e.g., radioimmunoassay, ELISA (enzyme-linked immunosorbant assay), "sandwich" immunoassays, immunoradiometric assays, gel diffusion precipitation reactions, immunodiffusion assays, in situ immunoassays (e.g., using colloidal gold, enzyme or radioisotope labels, for example), Western blots, precipitation reactions, agglutination assays (e.g., gel agglutination assays, hemagglutination assays, etc.), complement fixation assays, immunofluorescence assays, protein A assays, and immunoelectrophoresis assays, etc.

In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled.

In some embodiments, an automated detection assay is utilized. Methods for the automation of immunoassays include those described in U.S. Pat. Nos. 5,885,530, 4,981,785, 6,159,750, and 5,358,691. In some embodiments, the analysis and presentation of results is also automated. For example, in some embodiments, software that generates a prognosis based on the presence or absence of a series of proteins corresponding to cancer markers is utilized.

In other embodiments, the immunoassay described in U.S. Pat. Nos. 5,599,677 and 5,672,480.

In other embodiments, there are provided kits for the detection and characterization of proteins and/or nucleic acids. In some embodiments, the kits contain antibodies specific for a protein expressed from a gene of interest, in addition to detection reagents and buffers. In other embodiments, the kits contain reagents specific for the detection ofmRNA or cDNA (e.g., oligonucleotide probes or primers). In preferred embodiments, the kits contain all of the components necessary to perform a detection assay, including all controls, directions for performing assays, and any necessary software for analysis and presentation of results.

### XV. Pharmaceutical Formulations

The active compositions may include traditional pharmaceutical formulations. Administration of these formulations according to the present invention will be via any common route so long as the target tissue is available via that route. This includes topical, oral, nasal, buccal, rectal, vaginal or topical. Alternatively, administration may be by orthotopic, intradermal, subcutaneous, intramuscular, intraperitoneal or intravenous injection. Such formulations would normally be administered as a pharmaceutically acceptable composition.

### A. Solutions For Oral Or Parenteral Administration

The formulations, compositions, and other agents and drugs may be active compounds that may also be administered parenterally or orally. Solutions of active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations may contain a preservative.

Pharmaceutical formulations suitable for injectable use include, but are not limited to, sterile aqueous solutions or dispersions and sterile powders for the extemporaneous preparation of sterile injectable solutions or dispersions. In one embodiment, a formulation is sterile. In one embodiment, the formulation comprises fluid characteristics such that the formulation is easily manipulated using a syringe. In one embodiment, the formulation is stable under manufacture and/or storage conditions. In one embodiment, the formulation may further comprise a carrier such as a solvent or dispersion medium including, but not limited to, water, ethanol, polyol (for example, glycerol, propylene glycol, and liquid polyethylene glycol, and the like), suitable mixtures thereof, and vegetable oils. The proper fluidity can be maintained, for example, by use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions may be prepared by incorporating the active compounds in the required amount in the appropriate solvent with various ofthe other ingredients enumerated above, as required, followed by sterilization (i.e., for example, by filtration, radiation, and/or ethylene oxide exposure). Generally, dispersions may be prepared by incorporating the various sterilized active ingredients into a sterile vehicle which contains the basic dispersion medium and the required other ingredients from those enumerated above. In the case of sterile powders for the preparation of sterile injectable solutions, the preferred methods ofpreparation are vacuum-drying and freeze-drying techniques which yield a powder of the active ingredient plus any additional desired ingredient from a previously sterile-filtered solution thereof.

For oral administration, compositions having affinity for microbial spore surface proteins may be incorporated with excipients and used in the form of non-ingestible mouthwashes and dentifrices. A mouthwash may be prepared incorporating the active ingredient in the required amount in an appropriate solvent, such as a sodium borate solution (i.e., for example, Dobell's Solution). Alternatively, the active ingredient may be incorporated into an antiseptic wash containing sodium borate, glycerin and potassium bicarbonate. The active ingredient may also be dispersed in dentifrices, including: gels, pastes, powders and slurries. The active ingredient maybe added in a therapeutically effective amount to a paste dentifrice that may include water, binders, abrasives, flavoring agents, foaming agents, and humectants.

The compositions may be formulated in a neutral or salt form. Pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Upon formulation, solutions will be administered in a manner compatible with the dosage formulation and in such amount as is therapeutically effective. The formulations are easily administered in a variety of dosage forms such as injectable solutions, drug release capsules and the like. Routes of administration may be selected from intravenous, intrarterial, intrabuccal, intraperitoneal, intramuscular, subcutaneous, oral, topical, rectal, vaginal, nasal and intraocular.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the ali in light of the present disclosure. For example, one dosage could be dissolved in 1 ml of isotonic NaCI solution and either added to 1000 ml of hypodermoclysis fluid or injected at the proposed site of infusion. In: Remington's Pharmaceutical Sciences 15th Edition, pages 1035-1038 and 1570-1580. Some variation in dosage will necessarily occur depending on the condition ofthe subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### B. Liposomes For Topical Or Parenteral Administration

In one embodiment, the present invention contemplates a formulation comprising a liposomal preparation comprising a compound having affinity for a microbial spore surface protein. In one embodiment, the compound is encapsulated by a liposome, wherein the encapsulation prolongs the compound half-life when compared to conventional drug delivery systems.

A "liposome" is generally used as a generic term encompassing a variety of single and multilamellar lipid vehicles formed by the generation of enclosed lipid bilayers. For example, phospholipids may be used for preparing liposomes and can carry a net positive charge, a net negative charge, or are neutral. Dicetyl phosphate can be employed to confer a negative charge on the liposomes, and stearylamine can be used to confer a positive charge on the liposomes. Liposomes may be characterized by a phospholipid bilayer membrane and an inner aqueous medium. Multilamellar liposomes have multiple lipid layers separated by aqueous medium. Liposomes usually form spontaneously when phospholipids are suspended in an excess of aqueous solution. Although it is not necessary to understand the mechanism of an invention, it is believed that the lipid components undergo self-rearrangement before the formation of closed structures and entrap water and dissolved solutes between the lipid bilayers. Ghosh et aI, In: Liver diseases, targeted diagnosis and therapy using specific receptors and ligands, Wu G. and C. Wu (eds.), New York: Marcel Dekker, pp. 87-104, (1991). Also contemplated are cationic lipid-nucleic acid complexes, such as lipofectamine-nucleic acid complexes. In certain embodiments of the invention, the liposome may be complexed with a hemagglutinating virus (HVJ). HVJ has been shown to facilitate fusion with the cell membrane and promote cell entry. Kaneda et aI., J Biol Chem,. 264: 12126-12129 (1989).

Lipids suitable for making liposomes can be obtained from commercial sources. For example, dimyristyl phosphatidylcholine ("DMPC") can be obtained from Sigma Chemical Co., dicetyl phosphate ("DCP") is obtained from K & K Laboratories (Plainview, N.Y.); cholesterol ("Chol") is obtained from Calbiochem-Behring; dimyristyl phosphatidylglycerol ("DMPG") and other lipids may be obtained from Avanti Polar Lipids, Inc. (Birmingham, Ala.). Stock solutions of lipids in chloroform, chloroform/methanol or t-butanol can be stored at about -20°C. Preferably, chloroform is used as the only solvent since it is more readily evaporated than methanol.

Phospholipids from natural sources, such as egg or soybean phosphatidylcholine, brain phosphatidic acid, brain or plant phosphatidylinositol, heart cardiolipin and plant or bacterial phosphatidylethanolamine are preferably not used as the primary phosphatide, i.e., constituting 50% or more of the total phosphatide composition, because of the instability and leakiness of the resulting liposomes.

Liposomes can be made by different methods. The size of the liposomes varies depending on the method of synthesis. A liposome suspended in an aqueous solution is generally in the shape of a spherical vesicle, having one or more concentric layers of lipid bilayer molecules. Each layer consists of a parallel array of molecules represented by the formula XY, wherein X is a hydrophilic moiety and Y is a hydrophobic moiety. In aqueous suspension, the concentric layers are arranged such that the hydrophilic moieties tend to remain in contact with an aqueous phase and the hydrophobic regions tend to self-associate. For example, when aqueous phases are present both within and without the liposome, the lipid molecules will form a bilayer, known as a lamella, of the arrangement XY-YX.

Liposomes can be prepared in accordance with many laboratory techniques. In one embodiment, liposomes are prepared by mixing liposomallipids, in a solvent in a container, e.g., a glass, pear-shaped flask. The container should have a volume ten-times greater than the volume of the expected suspension ofliposomes. Using a rotary evaporator, the solvent is removed at approximately 40°C and under negative pressure. The solvent normally is removed within about 5 min to 2 hours, depending on the desired volume of the liposomes. The composition can be dried further in a desiccator under vacuum. The dried lipids generally are discarded after about 1 week because of a tendency to deteriorate with time.

Dried lipids can be hydrated at approximately 25-50 mM phospholipid in sterile, pyrogen-free water by shaking until all the lipid film is resuspended. The aqueous liposomes can be then separated into aliquots, each placed in a vial, lyophilized and sealed under vacuum.

In an alternative method, liposomes can be prepared in accordance with other procedures. Bangham et al., J Mol. Biol., 13:238-252, (1965); Gregoriadis (Ed), In. Drug Carriers in Biology and Medicine, pp 287-341 (1979); Deamer et al., "Liposome Preparation: Methods and Mechanisms," In. Liposomes, M. Ostro (Ed.) (1983); and Szoka et al., Proc. Natl Acad. Sci. USA, 75:4194-4198 (1978). The aforementioned methods may differ in their respective abilities to entrap aqueous material and their respective aqueous space-to-lipid ratios.

The dried lipids or lyophilized liposomes prepared as described above may be reconstituted in a solution of nucleic acid and diluted to an appropriate concentration with an suitable solvent, e.g., DPBS. The mixture is then vigorously shaken in a vortex mixer. Unencapsulated nucleic acid is removed by centrifugation at 29,000xg and the liposomal pellets washed. The washed liposomes are resuspended at an appropriate total phospholipid concentration, e.g., about 50-200 mM. The amount of nucleic acid encapsulated can be determined in accordance with standard methods. After determination of the amount of nucleic acid encapsulated in the liposome preparation, the liposomes may be diluted to appropriate concentration and stored at 4°C until use.

In one embodiment, a lipid dioleoylphosphatidylchoine is used to create a liposome formulation. In one embodiment, compositions having affinity for microbial spore surface peptides are mixed with lipids in the presence of excess t-butanol. The mixture was vortexed before being frozen in an acetone/dry ice bath. The frozen mixture was lyophilized and hydrated with HEPES-buffered saline (1 mM HEPES, 10 mM NaCl, pH 7.5) overnight, and then the liposomes were sonicated in a bath type sonicator for 10 to 15 min. The size of the peptide-liposomes typically range between 200-300 nm in diameter as determined by the submicron particle sizer autodilute model 370. (Nicomp, Santa Barbara, Calif).

Purified compositions comprising an infection control composition having affinity for a spore surface peptide maybe used without further modifications or it may be diluted in a pharmaceutically acceptable carrier. The infection control composition may be used independently or in combination with other antimicrobial drugs. Because of the stability of the compositions it is contemplated that the invention may be administered to humans or animals, included in food preparations, pharmaceutical preparations, medicinal and pharmaceutical products, cosmetic products, hygienic products, cleaning products and cleaning agents, as well as any material to which the peptides could be sprayed on or adhered to wherein the inhibition of microbial growth is desired.

### Experimental

The following examples are included to demonstrate preferred embodiments of the invention. These examples are intended to represent techniques that are capable of practicing specific embodiments of the present invention. However, many changes can be made in the specific embodiments which are disclosed and still obtain a like or similar result without departing from the scope of the invention as defined by the appended claims.

### Example I

### Spore Surface Peptide Synthesis

Spore surface peptides described may be synthesized by the solid-phase method employing an Applied Biosystems model 433A peptide synthesizer and Fastmoc strategy at the 0.1 mM scale. Peptide purification may be performed by reversed-phase HPLC on a Waters Delta Prep employing a Vydac 218TPI022 (22 x 250 mm) column. Separation is performed with a gradient system of aqueous 0.1% trifluoroacetic acid (solvent A) and 100% acetonitrile containing 0.085% trifuoroacetic acid (solvent B). A linear gradient from 0 to 100% B is applied over 70 min and fractions collected every 0.2 min. Fractions are subsequently monitored by analytical scale reversed-phase HPLC on a Beckman Gold System using a Vydac 218TP54 (4.6x250 mm) column at a flow rate of 0.5 ml/min under isocratic elution conditions. Select fractions are pooled and lyophilized; further characterization of peptides are provided by mass spectrometry and capillary electrophoresis. Mass measurements are performed by flow injection at 0.1 ml/min in 64% acetonitrile containing 0.05% trifluoroacetic acid with a Hewlett-Packard model 1100 MSD equipped with an electrospray ionization source.

Capillary electrophoresis is performed on a Hewlett-Packard 3D instrument equipped with an extended light path fused-silicate column 75 micrometers (ID) x 80.5 centimeters (total length). Capillary electrophoresis experiments are conducted at 18°C in 100 mM sodium phosphate buffer, pH 2.9 at 20,000 volts. Peptide concentration is determined by quantitative amino acid analysis on a Beckman 6300 Amino Acid analyzer.

### Example II

### Bacterial Strains And Spore Preparation

The following *Clostridium difficile* strains were used: ATCC 700057, ATCC BAA-1382 (Strain 630), ATCC 43594, and ATCC 9689. Spore crops were produced by anaerobic growth on Brucella blood agar oxyrase plates (BBAO) at 35°C for 2 days, followed by restreaking on BBAO. Spores were harvested into 15 ml 1x PBS and 0.1 % Tween-80 (PBST) after growing for 4 days at 35°C. Spores were centrifuged at 3000 x g for 20 min at 4°C, washed 3 times with 15 ml of(PBST), and resuspended in 10 ml HistoDenz (Sigma). The suspension was layer on top of 15 ml 50% HistoDenz solution to form a gradient. The gradient was centrifuged at 5000 x g for 15 min at 4°C. The pellet was washed three times in 15 ml PBST, previously described, and resuspended in 5 ml PBST. The sample was then sonicated two times for 5 s at power level 0.5 to lyse vegetative mother cells. The HistoDenz procedure, previously described, was then repeated. Final spore suspensions were stored at 4°C.

### Example III

### Thin Section Transmission Electron Microscopy

The following procedure was used for sample prep and Electron Microscopy:
1. Fix samples with 2.5% glutaraldehyde, 2% paraformaldehyde, 0.01M phosphate buffer, 2.5% DMSO at room temperature for 2 hours.
2. Wash in 0.1M phosphate buffer (3 changes over 15-30 minutes elapsed time).
3. 1% aqueous osmium tetroxide+1.5% potassium ferro cyanide for 1 h at RT.
4. Wash in DDW (3-4 changes over 15-30 minutes elapsed time) at RT.
5. Block staining: 0.5% aqueous uranyl acetate for overnight at 4C.
6. Dehydration (i.e., for example, with ethanol), each step for 10- 15 min.: a) 30%; b) 50%; c) 70% (possible for overnight at 4°C); d) 80%; e) 90%; f) 95%; g) 99%; and h) 100%
7. Propylene Oxide (PO): each step for 15-30 min
   a) Ethanol:PO = 50:50
   b) Ethanol:PO = 25:75
   c) PO = 100%
8. Epon: each step for 2 h
   a) Epon:PO = 30:70
   b) Epon:PO = 50:50
   c) Epon:PO = 75:25
   d) Epon = 100%
   e) Epon = 100%
9. Polymerization, i.e., for example by heating @ 60o∼70°C for 48 hr.
10. Sectioning
11. Uranyl acetate and Lead citrate staining
12. Carbon coating.
13. EM observation (JEOL 1200EX or Zeiss 902)

### Example IV

### Clostridium diffcile Exosporium Preparation

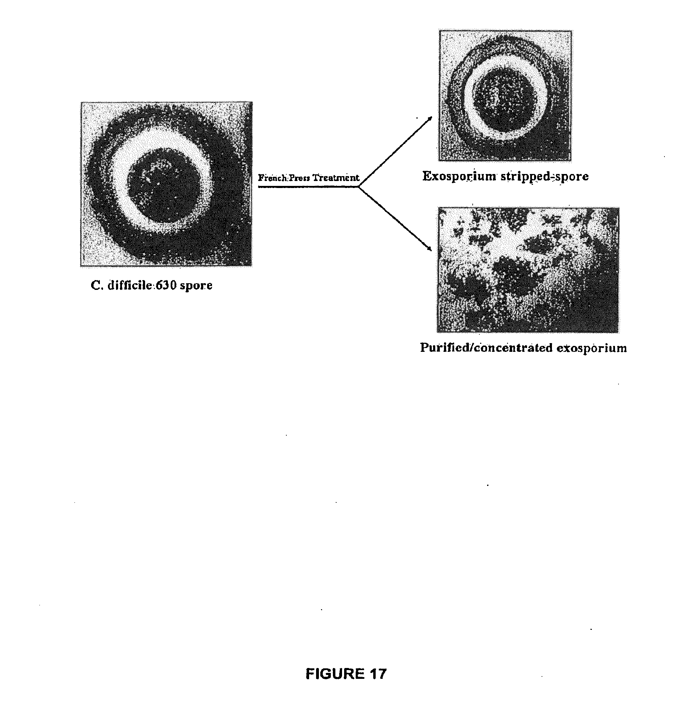

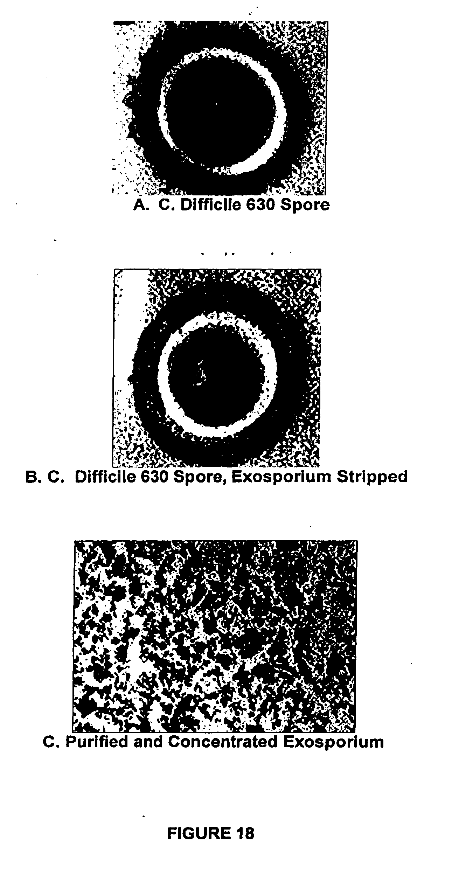

Approximately 109 spores were passed once through a chilled (4°C) French press at 20,000 lb/in² See, Figure 17. Transmission electron microscopic (TEM) examination confirmed that this procedure removed large exosporium fragments without detectable damage to the remainder of the spore when compared to unprocessed control exosporium fragments. The photographs compare spores before and after stripping along with purified exosporium attached.. See, Figure 18A versus Figure 18B. The sample extruded from the French press was centrifuged for 5 min at 9,000 x g and 4°C, and the supernatant was saved. The pellet was resuspended in 10 ml of cold TEP buffer, and centrifuged as described above. The pellet was resuspended in 10 ml of TEP Buffer and stored at -20°C while the supernatant was combined with the supernatant of the previous spin and centrifuged for 15 min at 9.500 x g and 4°C.

The resulting supernatant was then concentrated by centrifugation for approximately 60 min at 5000 x g and 4°C using Amicon Ultra Centrifugal Filter Devices (Millipore), final volume, 500 µl. Electron microscopic examination of this exosporium sample confirmed the virtual absence of spores. See, Figure 18C.

### Example V

### Exosporium Solubilization, Protein Electrophoresis, And Immunoblotting

Exosporium samples, 10µl, were solubilized by boiling for 5 min in 45 µl of 2x Novex Tris-Glycine SDS Sample Buffer (Invitrogen), 35 µl water and 10 µl 200 mM dithiothreitol. Solubilized proteins were separated by SDS-polyacrylamide gel electrophoresis (PAGE) with 4-12% Tris-Glycine gels. For immunoblotting, proteins were electrophoretically transferred from an SDS-polyacrylamide gel to a polyvinylidene difluoride membrane and treated as described in the manual for the Invitrogen Western Breeze assay kit. Briefly, each blot was blocked, probed with 1:1000 dilution of the primary MAb for 1 h, and washed. The blot was then probed with alkaline phosphatase conjugated, goat anti-rabbit IgG secondary antibody for 30 min, washed, and developed with the alkaline phosphatase developer solution.

### Example VI

### Amino-Terminal Protein Sequencing And Mass Spectrometry

The SDS-PAGE pieces washed with water and dehydrated in acetonitrile. The bands were then alkylated with iodoacetamide prior to the in-gel digestion. All bands were digested in-gel by incubating overnight at room temperature in trypsin, 5 µL 20 ng/µl trypsin in 50 mM ammonium bicarbonate. Formed peptides were extracted from the polyacrylamide in two aliquots of 30 µL 50% acetonitrile with 5% formic acid. These extracts were combined and evaporated to <10 µl in Speedvac and then resuspended in 1% acetic acid to make up a final volume of ∼30 µl for LC-MS analysis.

The LC-MS system (Finnigan LTQ) contained a self-packed 9 cm x 75 µm id Phenomenex Jupiter C18 reversed-phase capillary HPLC column. Ten µl volumes of the extract were injected and the peptides eluted were introduced into the source of the mass spectrometer on-line. The digest was analyzed using the data dependent multitask capability of the instrument acquiring full scan mass spectra to determine peptide molecular weights and product ion spectra to determine amino acid sequence in successive instrument scans. This mode of analysis produces approximately 2500 collisionally induced dissociation (CID) spectra of ions ranging in abundance over several orders of magnitude.

The data were analyzed by using all CID spectra collected in the experiment to search the NCBI non-redundant database with the search program Mascot using mammalian taxonomy filter. All matching spectra were verified by manual interpretation. The interpretation process was aided by additional searches using the programs Sequest and Blast as needed (Cleveland Clinic for Mass Spectrometry and Protein Sequencing).

### Example VII

### CD Spectroscopy

Circular dichroism (CD) spectroscopy may be performed on an AVIV 62 DS spectrometer (AVIV Associates, Lakewood, N.J.) equipped with a thermoelectric temperature control at 25°C. Samples contained 0.11 - 0.24 mg/mL peptide in either 50 mM sodium phosphate, pH 7.0; some samples also contain 40% trifluoroethanol or 0.1% (0.22 mM) lipopolysaccharide (LPS). Spectra are collected at 0.5 nm intervals with an averaging time of 2 s per datum point using a pathlength of 0.1 cm. The spectra are smoothed once over an interval of five data points prior to plotting with each spectrum representing the average of two scans. The average of two buffer scans are subtracted prior to data smoothing. The fractional helical content are calculated from the ratio of observed mean residue ellipticity to the mean residue ellipticity for a 100% helical peptide of identical length at 25°C. (Luo and Baldwin (1997).

### Example VIII

Calculation of Peptide Hydrophobic Moment and Hydrophobicity Peptide hydrophobicities and hydrophobic moments are calculated by using normalized consensus hydrophobicity scales. Eisenberg et al., J Cell Biochem 31: 11-7 25 (1986).

### Example XI

### Clostridium sporogenes Exosporium Removal

Exosporia are removed from C. *sporogenes* spores using homogenization in a Braun homogenizer was for 30-s intervals up to a total of 150 s, with 120 to 150 s required for optimal removal of exosporia as determined by crystal violet staining and light microscopy. Du et aI., "Bacillus thuringiensis HD-73 spores have surfaced localized Cry lAc toxin: physiological and pathogenic consequences" Appl. Environ. Microbiol. 62:3722-3726 (1996).

Spores are extracted to remove spore surface and exosporium components. Aronson et aI., "Comparative structural and functional aspects of spore surfaces" In: Spores VII. American Society for Microbiology. p. 54-61. G. Chambliss and J. C. Vary (ed.), Washington, D.C. (1978). Spores of *C*. *sporogenes* (1.1 x 109) are extracted in 5 mM cyclohexylaminoethane sulfonic acid-8 M urea-50 mM β-mercaptoethanol-0.8% sodium dodecyl sulfate (SDS) at pH 9.8 for 90 min at 378C. Spores are centrifuged at 12,000 x g for 10 min, and the supernatant extract boiled for 5 min. Extracts are applied directly to SDS-polyacrylamide gels containing 6 M urea. For molecular weight determination, gels are transferred to O.2-mm-pore size nitrocellulose. Blots are blocked with 3% bovine serum albumin in TBS, pH 7.4. The blots are then incubated with the appropriate biotinylated antibody (i.e., for example, C33 and/or C225, or D89 for C. sporogenes) diluted 1: 1,000 in TBS containing 0.05% Tween 20. Avidin labeled peroxidase (Sigma A7419) diluted 1:1,000 in TBS-0.05% Tween 20 is used to detect the antigens with the insoluble substrate 4-naphthol (Bio-Rad)

Glycoprotein determination is performed using periodic acid-Schiff staining. The gels are fixed overnight and treated with 0.7% periodic acid for 2 h and then with 0.2% metabisulfite for 2 h. Gels are then exposed to a Schiff reagent comprising basic fuchsin, sodium metabisulfite, and hydrochloric acid (Fisher Scientific, Pittsburgh, Pa.) The mobility of proteins that stain positive as glycoproteins from the spore extracts are determined and compared to the mobility of the antigens determined by Western blotting.

Ultrathin (ca. 100-mm) cryosections of spores for immunological labeling with colloidal gold are prepared. Chang et aI., "Immunocytochemical localization of antigens in B. cereus T spores" J Rapid Methods Automat. Microbiol. 2:229-233 (1993). C. *sporogenes* PA3679 spores from the same stock as had been used for the antigen are used for these experiments. The cryosections are floated on PBS containing 5% fetal calf serum, followed in sequence by PBS, PBS containing 0.1 M ammonium chloride, and PBS, each for 5 to 10 min at room temperature. Sections are floated on drops of primary reagent (the appropriate monoclonal antibody diluted 1: 100) for 1 to 2 h, followed by washing in PBS six times for 5 min each. Sections are then floated on drops of secondary reagent for 20 to 30 min. The secondary reagent is goat anti-mouse IgG plus goat anti- mouse IgM conjugated to 10-nm-diameter colloidal gold used at a dilution of 1:10. Sections are again washed six times in PBS, followed by three washes with distilled water. They are stained and embedded in methylcellulose by floating them on drops of 2.3 M methylcellulose containing about 20% (vol/vol) saturated aqueous uranyl acetate. Grids were picked up in loops, drained, and air dried. They are viewed in a Philips EM 300 electron microscope. Negative controls are spores treated with monoclonal antibodies of the IgGI and IgM isotypes which did not react with bacterial spores.

### Example X

### Cup-Scrub Assay Of Clostridium difficile Spores Bound To A Surface

This example describes one embodiment of a method that can assay spores that are bound to a surface. In this example, C. difficile spores are bound to a surface and assayed by the cup-scrub method. ATSM International, "Standard Test Method for Recovery of Microorganisms From Skin using the Cup Scrub Technique" E1874 - 09 (2009).

### Materials

Sterilizer-Any suitable steam sterilizer capable of producing the conditions of sterilization.

Scrub Cups-Sterile cylinders of suitable composition, preferably with rod handles to facilitate stabilization, height approximately 2.5 cm, inside diameter of convenient size. Useful sizes range from approximately 1.5 to 4.0 cm.

Polished Glass Rod or Rubber Policeman-Can be fashioned in the laboratory or purchased.

Pipettor-With disposable tips to deliver appropriate volume(s).

Sterile Beakers, Test Tubes or other container, to receive the cup scrub fluid.

Microbial Cultures-This test method can be used within a protocol targeting any microbial culture (i.e., for example, a *C*. *difficile* microbial culture).

Sampling and Dilution Fluid-Sterile Butterfield's phosphate buffered water or other recovery fluid of suitable composition; this should contain an antimicrobial inactivator specific for any antimicrobial that might be on the test site; inactivator efficacy should be determined by

### Test Method

### Test Control and Baseline Skin Sites

Skin sites are selected that are appropriate for *C*. *difficile* growth, providing contralateral sample sites for use as controls. A number of subjects (human or animal) selected for the assay depends on the statistical confidence needed for the expected test results, the variability encountered in the study, and the relative efficacy of any antibacterial agent that may be evaluated. There may be multiple sites available on subjects; randomization is required to suppress sample bias. The number of replicates required to discriminate effects will depend in part on the appropriateness and design of controls within the protocol. The use of this technique on isolated skin or equivalents is dependent on securing the test site in order to effectively perform the procedure.

### In Vivo Sample Collection

The area to be sampled is delineated by a sterile sampling cylinder. The cylinder is pressed firmly against the skin surface during sampling to ensure that the sampling fluid does not leak from the sampling site. A minimum 1.5-mL aliquot of sterile sampling fluid, with or without product neutralizers, is pipetted into the cylinder. The entire area is then scrubbed with moderate pressure for 60 ± 6 s using a sterile polished glass rod or policeman. After scrubbing, the sampling fluid is transferred by pipette into a sterile sample tube. This procedure is repeated once more with a fresh aliquot of sampling fluid. The sampling fluids are pooled. This procedure is repeated for each sampling site. The same pipettes, cylinders, glass rods, and policeman are used for both washes of a site, but new sterile equipment is used for each site. After samples are collected, paper toweling is used to blot the site dry. Care must be taken during this process to prevent the sampling fluid from spilling into an adjacent site that has not been sampled. Following all sampling, when using marker organisms, the sampling site should be decontaminated using 70 to 90 % isopropanol or equivalent, followed by a 4 % chlorhexidine scrub.

### Isolated Skin or Skin Equivalent Sample Collection

Quantitative microbial counts are obtained by the cup scrub technique. This procedure is used for test and control samples. Samples are positioned and secured as necessary to enable placement and effective use of the sampling cylinder. The area to be sampled is delineated by a sterile sampling cylinder. The cylinder is pressed firmly against the sample surface during sampling to ensure that the sampling fluid does not leak from the sampling site. A minimum 1.5-mL aliquot of sterile sampling fluid, with or without product neutralizers, is pipetted into the cylinder. The entire area is then scrubbed with moderate pressure for 60 ± 6 s using a sterile polished glass rod or policeman. After scrubbing, the sampling fluid is transferred by pipet into a sterile sample tube. This procedure is repeated once more with a fresh aliquot of sampling fluid. The sampling fluids are pooled. This procedure is repeated for each sampling site. The same pipettes, cylinders, glass rods, and policeman are used for both washes of a site, but new sterile equipment is used for each site. If there are multiple sample sites on the same piece of isolated tissue, care must be taken during this process to prevent the sampling fluid from spilling into an adjacent site that has not been sampled.

### Microbial Counts

Each sample is mixed thoroughly. Tenfold serial dilutions of each sample are prepared in dilution fluid. Duplicate quantitative pour or spread plates using soybeancasein digest agar with suitable neutralizer are prepared. Incubate plated samples at suitable growth temperature, 62°C for 24 to 72 h, or until colonies are visible on the plates.

### Example XI

### Spore Adherence Assays - Skin Proteins

This example provides one embodiment to assay spore adherence to a skin protein. For purposes of illustration, the example describes the generation of a involucrin skin protein to study adherence of C *difficile.*

### Plasmids

Compatible plasmids for this assay may including but not limited to, pIsdA IsdA overexpression vector, pMK4 E. coli-So aureus shuttle vector, pSRCOOl rIsdA complementation vector, pNZ8148 L. lactis expression plasmid, pLIsdA IsdA expression vector, pL_NEAT IsdA_NEAT expression vector, pL_C IsdA_C expression vector, pQE30 Overexpression plasmid (Qiagen), pHuman K, pQE30 human cytokeratin 10 expression, pCMC-SPORT6-INV Involucrin cDNA clone 4749952 (IMAGE consortium, MRC Geneservice), pQE30-INV 6 His-tagged involucrin, or pET11a-LOR Loricrin cDNA clone. Clarke et al., "IsdA of Staphylococcus aureus is a broad spectrum, iron-regulated adhesin" Mol. Microbiol. 51: 1509∼1519 (2004); Clarke et aI., "The Staphylococcus aureus surface protein IsdA mediates resistance to innate defenses of human skin" Cell Host Microbe 1:199-212 (2007); Sullivan et aI., "New shuttle vectors for Bacillus subtilis and Escherichia coli which allow rapid detection of inserted fragments" Gene 29:21-26 (1984); Kuipers, O. P., P. G. G. A. de Ruyter, M. Kleerebezem, and W. M. de Vos. 1998. Controlled overproduction ofproteins by lactic acid bacteria. J. Biotechnol. 64: 15-21; Sullivan et aI., "New shuttle vectors for Bacillus subtilis and Escherichia coli which allow rapid detection of inserted fragments" Gene 29:21-26 (1984); and Walsh et al., "Clumping factor B, a fibrinogen binding MSCRAMM (microbial surface component recognizing adhesive matrix molecules) adhesin of Staphylococcus aureus also binds the tail region of type I cytokeratin 10" J. Biol. Chem. 279:50691-50699 (2004).

*Clostridium difficile* is grown in a Luria-Bertani medium, using selection with kanamycin (50 µg/ml) where appropriate. C. *difficile* strains are grown in brain heart infusion medium (Oxoid) or chemically defined CL medium. Horsburgh et aI.., "In Staphylococcus aureus, Fur is an interactive regulator with PerR, contributes to virulence, and is necessary for oxidative stress resistance through positive regulation of catalase and iron homeostasis" J. Bacteriol. 183:468-475 (2001).. When included, antibiotics are added at the following concentrations: erythromycin, 5 µg/ml; lincomycin, 25 µg/ml; and tetracycline, 2.5 µg/ml. C. *difficile* cultures may be grown at 37°C.

### Cloning the involucrin gene

The coding sequence for involucrin is amplified from pCMV-SPORT6-INV by PCR employing Pfu polymerase and the forward primer CGCGGATCCATGTCCCAGCAA CACACAC (SEQ ID NO: 446), incorporating a BamHI site (underlined), and the reverse primer CCCAAGCTTTATTTATGTTTGGG TGGCCAC (SEQ ID NO: 447), incorporating a HindIII site (underlined). The fragment is cloned into pQE30 cut with BamHI and HindIII, forming pQE30-INV.

### Expression and purification of recombinant proteins.

The expression of hexahistidine-tagged recombinant IsdA (rIsdA), cytokeratin 10, and involucrin is induced by the addition of 100 µM isopropyl-β-D-thiogalactopyranoside (IPTG) to growing cells. Purification is achieved by using nickel chelate chromatography using the Hi-Trap system (Amersham Biosciences). Recombinant loricrin maybe affinity tagged or purified from lysed *E. coli* T4037.

### ELISA analysis of ligand binding.

An enzyme-linked immunosorbent assay (ELISA) is used to analyze the ability of rIsdA to bind ligands. Clarke et al., "Analysis of Ebh, a 1.1-megadalton cell wallassociated fibronectin-binding protein of Staphylococcus aureus" Infect. Immun 70:6680- 6687 (2002). Briefly, 100 µl of appropriate ligand or bovine serum albumin (BSA) in phosphate-buffered saline (PBS) (5 µg/ml) is added to wells of a 96-well microtiter plate (Nunc) overnight at 4°C. Plates are washed three times with PBST (PBS containing 0.05% [vol/vol] Tween 20), and the remaining protein-binding sites are blocked with 5% (wt/ vol) BSA in PBS for 2 h at room temperature. The plates are again washed three times in PBST, and different concentrations of purified rIsdA (i.e., for example, 0 to 15µM) diluted in PBS-O.1% (wt/vol) BSA were added. The plates are incubated for a further 1 h before being washed three times, and anti-IsdA (diluted 1:1,000) is added in PBS-O.1% (wtlvol) BSA, followed by 1 h of incubation.

Mouse antibodies used for the detection of bound IsdA may be raised in accordance with reported methods. Clarke et al., "IsdA of Staphylococcus aureus is a broad spectrum, iron-regulated adhesin" Mol. Microbiol. 51: 1509-1519 (2004). The plates are washed an additional three times, and alkaline phosphatase conjugated antimouse antibodies diluted 1:30,000 in PBS-O.1% (wt/vol) BSA is added and the plates incubated for 1 h. Finally, bound antibodies are detected by using the Sigma Fast p- nitrophenyl phosphate system (Sigma). Plates are read at 405 nm in a Victor microtiter plate reader (Wallac).

Studies of the inhibition of binding are performed by the incubation of rIsdA with various concentrations of inhibitor or BSA (0 to 10 µM) for 1 h at room temperature in PBS-0.1% (wt/vol) BSA. The reaction mixtures then are added to ligand-coated wells, and bound protein is detected.

### Atomic Force Microscopy

Atomic force microscopy (AFM) tips and supports are functionalized with IsdA and loricrin molecules as follows. AFM cantilevers (Microlevers; Veeco Metrology Group, Santa Barbara, CA) and silicon wafers (Siltronix, France) are coated using electron beam thermal evaporation with a 5-nm-thick Cr layer followed by a 30-nm-thick Au layer. Before use, the gold-coated surfaces are rinsed with ethanol, dried with a gentle nitrogen flow, and cleaned for 15 min by UV/ozone treatment (Jelight Co., Irvine, CA). Gold tips and gold supports are immersed overnight in ethanol solutions containing 0.05 mM nitrilotriacetate-terminated (5%) (Prochimia) and tri(ethylene glycol) [tri(EG)]terminated (95%) alkanethiols and rinsed with ethanol. Sonication is briefly applied to remove alkanethiol aggregates that can be adsorbed. The SAM (self-assembled monolayer)-coated surfaces then are immersed in a 40 mM aqueous solution of NiSO₄ (pH 7.2) for 1 h and rinsed with PBS. Finally, the samples are incubated in PBS with 2 µM His-tagged IsdA for 2 h and further rinsed several times with PBS. For loricrin and BSA, gold supports are immersed for 36 h in an ethanol solution containing a 20 µM mixture (95:5, mol/mol) of alkanethiols terminated with oligo(EG) (OEG) and OEG propionic acid. Following a rinse with ethanol, sonication is briefly applied to remove alkanethiol aggregates that may be adsorbed. The supports are immersed for 30 min into MilliQ water (Millipore) containing 20 g/LiterN-hydroxysuccinimide (NHS) (Aldrich) and 50 g/liter 1-ethyl-3-(3-dimethylaminopropyl)- carbodiimide (EDC) (Sigma), rinsed with MilliQ water, incubated with 10 µg/liter loricrin or BSA (sigma) in PBS for 3 h, further rinsed, and then used immediately.

AFM images and force-distance curves were obtained in PBS at room temperature using a Picoforce Multimode AFM (Veeco Metrology Group, Santa Barbara, CA). The supports were immobilized on a steel sample puck using a small piece of adhesive tape. The mounted samples were immediately transferred into the AFM liquid cell while avoiding dewetting. All force curves were recorded with a maximum applied force of 450 pN. The spring constants of the cantilevers were measured using the thermal noise method (Picoforce; Veeco Metrology Group), yielding values (0.011 N/m) that were slightly larger than those announced by the manufacturer (0.01 N/m). To account for the flexibility of the biomolecules, loading rates (in piconewtons per second) were estimated by multiplying the tip retraction velocity (in nanometers per second) by the slope of the rupture peaks (in piconewtons per nanometer).

### Microbial interactions with immobilized ligands

The binding of mid-log growth-phase cells to immobilized ligands may be measured using a previously described method. Ni Eidhin et al., Clumping factor B (ClfB), a new surface-located fibrinogenbinding adhesin of Staphylococcus aureus" Mol. Microbiol. 30:245-257 (1998). Further, squamous cells may be harvested from the anterior nares of healthy donors, and the binding of C. *difficile* grown in iron-free medium is assayed. O'Brien et al., "Staphylococcus aureus clumping factor B (ClfB) promotes adherence to human type I cytokeratin 10: implications for nasal colonization" Cell. Microbiol. 4:759-770 (2002). Comparisons between strains were performed using Student's t test.

### Example XII

### Expression and Purification of Recombinant Exosporium Proteins

Expression constructs were created containing recombinant exosporium proteins with a N-terminus polyHistidine tag and transformed in *E. coli* strains. Recombinant proteins were produced by inoculating I-liter cultures of Luria broth (supplemented with 100 µg/ml ampicillin) with 10 ml of an overnight culture of the expression construct described above. Following 2.5 h of growth at 37°C, isopropyl-β-D-thiogalactoside was added to a final concentration of 0.2 mM to induce protein expression and the cultures were allowed to grow for another 3 h. Bacteria were harvested by centrifugation, the supernatant decanted, and the cell pellets resuspended in PBS before being stored at -80 °C. The suspension was later thawed in an ambient-temperature water bath for 30 min and the cells lysed using a French press. Insoluble cell debris was removed by centrifugation at 28,000 xg for 20 min, followed by filtration through a 0.45-mm membrane. Recombinant exosporium protein was then initially purified using metal chelating chromatography. Bacterial lysates were applied to a 5-ml Ni₂-charged HiTrap chelating column (Amersham Pharmacia Biotech) and bound protein eluted with a 200-ml linear gradient of 0-200 mM imidazole in 4 mM Tris-HCl, 100 mM NaCI, pH 7.9, at a flow rate of 5 ml/min. Fractions corresponding to recombinant exosporium protein, as determined by SDS-PAGE, were pooled and dialyzed against 25 mM Tris-HCI, pH 8.0, before further purification by ion-exchange chromatography. Dialyzed protein was applied to a 5-ml HiTrap Q column (Amersham Pharmacia Biotech) and bound protein eluted with a 200-ml linear gradient of 0-0.5 M NaCI in 25 mM Tris-HCI, pH 8.0, at a flow rate of 5 ml/min. Fractions containing purified exosporium protein were identified by SDS-PAGE and estimated to be 90% pure.

### Example XIII

### Exosporium Protein Adherence Assays

Binding of exosporium proteins to human skin proteins and/or EpiDerm® differentiated human keratinocytes was performed essentially as described in Example X and XI above with the exception that FITC conjugated spores were replaced with the exosporium protein. Detection of bound protein was accomplished using fluorescein conjugated Streptavidin (Rockland Immunochemicals). The total fluorescence (Fₜₒₜₐₗ) per well was measured after a 1-h incubation using a Fluoroskan II fluorescence reader (Labsystems, Beverly, MA), with λₑₓ = 495 nm and λₑₘ = 528 nm.

## Claims

1. A screening method for screening for infection control compositions that inhibit, antagonize, interfere, displace and/or disrupt the binding of a microbial spore to an inanimate and/or animate surface, the method comprising: a) providing; i) an isolated peptide, wherein the peptide is derived from a *Clostridium* spore surface protein; and ii) an infection control test compound suspected of having an interaction with the peptide; b) contacting the peptide with an infection control test compound; c) detecting the interaction of the peptide with the infection control test compound; and d) where an interaction is detected, selecting the infection control test compound for further testing to identify effective infection control compositions, wherein the peptide is selected from the group consisting of SEQ ID NO: 1, SEQ ID NO: 3, SEQ ID NO: 5, SEQ ID NO: 7, and SEQ ID NO: 15.

2. The method of claim 1, wherein said microbial spore surface protein comprises a *Clostridium difficile* spore surface protein.

## Patentansprüche

1. Ein Screening-Verfahren für das Screening von Zusammensetzungen zur Infektionskontrolle, die die Bindung einer mikrobiellen Spore an eine unbelebte und/oder belebte Oberfläche blockieren, antagonisieren, beeinträchtigen, verschieben und/oder unterbrechen, das Verfahren bestehend aus: a) der Bereitstellung; i) eines isolierten Peptids, wobei das Peptid von einem Oberflächenprotein der *Clostridium*-Sporen abgeleitet ist; und ii) einer Testverbindung zur Infektionskontrolle, bei der davon ausgegangen wird, dass sie eine Wechselwirkung mit dem Peptid zeigt; b) dem Zusammenbringen des Peptids mit einer Testverbindung zur Infektionskontrolle; c) der Erfassung der Wechselwirkung des Peptids mit der Testverbindung zur Infektionskontrolle; und d) wenn eine Wechselwirkung festgestellt wird, der Auswahl der Testverbindung zur Infektionskontrolle für weitere Tests zur Identifizierung wirksamer Zusammensetzungen zur Infektionskontrolle, wobei das Peptid ausgewählt ist aus der Gruppe bestehend aus SEQ ID NO: 1, SEQ ID NO: 3, SEQ ID NO: 5, SEQ ID NO: 7 und SEQ ID NO: 15.

2. Das Verfahren in Anspruch 1, wobei besagtes mikrobielles Sporen-Oberflächenprotein ein Oberflächenprotein der *Clostridium difficile*-Spore umfasst.

## Revendications

1. Un procédé de criblage pour le criblage de compositions de prévention d'infections qui inhibent, antagonisent, interfèrent avec, déplacent et/ou rompent la liaison d'une spore microbienne à une surface animée et/ou inanimée, le procédé comprenant : a) la fourniture de i) un peptide isolé, où le peptide est dérivé d'une protéine de surface de la spore *Clostridium,* et ii) un composé de test de prévention d'infection dont on pense qu'il possède une interaction avec le peptide, b) la mise en contact du peptide avec un composé de test de prévention d'infection, c) la détection de l'interaction du peptide avec le composé de test de prévention d'infection, et d) si une interaction est détectée, la sélection du composé de test de prévention d'infection à des fins de tests complémentaires de façon à identifier des compositions de prévention d'infections efficaces, où le peptide est sélectionné dans le groupe se composant de SEQ ID NO:1, SEQ ID NO:3, SEQ ID NO:5, SEQ ID NO:7 et SEQ ID NO:15.

2. Le procédé selon la Revendication 1, où ladite protéine de surface de spore microbienne contient une protéine de surface de la spore *Clostridium difficile.*
